# FASCICULE DE BREVET EUROPEEN

(11) **EP 2 919 804 B1**
(45) Date de publication et mention de la délivrance du brevet: **31.01.2018**
(21) Numéro de dépôt: 13801658.9
(22) Date de dépôt: 13.11.2013
(51) Int. Cl.: A61K 38/28, A61K 9/00, A61K 47/26, A61K 47/34, A61K 47/36

(54) **FORMULATION À ACTION RAPIDE D'INSULINE COMPRENANT UN COMPOSÉ ANIONIQUE SUBSTITUÉ**
SCHNELL WIRKENDE INSULINFORMULIERUNG MIT EINER ANIONISCHEN SUBSTITUIERTEN VERBINDUNG
QUICK-ACTING INSULIN FORMULATION INCLUDING A SUBSTITUTED ANIONIC COMPOUND

(30) Priorité: 13.11.2012 FR 1260808; 13.11.2012 US 201261725775 P; 14.11.2012 FR 1260855; 14.11.2012 US 201261726349 P
(43) Date de publication de la demande: 23.09.2015
(73) Titulaire: Adocia, 69003 Lyon (FR)
(72) Inventeur: SOULA, Olivier, F-69330 Meyzieu (FR); SOULA, Gérard, F-69330 Meyzieu (FR); DAUTY, Emmanuel, F-69004 Lyon (FR); CHARVET, Richard, F-69140 Rillieux-la-Pape (FR)
(74) Mandataire: Tripoz, Inès
(86) Numéro de dépôt international: PCT/FR2013/052736
(87) Numéro de publication internationale: WO 2014/076423

(56) Documents cités:
- WO-A1-2013/064787
- US-A1- 2010 249 020
- BAUDYS M ET AL: "Extending insulin action in vivo by conjugation to carboxymethyl dextran", BIOCONJUGATE CHEMISTRY, ACS, WASHINGTON, DC, US, vol. 9, no. 2, 5 février 1998 (1998-02-05) , pages 176-183, XP002363506, ISSN: 1043-1802, DOI: 10.1021/BC970180A

## Description

La présente invention concerne une formulation à action rapide d'insuline.

Depuis la production d'insuline par génie génétique, au début des années 80, les patients diabétiques bénéficient d'insuline humaine pour se traiter. Ce produit a grandement amélioré cette thérapie puisque les risques immunologiques liés à l'utilisation d'insuline non humaine en particulier de porc se trouvent éliminés. Cependant, l'insuline humaine injectée par voie sous-cutanée a un effet hypoglycémiant seulement après 60 minutes, ce qui implique que les patients diabétiques traités avec l'insuline humaine doivent procéder à l'injection 30 minutes avant le repas.

Un des problèmes à résoudre pour améliorer la santé et le confort des patients diabétiques est de mettre à leur disposition des formulations d'insuline qui permettent d'apporter une réponse hypoglycémiante plus rapide que celle de l'insuline humaine et si possible s'approchant de la réponse physiologique de la personne saine. La sécrétion d'insuline endogène chez l'individu sain est immédiatement déclenchée par l'augmentation de la glycémie. L'objectif est de réduire le plus possible le délai entre l'injection d'insuline et le début du repas.

Aujourd'hui, il est admis que la mise à disposition de telles formulations est utile pour que la prise en charge de la maladie soit la meilleure possible.

Le génie génétique a permis d'apporter une réponse avec le développement d'insulines analogues rapides. Ces insulines sont modifiées sur un ou deux acides aminés pour être plus rapidement absorbées dans le compartiment sanguin après une injection sous-cutanée. Ces insulines lispro (Humalog®, Lilly), aspart (Novolog®,Novo) et glulisine (Apidra®, Sanofi Aventis) sont des solutions stables d'insuline avec une réponse hypoglycémiante plus rapide que celle de l'insuline humaine. Dès lors, les patients traités avec ces insulines analogues rapides peuvent procéder à l'injection d'insuline seulement 15 minutes avant le repas.

Le principe des insulines analogues rapides est de former des hexamères à la concentration de 100 UI/mL pour assurer la stabilité de l'insuline dans le produit commercial tout en favorisant la dissociation très rapide de ces hexamères en monomères après injection sous-cutanée afin d'obtenir une action rapide.

L'insuline humaine telle que formulée sous sa forme commerciale, ne permet pas d'obtenir une réponse hypoglycémiante proche en terme de cinétique de la réponse physiologique générée par le début d'un repas (hausse de la glycémie), car à la concentration d'usage (100 UI/mL), en présence de zinc et d'autres excipients tels que le phénol ou le *m*-crésol, elle s'assemble sous forme d'hexamère alors qu'elle est active sous forme de monomère et de dimère. L'insuline humaine est préparée sous forme d'hexamères pour être stable près de 2 ans à 4°C car sous forme de monomères, elle a une très forte propension à s'agréger puis à fibriller ce qui lui fait perdre son activité. De plus sous cette forme agrégée, elle présente un risque immunologique pour le patient.

Les dissociations des hexamères en dimères et des dimères en monomères retardent son action de près de 20 minutes comparativement à une insuline analogue rapide (Brange J., et al, Advanced Drug Delivery Review, 35,1999, 307-335).

En outre, la cinétique de passage des insulines analogues dans le sang, ainsi que leur cinétique de réduction de la glycémie, ne sont pas optimales et il existe un réel besoin d'une formulation ayant un temps d'action encore plus court afin de s'approcher des cinétiques de sécrétion d'insuline endogène chez les personnes saines.

La société Biodel a proposé une solution à ce problème avec une formulation d'insuline humaine comprenant de l'EDTA et de l'acide citrique telle que décrite dans la demande de brevet US200839365. L'EDTA par sa capacité à complexer les atomes de zinc et l'acide citrique par ses interactions avec les zones cationiques présentes à la surface de l'insuline sont décrits comme déstabilisant la forme hexamèrique de l'insuline et réduisant ainsi son temps d'action.

Cependant, une telle formulation présente notamment comme inconvénient de dissocier la forme hexamèrique de l'insuline qui est la seule forme stable susceptible de répondre aux exigences de stabilité de la réglementation pharmaceutique.

On connait également au nom de la demanderesse, la demande PCT WO2010/122385 qui décrit des formulations d'insuline humaine ou analogue et qui permet de résoudre les différents problèmes évoqués ci-dessus par l'addition d'un polysaccharide substitué comprenant des groupes carboxyles.

Cependant, les exigences entraînées par l'usage chronique et intensif voire l'usage pédiatrique de telles formulations conduisent l'homme de l'art à rechercher à utiliser des excipients dont la masse molaire et la taille soient les plus réduites possibles pour en faciliter l'élimination.

Les polysaccharides décrits dans les demandes WO 2010/122385A1 et US 2012/094902A1 comme excipients sont des composés constitués de chaînes dont les longueurs sont statistiquement variables et qui présentent une grande richesse de sites d'interaction possibles avec des principes actifs protéiques. Cette richesse pourrait induire un manque de spécificité en termes d'interaction et une molécule plus petite et mieux définie pourrait permettre d'être davantage spécifique à ce sujet.

En outre, une molécule avec un squelette bien défini est en général plus facilement traçable (MS/MS par exemple) dans les milieux biologiques lors d'expériences de pharmacocinétique ou d'ADME (administration, distribution, métabolisme, élimination) par rapport à un polymère qui donne généralement un signal très diffus et bruité en spectrométrie de masse.

A contrario, il n'est pas exclu qu'une molécule bien définie et plus courte puisse présenter un déficit de sites d'interaction possibles avec des principes actifs protéiques. En effet, en raison de leur taille réduite ils ne présentent pas les mêmes propriétés que les polymères de type polysaccharide car il y a perte de l'effet polymère comme cela est démontré dans les exemples comparatifs de la partie expérimentale, voir notamment les essais de solubilisation de l'insuline au point isoélectrique et les essais d'interaction avec une protéine modèle telle que l'albumine.

En dépit de ces résultats décourageants, la demanderesse a réussi à mettre au point des formulations susceptibles d'accélérer l'insuline en utilisant un composé anionique substitué en combinaison avec un composé polyanionique.

De plus comme dans le cas de l'utilisation de polysaccharides, la nature hexamèrique de l'insuline n'est pas affectée, donc la stabilité des formulations n'est pas affectée, comme cela est d'ailleurs confirmé par les exemples d'état d'association d'insuline humaine et analogue en dichroïsme circulaire en présence de composé anionique substitué selon l'invention.

La présente invention permet de résoudre les différents problèmes ci-dessus exposés puisqu'elle permet notamment de réaliser une formulation d'insuline, humaine ou analogue, capable après administration, d'accélérer le passage de l'insuline humaine ou de ses analogues dans le sang et de réduire plus rapidement la glycémie en comparaison des produits commerciaux d'insuline correspondants.

L'invention consiste en une composition, en solution aqueuse, comprenant de l'insuline sous forme hexamèrique, au moins un composé anionique substitué et un composé polyanionique, non polymèrique.

On entend par « composé anionique substitué » des composés constitués d'un squelette saccharidique formé d'un nombre discret u compris entre 1 et 8 (1 ≤ u ≤ 8) d'unités saccharidiques identiques ou différentes, liées par des liaisons glycosidiques identiques ou différentes, lesdites unités saccharidiques étant choisies dans le groupe constitué par les hexoses, sous forme cyclique ou sous forme réduite ouverte, ledit composé comportant des groupes fonctionnels carboxyles partiellement substitués, les groupes fonctionnels carboxyles non substitués étant salifiables.

Dans un mode de réalisation, l'insuline est sous forme hexamèrique.

Dans un mode de réalisation, l'insuline est l'insuline humaine.

On entend par insuline humaine une insuline obtenue par synthèse ou recombinaison dont la séquence peptidique est la séquence de l'insuline humaine, incluant les variations alléliques et les homologues.

Dans un mode de réalisation, l'insuline est une insuline humaine recombinante telle que décrite dans la Pharmacopée Européenne et la Pharmacopée Américaine.

Dans un mode de réalisation, l'insuline est une insuline analogue.

On entend par insuline analogue une insuline recombinante dont la séquence primaire contient au moins une modification par rapport à la séquence primaire de l'insuline humaine.

Dans un mode de réalisation l'insuline analogue est choisie dans le groupe constitué par l'insuline lispro (Humalog®), l'insuline aspart (Novolog®, Novorapid®) et l'insuline glulisine (Apidra®).

Dans un mode de réalisation, l'insuline analogue est l'insuline lispro (Humalog®).

Dans un mode de réalisation, l'insuline analogue est l'insuline aspart (Novolog®, Novorapid®).

Dans un mode de réalisation, l'insuline analogue est l'insuline glulisine (Apidra®).

Dans un mode de réalisation, le composé anionique substitué est choisi parmi les composés anioniques substitués, à l'état isolé ou en mélange, constitués d'un squelette formé d'un nombre discret u compris entre 1 et 8 (1 ≤ u ≤ 8) d'unités saccharidiques identiques ou différentes, liées par des liaisons glycosidiques identiques ou différentes, lesdites unités saccharidiques étant choisies dans le groupe constitué par les hexoses, sous forme cyclique ou sous forme réduite ouverte, caractérisés en ce qu'ils sont substitués par :
a) au moins un substituant de formule générale I :

   -[R₁]ₐ-[AA]ₘ Formule I

   - les substituants étant identiques ou différents lorsqu'il y a au moins deux substituants, dans lequel :
      - le radical -[AA] désigne un résidu d'acide aminé,
      - le radical -R₁- étant :
         - soit une liaison et alors a = 0, et le résidu d'acide aminé -[AA] est directement lié au squelette par une fonction G.
         - soit une chaîne carbonée et alors a = 1, en C2 à C15 éventuellement substituée et/ou comportant au moins un hétéroatome choisi parmi O, N et S et au moins une fonction acide avant la réaction avec l'acide aminé, ladite chaîne formant avec le résidu d'acide aminé -[AA] une fonction amide, et est fixée sur le squelette à l'aide d'une fonction F résultant d'une réaction entre une fonction hydroxyle portée par le squelette et une fonction ou un substituant porté par le précurseur du radical -R₁-,
   - F est une fonction choisie parmi les fonctions éther, ester ou carbamate,
   - G est une fonction carbamate,
   - m est égal à 1 ou 2,
   - le degré de substitution des unités saccharidiques, j, en -[R₁]ₐ-[AA]ₘ étant strictement supérieur à 0 et inférieur ou égal à 6, 0 < j ≤ 6
b) et, éventuellement, un ou plusieurs substituants -R'₁,
   - le substituant -R'₁ étant une chaîne carbonée en C2 à C15, éventuellement substituée et/ou comportant au moins un hétéroatome choisi parmi O, N et S et au moins une fonction acide sous forme de sel de cations alcalins ladite chaine étant liée au squelette par une fonction F' résultant d'une réaction entre une fonction hydroxyle portée par le squelette et une fonction ou un substituant porté par le précurseur du substituant -R'₁,
   - F' est une fonction éther, ester ou carbamate,
   - le degré de substitution des unités saccharidiques, i, en -R'₁, étant compris entre 0 et 6-j,0 ≤ i ≤ 6-j et,
   - F et F' sont identiques ou différentes,
   - F et G sont identiques ou différentes,
   - i+j ≤ 6.
   - -R'₁ identique ou différent de -R₁-,
   - Les fonctions acides salifiables libres portées par le substituant -R'₁ sont sous forme de sels de cations alcalins,
   - lesdites liaisons glycosidiques identiques ou différentes étant choisies dans le groupe constitué par les liaisons glycosidiques de type (1,1), (1,2), (1,3), (1,4) ou (1,6), dans une géométrie alpha ou béta,

Dans un mode de réalisation, le composé anionique substitué, à l'état isolé ou en mélange, est choisi parmi les composés anioniques substitués constitués d'un squelette saccharidique formé d'un nombre discret u compris entre 1 et 8 (1 ≤ u ≤ 8) d'unités saccharidiques identiques ou différentes, liées par des liaisons glycosidiques identiques ou différentes, lesdites unités saccharidiques étant choisies parmi les hexoses, sous forme cyclique ou sous forme réduite ouverte, caractérisés :
a) en ce qu'ils sont substitués de façon statistique par :
   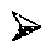 au moins un substituant de formule générale I :

      -[R₁]ₐ-[AA]ₘ Formule I

      - les substituants étant identiques ou différents lorsqu'il y a au moins deux substituants, dans lequel :
         - le radical -[AA]- désigne un résidu d'acide aminé, ledit acide aminé étant choisi dans le groupe constitué de la phénylalanine, l'alpha-méthyl-phénylalanine, la 3,4 dihydroxyphénylalanine, la tyrosine, l'alpha-méthyl-tyrosine, la O-méthyl-tyrosine, l'alpha-phénylglycine, la 4-hydroxyphénylglycine, la 3,5-dihydroxyphénylglycine et leurs sels de cations alcalins, lesdits dérivés étant de configuration absolue L ou D, -[AA] est fixé sur le squelette de la molécule par l'intermédiaire d'un bras de liaison -R₁- ou directement lié au squelette par une fonction G,
      - -R₁- étant :
         - soit une liaison G, et alors a = 0,
         - soit une chaîne carbonée, et alors a = 1, en C2 à C15 éventuellement substituée et/ou comportant au moins un hétéroatome choisi parmi O, N et S et portant au moins une fonction acide avant la réaction avec l'acide aminé, ladite chaîne formant avec le résidu d'acide aminé
         - [AA] une liaison amide, et est fixée sur le squelette saccharidique à l'aide d'une fonction F résultant d'une réaction entre une fonction hydroxyle portée par le squelette et une fonction portée par le précurseur de R₁,
      - F est une fonction éther, ester ou carbamate,
      - G est une fonction carbamate,
      - m est égal à 1 ou 2,
      - le degré de substitution, j, en -[R₁]ₐ-[AA]ₘ étant strictement supérieur à 0 et inférieur ou égal à 6, 0 < j ≤ 6,
      et, éventuellement,
   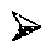 un ou plusieurs substituants -R'₁
      - -R'₁ étant une chaîne carbonée en C2 à C15, éventuellement substituée et/ou comportant au moins un hétéroatome (tels que O, N et S) et portant au moins une fonction acide sous forme de sel de cations alcalins ladite chaine étant fixée sur le squelette saccharidique par une fonction F' résultant d'une réaction entre une fonction hydroxyle portée par le squelette et une fonction portée par le précurseur de -R'₁,
      - F' est une fonction éther, ester ou carbamate,
      - le degré de substitution i, en -R'₁, étant compris entre 0 et 6-j, 0 ≤ i ≤ 6-j, et,
      - -R'₁- identique ou différent de -R₁,
      - F et F' identiques ou différentes,
      - F' et G identiques ou différentes
      - Les fonctions acides salifiables libres sont sous forme de sels de cations alcalins,
b) lesdites liaisons glycosidiques identiques ou différentes étant choisies dans le groupe constitué par les liaisons glycosidiques de type (1,1), (1,2), (1,3), (1,4) ou (1,6), dans une géométrie alpha ou béta,
c) i +j ≤ 6

Dans un mode de réalisation, m est égal à 1.

Dans un mode de réalisation, -R₁ et -R'1, identiques ou différents sont une chaîne carbonée en C2 à C8.

Dans un mode de réalisation, -R₁ et -R'1, identiques ou différents sont une chaîne carbonée en C2 à C4.

i et j sont des degrés de substitution statistiques et représentent le nombre moyen de substituants par unité saccharidique. Chaque unité saccharidique possédant plusieurs fonctions hydroxyles de réactivité différente, la distribution des substituants sur les composés anioniques substitués peut être différente d'une unité saccharidique à une autre au sein d'un même composé polyanionique.

Dans un mode de réalisation 0,3 ≤ i.

Dans un mode de réalisation 0,4 ≤ i.

Dans un mode de réalisation i ≤ 3.

Dans un mode de réalisation i ≤ 2,5.

Dans un mode de réalisation 0,3 ≤ j.

Dans un mode de réalisation 0,4 ≤ j.

Dans un mode de réalisation j ≤ 2.

Dans un mode de réalisation j ≤ 1,8.

Dans un mode de réalisation, on a i et j tels que 0 < i + j ≤ 6.

Dans un mode de réalisation, 0 < i + j ≤ 5.

Dans un mode de réalisation, 0 < i + j ≤ 4.

Dans un mode de réalisation, 0 < i + j ≤ 3.

Dans un mode de réalisation, 0 < i + j ≤ 2,5.

Dans un mode de réalisation, 0 < i + j ≤ 2.

Dans un mode de réalisation, 0,5 ≤ i + j ≤ 3.

Dans un mode de réalisation, 0,5 ≤ i + j ≤ 2,5.

Dans un mode de réalisation, 0,5 ≤ i + j ≤ 2.

Dans un mode de réalisation, 0,6 ≤ i + j ≤ 3,5.

Dans un mode de réalisation, 0,8 ≤ i + j ≤ 2,5.

Dans un mode de réalisation, 0,7 ≤ i + j ≤ 2,5.

Dans un mode de réalisation, 0,7 ≤ i + j ≤ 2.

Dans un mode de réalisation, 1 < i + j ≤ 2,5.

Dans un mode de réalisation, 1 < i + j ≤ 2.

Dans un mode de réalisation, -R1 et -R'1 sont fixés au squelette par une liaison éther.

Dans un mode de réalisation lorsque -R1- est une chaîne carbonée elle est directement fixée sur le squelette par une liaison éther.

Dans un mode de réalisation, lorsque -R1- est une chaîne carbonée elle comporte éventuellement un hétéroatome choisi dans le groupe constitué de O, N et S.

Dans un mode de réalisation, -R1- forme avec le résidu d'acide aminé AA une liaison amide, et est directement fixé sur le squelette par une fonction F éther.

Dans un mode de réalisation, -R1- forme avec le résidu d'acide aminé AA une liaison amide, et est directement fixé sur le squelette par une fonction F carbamate.

Dans un mode de réalisation, -R1- forme avec le résidu d'acide aminé AA une liaison amide, et est directement fixé sur le squelette par une fonction F ester.

Dans un mode de réalisation, -R1- et-R'1 sont choisis parmi les radicaux de formules II et III 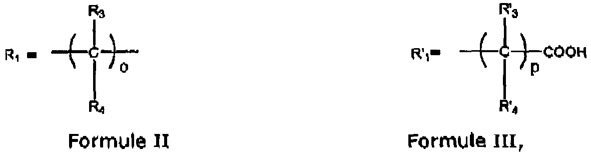 dans lesquelles ;
- o et p identiques ou différents, supérieurs ou égaux à 1 et inférieurs ou égaux à 12, et
- -R₃, -R'₃, -R₄ et -R'₄ identiques ou différents sont choisis dans le groupe constitué par un atome d'hydrogène, un alkyle saturé ou insaturé, linéaire, ramifié ou cyclique en C1 à C6, un benzyle, un alkyle-aryle en C7 à C10 et comportant éventuellement des hétéroatomes choisis dans le groupe constitué par O, N et/ou S, ou des fonctions choisies dans le groupe constitué par les fonctions acide carboxylique, amine, alcool ou thiol.

Dans un mode de réalisation,-R1- avant rattachement à -AA-, est -CH₂-COOH.

Dans un mode de réalisation, les composés anioniques substitués selon l'invention, sont caractérisés en ce que le radical -R'1 est -CH₂-COOH.

Dans un mode de réalisation, -R1- avant rattachement éventuel à -AA-, est issu de l'acide citrique.

Dans un mode de réalisation, -R1- avant rattachement éventuel à -AA-, est issu de l'acide malique.

Dans un mode de réalisation, -R'1 est issu de l'acide citrique.

Dans un mode de réalisation, -R'1 est Issu de l'acide malique.

Dans un mode de réalisation, -R1-, avant rattachement à -AA, est choisi parmi les groupes suivants, dans lesquels * représente le site de rattachement à F : 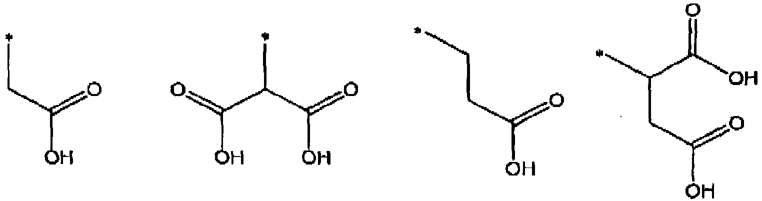 ou leurs sels de cations alcalins choisis dans le groupe constitué de Na⁺ ou K⁺.

Dans un mode de réalisation, -R'₁ est choisi parmi les groupes suivants, dans lesquels * représente le site de rattachement à F : 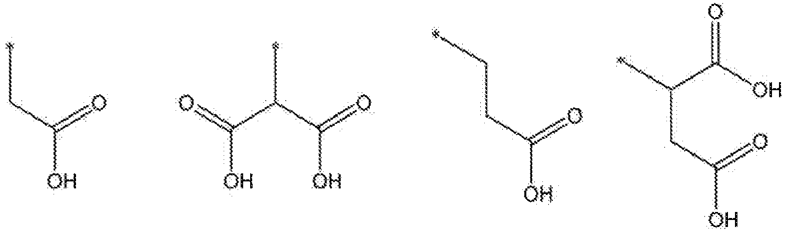 ou leurs sels de cations alcalins choisis dans le groupe constitué de Na⁺ ou K⁺.

Dans un mode de réalisation, le radical -[AA] est un résidu de la phénylalanine et de ses sels de cations alcalins de configuration absolue L, D ou racémique.

Dans un mode de réalisation, le radical -[AA] est un résidu d'alpha-méthyl-phénylalanine et de ses sels de cations alcalins de configuration absolue L, D ou racémique.

Dans un mode de réalisation, le radical -[AA] est un résidu de la 3,4 dihydroxyphénylalanine et de ses sels de cations alcalins de configuration absolue L, D ou racémique.

Dans un mode de réalisation, le radical -[AA] est un résidu de la tyrosine et de ses sels de cations alcalins de configuration absolue L, D ou racémique.

Dans un mode de réalisation, le radical -[AA] est un résidu de l'alpha-methyl-tyrosine et de ses sels de cations alcalins de configuration absolue L, D ou racémique.

Dans un mode de réalisation, le radical -[AA] est un résidu de la O-méthyl-tyrosine et de ses sels de cations alcalins de configuration absolue L, D ou racémique.

Dans un mode de réalisation, le radical -[AA] est un résidu de l'alpha-phénylglycine et de ses sels de cations alcalins de configuration absolue L, D ou racémique.

Dans un mode de réalisation, le radical -[AA] est un résidu de la 4-hydroxyphénylglycine et de ses sels de cations alcalins de configuration absolue L, D ou racémique.

Dans un mode de réalisation, le radical -[AA] est un résidu de la 3,5-dihydroxyphénylglycine et de ses sels de cations alcalins de configuration absolue L, D ou racémique.

Dans un mode de réalisation, le radical -[AA] est un résidu d'acide aminé sous forme de mélange racémique.

Dans un mode de réalisation, le radical -[AA] est un résidu d'acide aminé sous forme d'isomères de configuration absolue D, isolés.

Dans un mode de réalisation, le radical -[AA] est un résidu d'acide aminé sous forme de d'isomères de configuration absolue L, isolés.

Dans un mode de réalisation, u est compris entre 1 et 5.

Dans un mode de réalisation, u est compris entre 3 et 5.

Dans un mode de réalisation, u = 8.

Dans un mode de réalisation, u = 7.

Dans un mode de réalisation, u = 6.

Dans un mode de réalisation, u = 5.

Dans un mode de réalisation, u = 4.

Dans un mode de réalisation, u = 3.

Dans un mode de réalisation, u = 2.

Dans un mode de réalisation, u = 1.

Dans un mode de réalisation, les hexoses sont choisis dans le groupe constitué du mannose, du glucose, du fructose, du sorbose, du tagatose, du psicose, du galactose, de l'allose, de l'altrose, du talose, de l'idose, du gulose, du fucose, du fuculose, du rhamnose, du mannitol, du sorbitol et du galactitol (dulcitol).

Dans un mode de réalisation, les liaisons glycosidiques sont de types (1,4) ou (1,6).

Dans un mode de réalisation, le composé anionique substitué, est choisi parmi les composés anioniques constitués d'un squelette saccharidique formé d'un nombre discret u=2 d'unités saccharidiques identiques ou différentes choisies parmi les hexoses liées par une liaison glycosidique de type (1,1).

Dans un mode de réalisation, le composé anionique substitué, est choisi parmi les composés anioniques constitués d'un squelette saccharidique formé d'un nombre discret u=2 d'unités saccharidiques différentes choisies parmi les hexoses et liées par une liaison glycosidique de type (1,1), ledit squelette saccharidique étant choisi dans le groupe constitué du tréhalose et du sucrose.

Dans un mode de réalisation, le composé anionique substitué, est choisi parmi les composés anioniques constitués d'un squelette saccharidique formé d'un nombre discret u=2 d'unités saccharidiques identiques ou différentes choisies parmi les hexoses liées par une liaison glycosidique de type (1,2).

Dans un mode de réalisation, le composé anionique substitué, est choisi parmi les composés anioniques constitués d'un squelette saccharidique formé d'un nombre discret u=2 d'unités saccharidiques identiques ou différentes choisies parmi les hexoses liées par une liaison glycosidique de type (1,2), ledit squelette saccharidique étant le kojibiose.

Dans un mode de réalisation, le composé anionique substitué, est choisi parmi les composés anioniques constitués d'un squelette saccharidique formé d'un nombre discret u=2 d'unités saccharidiques identiques ou différentes choisies parmi les hexoses liées par une liaison glycosidique de type (1,3).

Dans un mode de réalisation, le composé anionique substitué, est choisi parmi les composés anioniques constitués d'un squelette saccharidique formé d'un nombre discret u=2 d'unités saccharidiques identiques ou différentes choisies parmi les hexoses liées par une liaison glycosidique de type (1,3), ledit squelette saccharidique étant choisi dans le groupe constitué du nigeriose et du laminaribiose.

Dans un mode de réalisation, le composé anionique substitué, est choisi parmi les composés anioniques constitués d'un squelette saccharidique formé d'un nombre discret u=2 d'unités saccharidiques identiques ou différentes choisies parmi les hexoses liées par une liaison glycosidique de type (1,4).

Dans un mode de réalisation, le composé anionique substitué, est choisi parmi les composés anioniques constitués d'un squelette saccharidique formé d'un nombre discret u=2 d'unités saccharidiques identiques ou différentes choisies parmi les hexoses liées par une liaison glycosidique de type (1,4), ledit squelette saccharidique étant choisi dans le groupe constitué du maltose, du lactose et de la cellobiose.

Dans un mode de réalisation le composé anionique substitué, est choisi parmi les composés anioniques constitués d'un squelette saccharidique formé d'un nombre discret u=2 d'unités saccharidiques identiques ou différentes choisies parmi les hexoses liées par une liaison glycosidique de type (1,6).

Dans un mode de réalisation, le composé anionique substitué, est choisi parmi les composés anioniques constitués d'un squelette saccharidique formé d'un nombre discret u=2 d'unités saccharidiques identiques ou différentes choisies parmi les hexoses liées par une liaison glycosidique de type (1,6), ledit squelette saccharidique étant choisi dans le groupe constitué de l'isomaltose, du mélibiose et de la gentiobiose.

Dans un mode de réalisation, le composé anionique substitué, est choisi parmi les composés anioniques constitués d'un squelette saccharidique formé d'un nombre discret u=2 d'unités saccharidiques identiques ou différentes choisies parmi les hexoses liées par une liaison glycosidique de type (1,6), ledit squelette saccharidique étant l'isomaltose.

Dans un mode de réalisation, le composé anionique substitué, est choisi parmi les composés anioniques constitués d'un squelette saccharidique formé d'un nombre discret u=2 d'unités saccharidiques dont l'une est sous forme cyclique et l'autre sous forme réduite ouverte.

Dans un mode de réalisation, le composé anionique substitué, est choisi parmi les composés anioniques constitués d'un squelette saccharidique formé d'un nombre discret u=2 d'unités saccharidiques dont l'une est sous forme cyclique et l'autre sous forme réduite ouverte, ledit squelette saccharidique étant choisi dans le groupe constitué du maltitol et de l'isomaltitol.

Dans un mode de réalisation, le composé anionique substitué selon l'invention, est caractérisé en ce que le squelette saccharidique est formé d'un nombre discret 3 ≤ u ≤ 8 d'unités saccharidiques identiques ou différentes.

Dans un mode de réalisation, le composé anionique substitué selon l'invention, est caractérisé en ce qu'au moins une des unités saccharidiques identiques ou différentes, qui composent le squelette saccharidique formé d'un nombre discret 3 ≤ u ≤ 8 d'unités saccharidiques, est choisie dans le groupe constitué de motifs hexose reliés par des liaisons glycosidiques identiques ou différentes.

Dans un mode de réalisation, le composé anionique substitué selon l'invention, est caractérisé en ce que les unités saccharidiques identiques ou différentes, qui composent le squelette saccharidique formé d'un nombre discret 3 ≤ u ≤ 8 d'unités saccharidiques, sont choisies parmi les hexoses et liées par au moins une liaison glycosidique de type (1,2).

Dans un mode de réalisation, le composé anionique substitué selon l'invention, est caractérisé en ce que les unités saccharidiques identiques ou différentes, qui composent le squelette saccharidique formé d'un nombre discret 3 ≤ u ≤ 8 d'unités saccharidiques, sont choisies parmi les hexoses et liées par au moins une liaison glycosidique de type (1,3).

Dans un mode de réalisation, le composé anionique substitué selon l'invention, est caractérisé en ce que les unités saccharidiques identiques ou différentes, qui composent le squelette saccharidique formé d'un nombre discret 3 ≤ u ≤ 8 d'unités saccharidiques, sont choisies parmi les hexoses et liées par au moins une liaison glycosidique de type (1,4).

Dans un mode de réalisation, le composé anionique substitué selon l'invention, est caractérisé en ce que les unités saccharidiques identiques ou différentes, qui composent le squelette saccharidique formé d'un nombre discret 3 ≤ u ≤ 8 d'unités saccharidiques, sont choisies parmi les hexoses et liées par au moins une liaison glycosidique de type (1,6).

Dans un mode de réalisation, le composé anionique substitué selon l'invention, est caractérisé en ce que le squelette saccharidique est formé d'un nombre discret u=3 d'unités saccharidiques identiques ou différentes.

Dans un mode de réalisation, le composé anionique substitué selon l'invention, est caractérisé en ce qu'ils comportent au moins une unité saccharidique choisie dans le groupe constitué des hexoses sous forme cyclique et au moins une unité saccharidique choisie dans le groupe constitué des hexoses sous forme ouverte.

Dans un mode de réalisation, le composé anionique substitué selon l'invention, est caractérisé en ce que les trois unités saccharidiques sont identiques.

Dans un mode de réalisation le composé anionique substitué selon l'invention, est caractérisé en ce que deux des trois unités saccharidiques sont identiques.

Dans un mode de réalisation, le composé anionique substitué selon l'invention, est caractérisé en ce que les unités saccharidiques identiques sont choisies parmi les hexoses dont deux sont sous forme cyclique et une sous forme réduite ouverte et liées par des liaisons glycosidique de type (1,4).

Dans un mode de réalisation, le composé anionique substitué selon l'invention, est caractérisé en ce que les unités saccharidiques identiques sont choisies parmi les hexoses dont deux sont sous forme cyclique et une sous forme réduite ouverte et liées par des liaisons glycosidique de type (1,6).

Dans un mode de réalisation le composé anionique substitué selon l'invention, est caractérisé en ce que les unités saccharidiques identiques ou différentes sont choisies parmi les hexoses et que l'hexose central est lié par une liaison glycosidique de type (1,2) et par une liaison glycosidique de type (1,4).

Dans un mode de réalisation, le composé anionique substitué selon l'invention, est caractérisé en ce que les unités saccharidiques identiques ou différentes sont choisies parmi les hexoses et que l'hexose central est lié par une liaison glycosidique de type (1,3) et par une liaison glycosidique de type (1,4).

Dans un mode de réalisation, le composé anionique substitué selon l'invention, est caractérisé en ce que les unités saccharidiques identiques ou différentes sont choisies parmi les hexoses et que l'hexose central est lié par une liaison glycosidique de type (1,2) et par une liaison glycosidique de type (1,6).

Dans un mode de réalisation le composé anionique substitué selon l'invention, est caractérisé en ce que les unités saccharidiques identiques ou différentes sont choisies parmi les hexoses et que l'hexose central est lié par une liaison glycosidique de type (1,2) et par une liaison glycosidique de type (1,3).

Dans un mode de réalisation, le composé anionique substitué selon l'invention, est caractérisé en ce que les unités saccharidiques identiques ou différentes sont choisies parmi les hexoses et que l'hexose central est lié par une liaison glycosidique de type (1,4) et par une liaison glycosidique de type (1,6).

Dans un mode de réalisation, le composé anionique substitué selon l'invention, est caractérisé en ce que le squelette saccharidique est l'erlose.

Dans un mode de réalisation, le composé anionique substitué selon l'invention, est caractérisé en ce que les trois unités saccharidiques identiques ou différentes sont des motifs hexose choisis dans le groupe constitué par le mannose et le glucose.

Dans un mode de réalisation, le composé anionique substitué selon l'invention, est caractérisé en ce que le squelette saccharidique est le maltotriose.

Dans un mode de réalisation, le composé anionique substitué selon l'invention, est caractérisé en ce que le squelette saccharidique est l'isomaltotriose.

Dans un mode de réalisation, le composé anionique substitué selon l'invention, est caractérisé en ce que le squelette saccharidique est formé d'un nombre discret u=4 d'unités saccharidiques identiques ou différentes.

Dans un mode de réalisation, le composé anionique substitué selon l'invention, est caractérisé en ce que les quatre unités saccharidiques sont identiques.

Dans un mode de réalisation, le composé anionique substitué selon l'invention, est caractérisé en ce que trois des quatre unités saccharidiques sont identiques.

Dans un mode de réalisation, le composé anionique substitué selon l'invention, est caractérisé en ce que les quatre unités saccharidiques sont des motifs hexose choisis dans le groupe constitué par le mannose et le glucose.

Dans un mode de réalisation, le composé anionique substitué selon l'invention, est caractérisé en ce que le squelette saccharidique est le maltotétraose.

Dans un mode de réalisation, le composé anionique substitué selon l'invention, est caractérisé en ce que les unités saccharidiques identiques ou différentes sont choisies parmi les hexoses et qu'un hexose terminal est lié par une liaison glycosidique de type (1,2) et que les autres sont liés entre eux par une liaison glycosidique de type (1,6).

Dans un mode de réalisation, le composé anionique substitué selon l'invention, est caractérisé en ce que les unités saccharidiques identiques ou différentes sont choisies parmi les hexoses et liées par une liaison glycosidique de type (1,6).

Dans un mode de réalisation, le composé anionique substitué selon l'invention, est caractérisé en ce que le squelette saccharidique est formé d'un nombre discret u=5 d'unités saccharidiques identiques ou différentes.

Dans un mode de réalisation, le composé anionique substitué selon l'invention, est caractérisé en ce que les cinq unités saccharidiques sont identiques.

Dans un mode de réalisation, le composé anionique substitué selon l'invention, est caractérisé en ce que les cinq unités saccharidiques sont des motifs hexose choisis dans le groupe constitué par le mannose et le glucose.

Dans un mode de réalisation, le composé anionique substitué selon l'invention, est caractérisé en ce que les unités saccharidiques identiques ou différentes sont choisies parmi les hexoses et liées par une liaison glycosidiques de type (1,4).

Dans un mode de réalisation, le composé anionique substitué selon l'invention, est caractérisé en ce que le squelette saccharidique est le maltopentaose.

Dans un mode de réalisation, le composé anionique substitué selon l'invention, est caractérisé en ce que le squelette saccharidique est formé d'un nombre discret u=6 d'unités saccharidiques identiques ou différentes.

Dans un mode de réalisation, le composé anionique substitué selon l'invention, est caractérisé en ce que les six unités saccharidiques sont identiques.

Dans un mode de réalisation, le composé anionique substitué selon l'invention, est caractérisé en ce que les unités saccharidiques identiques ou différentes sont choisies parmi les hexoses et liées par une liaison glycosidique de type (1,4).

Dans un mode de réalisation, le composé anionique substitué selon l'invention, est caractérisé en ce que les six unités saccharidiques identiques ou différentes sont des motifs hexose choisis dans le groupe constitué par le mannose et le glucose.

Dans un mode de réalisation, le composé anionique substitué selon l'invention, est caractérisé en ce que le squelette saccharidique est le maltohexaose.

Dans un mode de réalisation, le composé anionique substitué selon l'invention, est caractérisé en ce que le squelette saccharidique est formé d'un nombre discret u=7 d'unités saccharidiques identiques ou différentes.

Dans un mode de réalisation le composé anionique substitué selon l'invention, est caractérisé en ce que les sept unités saccharidiques sont identiques.

Dans un mode de réalisation, le composé anionique substitué selon l'invention, est caractérisé en ce que les unités saccharidiques identiques ou différentes sont choisies parmi les hexoses et liées par une liaison glycosidique de type (1,4).

Dans un mode de réalisation, le composé anionique substitué selon l'invention, est caractérisé en ce que les sept unités saccharidiques sont des motifs hexose choisis dans le groupe constitué par le mannose et le glucose.

Dans un mode de réalisation, le composé anionique substitué selon l'invention, est caractérisé en ce que le squelette saccharidique est le maltoheptaose.

Dans un mode de réalisation, le composé anionique substitué selon l'invention, est caractérisé en ce que le squelette saccharidique est formé d'un nombre discret u=8 d'unités saccharidiques identiques ou différentes.

Dans un mode de réalisation, le composé anionique substitué selon l'invention, est caractérisé en ce que les huit unités saccharidiques sont identiques.

Dans un mode de réalisation, le composé anionique substitué selon l'invention, est caractérisé en ce que les unités saccharidiques identiques ou différentes sont choisies parmi les hexoses et liées par une liaison glycosidique de type (1,4).

Dans un mode de réalisation, le composé anionique substitué selon l'invention, est caractérisé en ce que les huit unités saccharidiques sont des motifs hexose choisis dans le groupe constitué par le mannose et le glucose.

Dans un mode de réalisation, le composé anionique substitué selon l'invention, est caractérisé en ce que le squelette saccharidique est le maltooctaose.

Dans un mode de réalisation le composé anionique substitué comportant un nombre discret de motifs saccharidiques est un composé naturel.

Dans un mode de réalisation, le composé anionique substitué comportant un nombre discret de motifs saccharidiques est un composé synthétique.

Dans un mode de réalisation, le composé anionique substitué selon l'invention, est caractérisé en ce qu'il est obtenu par dégradation enzymatique d'un polysaccharide suivie d'une purification.

Dans un mode de réalisation, le composé anionique substitué selon l'invention, est caractérisé en ce qu'il est obtenu par dégradation chimique d'un polysaccharide suivie d'une purification.

Dans un mode de réalisation, le composé anionique substitué selon l'invention, est caractérisé en ce qu'il est obtenu par voie chimique, par couplage covalent de précurseurs de plus bas poids moléculaire.

Dans un mode de réalisation, le composé anionique substitué selon l'invention, est caractérisé en ce que le squelette saccharidique est le sophorose.

Dans un mode de réalisation, le composé anionique substitué selon l'invention, est caractérisé en ce que le squelette saccharidique est le saccharose.

Dans un mode de réalisation, le composé anionique substitué selon l'invention, est caractérisé en ce que le squelette saccharidique est le lactulose.

Dans un mode de réalisation, le composé anionique substitué selon l'invention, est caractérisé en ce que le squelette saccharidique est le maltulose.

Dans un mode de réalisation, le composé anionique substitué selon l'invention, est caractérisé en ce que le squelette saccharidique est le leucrose.

Dans un mode de réalisation, le composé anionique substitué selon l'invention, est caractérisé en ce que le squelette saccharidique est le rutinose.

Dans un mode de réalisation, le composé anionique substitué selon l'invention, est caractérisé en ce que le squelette saccharidique est le isomaltulose.

Dans un mode de réalisation, le composé anionique substitué selon l'invention, est caractérisé en ce que le squelette saccharidique est le fucosyllactose.

Dans un mode de réalisation, le composé anionique substitué selon l'invention, est caractérisé en ce que le squelette saccharidique est le gentianose.

Dans un mode de réalisation, le composé anionique substitué selon l'invention, est caractérisé en ce que le squelette saccharidique est le raffinose.

Dans un mode de réalisation, le composé anionique substitué selon l'invention, est caractérisé en ce que le squelette saccharidique est le mélézitose.

Dans un mode de réalisation, le composé anionique substitué selon l'invention, est caractérisé en ce que le squelette saccharidique est le panose.

Dans un mode de réalisation, le composé anionique substitué selon l'invention, est caractérisé en ce que le squelette saccharidique est le kestose.

Dans un mode de réalisation, le composé anionique substitué selon l'invention, est caractérisé en ce que le squelette saccharidique est le stachyose.

Dans un mode de réalisation, le composé polyanionique est un composé polyanionique non polymérique (PNP) dont l'affinité pour le zinc est inférieure à l'affinité de l'insuline pour le zinc et dont la constante de dissociation Kd_{Ca} =[composé PNP]^{r} [Ca²⁺]^{s}/[composé PNP)^{r}-(Ca²⁺)^{s}] est inférieure ou égale à 10^{-1,5}.

Les constantes de dissociation (Kd) des différents composés polyanioniques vis à vis des ions de calcium sont déterminées par calibration externe à l'aide d'une électrode spécifique aux ions Calcium (Mettler Toledo) et d'une électrode de référence. Toutes les mesures sont effectuées dans 150 mM de NaCl à pH 7. Seules les concentrations en ions calcium libres sont déterminées ; les ions calcium liés au composé polyanionique n'induisent pas de potentiel d'électrode.

Dans un mode de réalisation, le composé polyanionique est choisie dans le groupe constitué des polyacides carboxyliques et leurs sels de Na⁺, K⁺, Ca²⁺ ou Mg²⁺..

Dans un mode de réalisation, le polyacide carboxylique est choisi dans le groupe constitué par l'acide citrique, l'acide tartrique, et leurs sels de Na⁺, K⁺, Ca²⁺ ou Mg²⁺.

Dans un mode de réalisation, le composé polyanionique est choisi dans le groupe constitué des polyacides phosphoriques et leurs sels de Na⁺, K⁺, Ca²⁺ ou Mg²⁺.

Dans un mode de réalisation, le polyacide phosphorique est le triphosphate et ses sels de Na⁺, K⁺, Ca²⁺ ou Mg²⁺.

Dans un mode de réalisation, le composé polyanionique est l'acide citrique et ses sels de Na⁺, K⁺, Ca²⁺ ou Mg²⁺.

Dans un mode de réalisation, le composé polyanionique est l'acide tartrique et ses sels de Na⁺, K⁺, Ca²⁺ ou Mg²⁺.

Dans un mode de réalisation, le composé polyanionique est l'acide triphosphorique et ses sels de Na⁺, K⁺, Ca²⁺ ou Mg²⁺.

Dans un mode de réalisation, le composé polyanionique est un composé constitué d'un squelette saccharidique formé d'un nombre discret d'unités saccharidiques obtenu à partir d'un composé disaccharide choisi dans le groupe constitué par le tréhalose, le maltose, le lactose, le saccharose, le cellobiose, l'isomaltose, le maltitol et l'isomaltitol.

Dans un mode de réalisation, le composé polyanionique constitué d'un squelette saccharidique formé d'un nombre discret d'unités saccharidiques est obtenu à partir d'un composé constitué d'un squelette formé d'un nombre discret d'unités saccharidiques choisi dans le groupe constitué par le maltotriose, le maltotétraose, le maltopentaose, le maltohexaose, le maltoheptaose, le maltooctaose et l'isomaltotriose

Dans un mode de réalisation, le composé polyanionique constitué d'un squelette saccharidique formé d'un nombre discret d'unités saccharidiques est choisi dans le groupe constitué par le carboxyméthylmaltotriose, le carboxyméthylmaltotétraose, le carboxyméthylmaltopentaose, le carboxyméthylmaltohexaose, le carboxyméthylmaltoheptaose, le carboxyméthylmaltooactose et le carboxyméthylisomaltotriose.

Dans un mode de réalisation, le ratio (nombre de moles de fonctions acides portées par le composé polyanionique / nombre de moles de composé anionique) est supérieur ou égal à 3.

Dans un mode de réalisation, le ratio (nombre de moles de fonctions acides portées par le composé polyanionique / nombre de moles de composé anionique) est supérieur ou égal à 4.

Dans un mode de réalisation, le ratio (nombre de moles de fonctions acides portées par le composé polyanionique constitué d'un squelette saccharidique / nombre de moles de composé anionique) est supérieur ou égal à 5.

Dans un mode de réalisation, le ratio (nombre de moles de fonctions acides portées par le composé polyanionique constitué d'un squelette saccharidique / nombre de moles de composé anionique) est supérieur ou égal à 8.

Dans un mode de réalisation, les ratios molaires composé anionique substitué/insuline sont compris entre 0,6 et 75.

Dans un mode de réalisation, les ratios molaires sont compris entre 0,7 et 50.

Dans un mode de réalisation, les ratios molaires sont compris entre 1,4 et 35.

Dans un mode de réalisation, les ratios molaires sont compris entre 1,9 et 30.

Dans un mode de réalisation, les ratios molaires sont compris entre 2,3 et 30.

Dans un mode de réalisation, le ratio molaire composé anionique substitué/insuline est égal à 8.

un mode de réalisation, le ratio molaire composé anionique substitué/insuline est égal à 12.

Dans un mode de réalisation, le ratio molaire composé anionique substitué/insuline est égal à 16.

Dans un mode de réalisation, les ratios massiques composé anionique substitué/insuline sont compris entre 0,5 et 10.

Dans un mode de réalisation, les ratios massiques sont compris entre 0,6 et 7.

Dans un mode de réalisation, les ratios massiques sont compris entre 1,2 et 5.

Dans un mode de réalisation, les ratios massiques sont compris entre 1,6 et 4.

Dans un mode de réalisation, les ratios massiques sont compris entre 2 et 4.

Dans un mode de réalisation, le ratio massique composé anionique substitué/insuline est 2.

Dans un mode de réalisation, le ratio massique composé anionique substitué/insuline est 3.

Dans un mode de réalisation, le ratio massique composé anionique substitué/insuline est 4.

Dans un mode de réalisation, le ratio massique composé anionique substitué/insuline est 6

Dans un mode de réalisation, la concentration en composé anionique substitué est comprise entre 1,8 et 36 mg/mL.

Dans un mode de réalisation, la concentration en composé anionique substitué est comprise entre 1,8 et 36,5 mg/mL.

Dans un mode de réalisation, la concentration en composé anionique substitué est comprise entre 2,1 et 25 mg/mL.

Dans un mode de réalisation, la concentration en composé anionique substitué est comprise entre 4,2 et 18 mg/mL.

Dans un mode de réalisation, la concentration en composé anionique substitué est comprise entre 5,6 et 15 mg/mL.

Dans un mode de réalisation, la concentration en composé anionique substitué est comprise entre 7 et 15 mg/mL.

Dans un mode de réalisation, la concentration en composé anionique substitué est 7,3 mg/mL.

Dans un mode de réalisation, la concentration en composé anionique substitué est 10,5 mg/mL.

Dans un mode de réalisation, la concentration en composé anionique substitué est 14,6 mg/mL.

Dans un mode de réalisation, la concentration en composé anionique substitué est 21,9 mg/mL.

Dans un mode de réalisation, la concentration en composé polyanionique est comprise entre 2 et 150 mM.

Dans un mode de réalisation, la concentration en composé polyanionique est comprise entre 2 et 100 mM.

Dans un mode de réalisation, la concentration en composé polyanionique est comprise entre 2 et 75 mM.

Dans un mode de réalisation, la concentration en composé polyanionique est comprise entre 2 et 50 mM.

Dans un mode de réalisation, la concentration en composé polyanionique est comprise entre 2 et 30 mM.

Dans un mode de réalisation, la concentration en composé polyanionique est comprise entre 2 et 20 mM.

Dans un mode de réalisation, la concentration en composé polyanionique est comprise entre 2 et 10 mM.

Dans un mode de réalisation, la concentration en composé polyanionique est comprise entre 5 et 150 mM.

Dans un mode de réalisation, la concentration en composé polyanionique est comprise entre 5 et 100 mM.

Dans un mode de réalisation, la concentration en composé polyanionique est comprise entre 5 et 75 mM.

Dans un mode de réalisation, la concentration en composé polyanionique est comprise entre 5 et 50 mM.

Dans un mode de réalisation, la concentration en composé polyanionique est comprise entre 5 et 30 mM.

Dans un mode de réalisation, la concentration en composé polyanionique est comprise entre 5 et 20 mM.

Dans un mode de réalisation, la concentration en composé polyanionique est comprise entre 5 et 10 mM.

Dans un mode de réalisation, la concentration en composé polyanionique est comprise entre 0,5 et 30 mg/mL.

Dans un mode de réalisation, la concentration en composé polyanionique est comprise entre 0,5 et 25 mg/mL.

Dans un mode de réalisation, la concentration en composé polyanionique est comprise entre 0,5 et 10 mg/mL.

Dans un mode de réalisation, la concentration en composé polyanionique est comprise entre 0,5 et 8 mg/mL.

Dans un mode de réalisation, la concentration en composé polyanionique est comprise entre 1 et 30 mg/mL.

Dans un mode de réalisation, la concentration en composé polyanionique est comprise entre 1,5 et 25 mg/mL.

Dans un mode de réalisation, la concentration en composé polyanionique est comprise entre 2 et 25 mg/mL.

Dans un mode de réalisation, la concentration en composé polyanionique est comprise entre 2 et 10 mg/mL.

Dans un mode de réalisation, la concentration en composé polyanionique est comprise entre 2 et 8 mg/mL.

Dans un mode de réalisation, le composé anionique substitué est le maltotrioseméthylcarboxylate de sodium modifié par le phénylalaninate de sodium, u= 3, i = 0,65, j = 1,0.

Dans un mode de réalisation, le composé anionique substitué est le maltotrioseméthylcarboxylate de sodium modifié par le phénylalaninate de sodium, u = 3, i = 1,0, j = 0,65.

Dans un mode de réalisation, le composé anionique substitué est le maltotrioseméthylcarboxylate de sodium modifié par le phénylalaninate de sodium, u = 3, i = 0,46, j = 1,2.

Dans un mode de réalisation, le composé anionique substitué est le maltotrioseméthylcarboxylate de sodium modifié par le phénylalaninate de sodium, u = 3, i = 0,35, j = 0,65.

Dans un mode de réalisation, le composé polyanionique est le maltotrioseméthylcarboxylate de sodium.

Dans un mode de réalisation, le composé polyanionique est le citrate de sodium.

Dans un mode de réalisation, le composé polyanionique est le triphosphate sous forme acide ou sous forme basique sous forme de sel de sodium ou de sel de potassium.

Dans un mode de réalisation, le composé polyanionique est le tartrate, sous forme acide ou sous forme basique sous forme de sel de sodium ou de sel de potassium

L'invention concerne également une formulation pharmaceutique d'insuline comprenant une composition selon l'invention dans laquelle l'insuline est sous forme hexamèrique.

Dans un mode de réalisation, elle concerne une formulation pharmaceutique caractérisée en ce que la concentration en insuline est comprise entre 240 et 3000 µM (40 à 500 UI/mL).

Dans un mode de réalisation, elle concerne une formulation pharmaceutique caractérisée en ce que la concentration en insuline est comprise entre 600 et 3000 µM (100 à 500 UI/mL).

Dans un mode de réalisation, elle concerne une formulation pharmaceutique caractérisée en ce que la concentration en insuline est comprise entre 600 et 2400 µM (100 à 400 UI/mL).

Dans un mode de réalisation, elle concerne une formulation pharmaceutique caractérisée en ce que la concentration en insuline est comprise entre 600 et 1800 µM (100 à 300 UI/mL).

Dans un mode de réalisation, elle concerne une formulation pharmaceutique caractérisée en ce que la concentration en insuline est comprise entre 600 et 1200 µM (100 à 200 UI/mL).

Dans un mode de réalisation, elle concerne une formulation pharmaceutique caractérisée en ce que la concentration en insuline est 600 µM (100 UI/mL).

Dans un mode de réalisation, elle concerne une formulation pharmaceutique caractérisée en ce que la concentration en insuline est 1200 µM (200 UI/mL).

Dans un mode de réalisation, elle concerne une formulation pharmaceutique caractérisée en ce que la concentration en insuline est 1800 µM (300 UI/mL).

Dans un mode de réalisation, elle concerne une formulation pharmaceutique caractérisée en ce que la concentration en insuline est 2400 µM (400 UI/mL).

Dans un mode de réalisation, elle concerne une formulation pharmaceutique caractérisée en ce que la concentration en insuline est 3000 µM (500 UI/mL).

L'invention concerne l'utilisation d'au moins un composé anionique substitué, ledit composé étant constitué d'un squelette saccharidique formé d'un nombre discret u compris entre 1 et 8 (1 ≤ u ≤ 8) d'unités saccharidiques identiques ou différentes, liées par des liaisons glycosidiques identiques ou différentes, lesdites unités saccharidiques étant choisies dans le groupe constitué par les hexoses, sous forme cyclique ou sous forme réduite ouverte, ledit composé comportant des groupes fonctionnels carboxyles partiellement substitués, les groupes fonctionnels carboxyles non substitués étant salifiables pour préparer une formulation pharmaceutique d'insuline humaine, en combinaison avec un composé polyanionique, permettant, après administration, d'accélérer le passage de l'insuline dans le sang et de réduire plus rapidement la glycémie par rapport à une formulation exempte de composé anionique substitué, et éventuellement de composés anioniques.

Dans un mode de réalisation, l'invention concerne l'utilisation d'au moins un composé anionique substitué, ledit composé comportant des groupes fonctionnels carboxyles partiellement substitués, les groupes fonctionnels carboxyles non substitués étant salifiables, pour préparer une formulation pharmaceutique d'insuline humaine, en combinaison avec un composé polyanionique, permettant, après administration, d'accélérer le passage de l'insuline humaine dans le sang et de réduire plus rapidement la glycémie par rapport à une formulation exempte de composé anionique substitué, et éventuellement de composés anioniques.

Dans un mode de réalisation, l'invention concerne l'utilisation d'au moins un composé anionique substitué, ledit composé comportant des groupes fonctionnels carboxyles partiellement substitués, les groupes fonctionnels carboxyles non substitués étant salifiables, pour préparer une formulation d'insuline analogue, en combinaison avec un composé polyanionique, permettant, après administration, d'accélerer le passage de l'insuline analogue dans le sang et de réduire plus rapidement la glycémie par rapport à une formulation exempte de composé anionique substitué, et éventuellement de composés anioniques.
Utilisation d'au moins un composé anionique fonctionnalisé constitués d'un squelette formé d'un nombre discret u compris entre 1 et 3 (1 ≤ u≤ 3) d'unités saccharidiques identiques ou différentes, liées par des liaisons glycosidiques identiques ou différentes, lesdites unités saccharidiques étant choisies dans le groupe constitué par, les hexoses, sous forme cyclique ou sous forme réduite ouverte, ledit composé comportant des groupes fonctionnels carboxyles partiellement substitués, les groupes fonctionnels carboxyles non substitués étant salifiables pour préparer une formulation pharmaceutique d'insuline permettant, après administration, d'accélérer le passage de l'insuline dans le sang et de réduire plus rapidement la glycémie par rapport à une formulation exempte de composé anionique substitué en association avec au moins ledit composé polyanionique étant un composé polyanionique non polymérique (PNP) dont l'affinité pour le zinc est inférieure à l'affinité pour le zinc de l'insuline et dont la constante de dissociation KdCa =[composé PNP]^{r} [Ca²⁺]^{s}/[(composé PNP)ᵣ-(Ca²⁺)s] inférieure ou égale à 10^{-1,5}. 25
Dans un mode de réalisation, l'utilisation est caractérisée en ce que le composé anionique fonctionnalisé est en mélange avec un composé polyanionique.

Dans un mode de réalisation, l'insuline est l'insuline humaine.

On entend par insuline humaine une insuline obtenue par synthèse ou recombinaison dont la séquence peptidique est la séquence de l'insuline humaine, incluant les variations alléliques et les homologues.

Dans un mode de réalisation, l'insuline est une insuline humaine recombinante telle que décrite dans la Pharmacopée Européenne et la Pharmacopée américaine.

Dans un mode de réalisation, l'insuline est une insuline analogue.

On entend par insuline analogue une insuline recombinante dont la séquence primaire contient au moins une modification par rapport à la séquence primaire de l'insuline humaine.

Dans un mode de réalisation l'insuline analogue est choisie dans le groupe constitué par l'insuline lispro (Humalog®), l'insuline aspart (Novolog®, Novorapid®) et l'insuline glulisine (Apidra®).

Dans un mode de réalisation, l'insuline analogue est l'insuline lispro (Humalog®).

Dans un mode de réalisation, l'Insuline analogue est l'insuline aspart (Novolog®, Novorapid®).

Dans un mode de réalisation, l'Insuline analogue est l'insuline glulisine (Apidra®).

Dans un mode de réalisation, l'utilisation est caractérisée en ce que le composé anionique substitué, est choisi parmi les composés anioniques substitués à l'état isolé ou en mélange, constitués d'un squelette saccharidique formé d'un nombre discret u compris entre 1 et 8 (1 ≤ u ≤ 8) d'unités saccharidiques identiques ou différentes, liées par des liaisons glycosldlques identiques ou différentes, lesdites unités saccharidiques étant choisies parmi les hexoses, sous forme cyclique ou sous forme réduite ouverte, caractérisés en ce qu'ils sont substitués par :
a) au moins un substituant de formule générale I :

   -[R₁]ₐ-[AA]ₘ Formule I

   - les substituants étant identiques ou différents lorsqu'il y a au moins deux substituants, dans lequel ;
   - le radical -[AA] désigne un résidu d'acide aminé,
   - le radical -R₁- étant :
      - soit une liaison et alors a = 0, et le résidu d'acide aminé -[AA]- est directement lié au squelette par une fonction G.
      - soit une chaîne carbonée et alors a = 1, en C2 à C15 éventuellement substituée et/ou comportant au moins un hétéroatome choisi parmi O, N et S et au moins une fonction acide avant la réaction avec l'acide aminé, ladite chaîne formant avec le résidu d'acide aminé -[AA]- une fonction amide, et est fixée sur le squelette à l'aide d'une fonction F résultant d'une réaction entre une fonction hydroxyle portée par le squelette et une fonction ou un substituant porté par le précurseur du radical -R₁-,
   - F est une fonction choisie parmi les fonctions éther, ester ou carbamate,
   - G est une fonction carbamate,
   - m est égal à 1 ou 2,
   - le degré de substitution des unités saccharidiques, j, en -[R₁]ₐ-[AA]ₘ -étant strictement supérieur à 0 et inférieur ou égal à 6, 0 < j ≤ 6
b) et, éventuellement, un ou plusieurs substituants -R'₁,
   - le substituant -R'₁ étant une chaîne carbonée en C2 à C15, éventuellement substituée et/ou comportant au moins un hétéroatome choisie parmi O, N et S et au moins une fonction acide sous forme de sel de cations alcalins ladite chaine étant liée au squelette par une fonction F' résultant d'une réaction entre une fonction hydroxyle portée par le squelette et une fonction ou un substituant porté par le précurseur du substituant -R'₁,
   - F' est une fonction éther, ester, ou carbamate,
   - le degré de substitution des unités saccharidiques, I, en -R'₁, étant compris entre 0 et 6-j, 0 ≤ i ≤ 6-j et,
   - F et F' sont identiques ou différentes,
   - F et G sont identiques ou différentes,
   - i+j ≤ 6.
   - -R'₁ identique ou différent de -R₁-,
   - Les fonctions acides salifiables libres portées par le substituant -R'1 sont sous forme de sels de cations alcalins,
   - lesdites liaisons glycosidiques identiques ou différentes étant choisies dans le groupe constitué par les liaisons glycosidiques de type (1,1), (1,2), (1,3), (1,4) ou (1,6), dans une géométrie alpha ou béta,

Dans un mode de réalisation, l'utilisation est caractérisée en ce que le composé anionique substitué, à l'état isolé ou en mélange, est choisi parmi les composés anioniques substitués constitués d'un squelette saccharidique formé d'un nombre discret u compris entre 1 et 8 (1 ≤ u ≤ 8) d'unités saccharidiques identiques ou différentes, liées par des liaisons glycosidiques identiques ou différentes, lesdites unités saccharidiques étant choisies parmi les hexoses, sous forme cyclique ou sous forme réduite ouverte, caractérisés :
a) en ce qu'ils sont substitués de façon statistique par :
   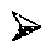 au moins un substituant de formule générale I :

      -[R₁]ₐ-[AA]ₘ Formule I

      - les substituants étant identiques ou différents lorsqu'il y a au moins deux substituants, dans lequel :
      - le radical -[AA]- désigne un résidu d'acide aminé, ledit acide aminé étant choisi dans le groupe constitué de la phénylalanine, l'alpha-méthyl-phénylalanine, la 3,4 dihydroxyphénylalanine, la tyrosine, l'alpha-méthyl-tyrosine, la O-méthyl-tyrosine, l'alpha-phénylglycine, la 4-hydroxyphénylglycine, la 3,5-dihydroxyphénylglycine et leurs sels de cations alcalins, lesdits dérivés étant de configuration absolue L ou D, -[AA]- est fixé sur le squelette de la molécule par l'intermédiaire d'un bras de liaison -R₁- ou directement lié au squelette par une fonction G,
      - -R₁- étant :
         - soit une liaison G, et alors a = 0,
         - soit une chaîne carbonée, et alors a = 1, en C2 à C15 éventuellement substituée et/ou comportant au moins un hétéroatome choisi parmi O, N et S et portant au moins une fonction acide avant la réaction avec l'acide aminé, ladite chaîne formant avec le résidu d'acide aminé -[AA]- une liaison amide, et est fixée sur le squelette saccharidique à l'aide d'une fonction F résultant d'une réaction entre une fonction hydroxyle portée par le squelette et une fonction portée par le précurseur de R₁,
      - F est une fonction éther, ester ou carbamate,
      - G est une fonction carbamate,
      - m est égal à 1 ou 2,
      - le degré de substitution, j, en -[R₁]ₐ-[AA]ₘ étant strictement supérieur à 0 et inférieur ou égal à 6, 0 < j ≤ 6,
      et, éventuellement, 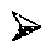
   un ou plusieurs substituants -R'₁
      - -R'₁ étant une chaîne carbonée en C2 à C15, éventuellement substituée et/ou comportant au moins un hétéroatome (tels que O, N et S) et portant au moins une fonction acide sous forme de sel de cations alcalins ladite chaîne étant fixée sur le squelette saccharidique par une fonction F' résultant d'une réaction entre une fonction hydroxyle portée par le squelette et une fonction portée par le précurseur de -R'₁,
      - F' est une fonction éther, ester ou carbamate,
      - le degré de substitution i, en -R'₁, étant compris entre 0 et 6-j, 0 ≤ i ≤ 6-j, et,
      - -R'₁- identique ou différent de -R₁,
      - F et F' identiques ou différentes,
      - F' et G identiques ou différentes
      - Les fonctions acides salifiables libres sont sous forme de sels de cations alcalins,
b) lesdites liaisons glycosidiques identiques ou différentes étant choisies dans le groupe constitué par les liaisons glycosidiques de type (1,1), (1,2), (1,3), (1,4) ou (1,6), dans une géométrie alpha ou béta,
c) i + j ≤ 6

Dans un mode de réalisation, m est égal à 1.

Dans un mode de réalisation, -R1 et -R'1, identiques ou différents sont une chaîne carbonée en C1 à C8.

Dans un mode de réalisation, -R1 et -R'1, identiques ou différents sont une chaîne carbonée en C1 à C4.

Dans un mode de réalisation, -R1 et -R'1, identiques ou différents sont une chaîne carbonée en C1 à C2.

Il est connu de l'homme de l'art que le délai d'action des insulines est dépendant de la concentration en insuline. Seules les valeurs de délai d'action des formulations à 100 UI/mL sont documentées.

Les formulations d'insuline humaine « regular » sur le marché à une concentration de 600 µM (100 UI/mL) ont un délai d'action compris entre 50 et 90 minutes et une fin d'action d'environ 360 à 420 minutes chez l'humain. Le temps pour atteindre la concentration maximale en insuline dans le sang est compris entre 90 et 180 minutes chez l'humain.

Les formulations d'insulines analogues rapides sur le marché à une concentration de 600 µM (100 UI/mL) ont un délai d'action compris entre 30 et 60 minutes et une fin d'action d'environ 240-300 minutes chez l'humain. Le temps pour atteindre la concentration maximale en insuline dans le sangest compris entre 50 et 90 minutes chez l'humain.

L'invention concerne également une méthode de préparation d'une formulation d'insuline humaine ayant une concentration en insuline comprise entre 240 et 3000 µM (40 et 500 UI/mL), dont le délai d'action chez l'humain est inférieur à celui de la formulation de référence à la même concentration en insuline en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline humaine ayant une concentration en insuline comprise entre 600 et 1200 µM (100 et 200 UI/mL), dont le délai d'action chez l'humain est inférieur à celui de la formulation de référence à la même concentration en insuline en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline humaine ayant une concentration en insuline de 600 µM (100 UI/mL), dont le délai d'action chez l'humain est inférieur à 60 minutes caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline humaine ayant une concentration en insuline de 1200 µM (200 UI/mL), dont le délai d'action chez l'humain est inférieur d'au moins 10% à celui de la formulation de l'insuline humaine à la même concentration (200 UI/mL) et en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline humaine ayant une concentration en insuline de 1800 µM (300 UI/mL), dont le délai d'action chez l'humain est inférieur d'au moins 10% à celui de la formulation de l'insuline humaine à la même concentration (300 UI/mL) et en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline humaine ayant une concentration en insuline de 2400 µM (400 UI/mL), dont le délai d'action chez l'humain est inférieur d'au moins 10% à celui de la formulation de l'insuline humaine à la même concentration (400 UI/mL) et en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline humaine ayant une concentration en insuline de 3000 µM (500 UI/mL), dont le délai d'action chez l'humain est inférieur d'au moins 10% à celui de la formulation de l'insuline humaine à la même concentration (500 UI/mL) et en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention consiste en la préparation d'une formulation d'insuline humaine dite rapide caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline humaine à une concentration de 600 µM (100 UI/mL) dont le délai d'action chez l'humain est inférieur à 60 minutes, de préférence inférieur à 45 minutes, et encore de préférence inférieur à 30 minutes caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline analogue ayant une concentration en insuline comprise entre 240 et 3000 µM (40 et 500 UI/mL), dont le délai d'action chez l'humain est inférieur à celui de la formulation de référence à la même concentration en insuline en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline analogue ayant une concentration en insuline comprise entre 600 et 1200 µM (100 et 200 UI/mL), dont le délai d'action chez l'humain est inférieur à celui de la formulation de référence à la même concentration en insuline analogue en l'absence de composé anionique substitué composé anionique substitué et de composé polyanionique, caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline analogue ayant une concentration en insuline de 600 µmol/L (100 UI/mL), dont le délai d'action chez l'humain est inférieur à 30 minutes, caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline analogue ayant une concentration en insuline de 1200 µM (200 UI/mL), dont le délai d'action chez l'humain est inférieur d'au moins 10% à celui de la formulation de l'insuline analogue en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline analogue ayant une concentration en insuline de 1800 µM (300 UI/mL), dont le délai d'action chez l'humain est inférieur d'au moins 10% à celui de la formulation de l'insuline analogue en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline analogue ayant une concentration en insuline de 2400 µM (400 UI/mL), dont le délai d'action chez l'humain est inférieur d'au moins 10% à celui de la formulation de l'insuline analogue en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention concerne également une méthode de préparation d'une formulation d'insuline analogue ayant une concentration en insuline de 3000 µM (500 UI/mL), dont le délai d'action chez l'humain est inférieur d'au moins 10% à celui de la formulation de l'insuline analogue en l'absence de composé anionique substitué et de composé polyanionique caractérisée en ce qu'elle comprend (1) une étape d'addition à ladite formulation d'au moins un composé anionique substitué, ledit composé comportant des groupes fonctionnels carboxyles partiellement substitués, les groupes fonctionnels carboxyles non substitués étant salifiables, et (2) une étape d'addition à ladite formulation d'au moins un composé polyanionique.

Dans un mode de réalisation, l'insuline est sous forme hexamèrique.

L'invention consiste en la préparation d'une formulation d'insuline analogue dite très rapide caractérisée en ce qu'elle comprend une étape d'addition à ladite formulation d'au moins un composé anionique substitué, ledit composé comportant des groupes fonctionnels carboxyles partiellement substitués, les groupes fonctionnels carboxyles non substitués étant salifiables

Dans un mode de réalisation, la préparation comprend en outre une étape d'addition à ladite formulation d'au moins un composé polyanionique.

Dans un mode de réalisation, l'insuline est sous forme hexamèrique.

Dans un mode de réalisation l'insuline analogue est choisie dans le groupe constitué par l'insuline lispro (Humalog®), l'insuline aspart (Novolog®, Novorapid®) et l'insuline glulisine (Apidra®).

Dans un mode de réalisation, l'insuline analogue est l'insuline lispro (Humalog®).

Dans un mode de réalisation, l'insuline analogue est l'insuline aspart (Novolog®, Novorapid®).

Dans un mode de réalisation, l'insuline analogue est l'insuline glulisine (Apidra®).

Dans un mode de réalisation, l'insuline est une insuline humaine recombinante telle que décrite dans la Pharmacopée Européenne et la Pharmacopée américaine.

Dans un mode de réalisation, l'insuline est une insuline analogue choisie dans le groupe constitué par l'insuline lispro (Humalog®), l'insuline aspart (Novolog® , Novorapid®) et l'insuline glulisine (Apidra®).

La composition peut de plus être réalisée par simple mélange d'une solution aqueuse d'insuline humaine ou analogue et d'une solution aqueuse de composé anionique substitué en mélange avec un composé polyanionique.

Dans un mode de réalisation, la composition peut être réalisée par simple mélange d'une solution aqueuse d'insuline humaine ou analogue, d'une solution aqueuse de composé anionique substitué et de composé polyanionique en solution ou sous forme de lyophilisat.

Dans un mode de réalisation, la composition peut être réalisée par simple mélange d'une solution aqueuse d'insuline humaine ou analogue, de composé anionique substitué sous forme de lyophilisat et de composé polyanionique en solution ou sous forme de lyophilisat.

De préférence cette composition est sous forme d'une solution injectable.

Dans un mode de réalisation, la concentration en insuline humaine ou analogue est comprise entre 240 et 3000 µM (40 à 500 UI/mL).

Dans un mode de réalisation, la concentration en insuline humaine ou analogue est comprise entre 600 et 3000 µM (100 à 500 UI/mL).

Dans un mode de réalisation, la concentration en insuline humaine ou analogue est comprise entre 600 et 2400 µM (100 à 400 UI/mL).

Dans un mode de réalisation, la concentration en insuline humaine ou analogue est comprise entre 600 et 1800 µM (100 à 300 UI/mL).

Dans un mode de réalisation, la concentration en insuline humaine ou analogue est comprise entre 600 et 1200 µM (100 à 200 UI/mL).

Dans un mode de réalisation, la concentration en insuline humaine ou analogue est 600 µM (100 UI/mL).

Dans un mode de réalisation, la concentration en insuline humaine ou analogue est 1200 µM (200 UI/mL).

Dans un mode de réalisation, la concentration en insuline humaine ou analogue de 600 µM (100 UI/mL) peut être réduite par simple dilution, en particulier pour les applications pédiatriques.

Dans un mode de réalisation, la concentration en insuline humaine ou analogue est 1800 µM (300 UI/mL).

Dans un mode de réalisation, la concentration en insuline humaine ou analogue est 2400 µM (400 UI/mL).

Dans un mode de réalisation, la concentration en insuline humaine ou analogue est 3000 µM (500 UI/mL).

L'invention concerne également une formulation pharmaceutique selon l'invention, caractérisée en ce qu'elle est obtenue par séchage et/ou lyophilisation.

Dans un mode de réalisation, les compositions selon l'invention comprennent en outre l'addition de sels de zinc à une concentration comprise entre 0 et 500 µM.

Dans un mode de réalisation, les compositions selon l'invention comprennent en outre l'addition de sels de zinc à une concentration comprise entre 0 et 300 µM.

Dans un mode de réalisation, les compositions selon l'invention comprennent en outre l'addition de sels de zinc à une concentration comprise entre 0 et 200 µM.

Dans un mode de réalisation, les compositions selon l'invention comprennent des tampons à des concentrations comprises entre 0 et 100 mM, de préférence entre 0 et 50 mM ou entre 15 et 50 mM.

Dans un mode de réalaistion le tampon est le Tris.

Dans un mode de réalisation, les compositions selon l'invention comprennent en outre des conservateurs.

Dans un mode de réalisation, les conservateurs sont choisis dans le groupe constitué par le m-crésol et le phénol seuls ou en mélange.

Dans un mode de réalisation, la concentration des conservateurs est comprise entre 10 et 50 mM.

Dans un mode de réalisation, la concentration des conservateurs est comprise entre 10 et 40 mM.

Les compositions selon l'invention peuvent en outre comprendre des additifs tels que des agents de tonicité comme la glycérine, le chlorure de sodium (NaCl), le mannitol et la glycine.

Les compositions selon l'invention peuvent en outre comprendre des additifs conformes aux pharmacopées comme des tensloactlfs par exemple du polysorbate.

Les compositions selon l'invention peuvent comprendre en outre tous les excipients conformes aux pharmacopées et compatibles avec les insulines utilisées aux concentrations d'usage.

Dans le cas des libérations locale et systémique, les modes d'administration envisagés sont par vole intraveineuse, sous-cutanée, intradermique ou intramusculaire.

Les voles d'administration transdermique, orale, nasale, vaginale, oculaire, buccale, pulmonaire sont également envisagées.

L'invention concerne également l'utilisation d'une composition selon l'invention pour la formulation d'une solution d'insuline humaine ou analogue de concentration de 100 UI/mL destinée aux pompes à insuline implantables ou transportables.

L'invention concerne également l'utilisation d'une composition selon l'invention pour la formulation d'une solution d'insuline humaine ou analogue de concentration de 200 UI/mL destinée aux pompes à insuline implantables ou transportables.

L'invention concerne également les composés anioniques substitués, à l'état isolé ou en mélange, choisis parmi les composés anioniques substitués constitués d'un squelette saccharidique formé d'un nombre discret u compris entre 1 et 8 (1 ≤ u ≤ 8) d'unités saccharidiques identiques ou différentes, liées par des liaisons glycosidiques identiques ou différentes, lesdites unités saccharldiques étant choisies parmi les hexoses, sous forme cyclique ou sous forme réduite ouverte, caractérisés en ce qu'ils sont substitués par :
a) au moins un substituant de formule générale I :

   -[R₁]ₐ-[AA]ₘ Formule I

   - les substituants étant identiques ou différents lorsqu'il y a au moins deux substituants, dans lequel :
      - le radical -[AA] désigne un résidu d'acide aminé,
      - le radical -R₁- étant :
         - soit une liaison et alors a = 0, et le résidu d'acide aminé -[AA]- est directement lié au squelette par une fonction G.
         - soit une chaîne carbonée et alors a = 1, en C2 à C15 éventuellement substituée et/ou comportant au moins un hétéroatome choisi parmi O, N et S et au moins une fonction acide avant la réaction avec l'acide aminé, ladite chaîne formant avec le résidu d'adde aminé -[AA]- une fonction amide, et est fixée sur le squelette à l'aide d'une fonction F résultant d'une réaction entre une fonction hydroxyle portée par le squelette et une fonction ou un substituant porté par le précurseur du radical -R₁-,
   - F est une fonction choisie parmi les fonctions éther, ester ou carbamate,
   - G est une fonction carbamate,
   - m est égal à 1 ou 2,
   - le degré de substitution des unités saccharidiques, j, en -[R₁]ₐ-[AA]ₘ, -étant strictement supérieur à 0 et inférieur ou égal à 6, 0 < j ≤ 6
b) et, éventuellement, un ou plusieurs substituants -R'₁,
   - le substituant -R'₁ étant une chaîne carbonée en C2 à C15, éventuellement substituée et/ou comportant au moins un hétéroatome choisi parmi O, N et S et au moins une fonction acide sous forme de sel de cations alcalins ladite chaine étant liée au squelette par une fonction F' résultant d'une réaction entre une fonction hydroxyle portée par le squelette et une fonction au un substituant porté par le précurseur du substituant -R'₁,
   - F' est une fonction éther, ester ou carbamate,
   - le degré de substitution des unités saccharidiques, i, en -R'₁, étant compris entre 0 et 6-j, 0 ≤ i ≤ 6-j et,
   - F et F' sont identiques ou différentes,
   - F et G sont identiques ou différentes,
   - l+j ≤6.
   - -R'₁ identique ou différent de -R₁-,
   - Les fonctions acides salifiables libres portées par le substituant -R'1 sont sous forme de sels de cations alcalins,
   - lesdites liaisons glycosidiques identiques ou différentes étant choisies dans le groupe constitué par les liaisons glycosidiques de type (1,1), (1,2), (1,3), (1,4) ou (1,6), dans une géométrie alpha ou béta,

Dans la formule ci-dessus les différentes variables ont les valeurs citées ci-dessus.

Les composés anioniques substitués selon l'invention peuvent être obtenus par greffage statistique des substituants sur le squelette saccharidique.

Dans un mode de réalisation, les composés anioniques substitués choisis parmi les composés anioniques substitués par des substituants de formules I ou II sont caractérisés en ce qu'ils peuvent être obtenus par greffage des substituants à des positions précises sur les unités saccharidiques par un procédé mettant en oeuvre des étapes de protection/déprotection des groupements alcool ou acide carboxylique naturellement portés par le squelette. Cette stratégie conduit à un greffage sélectif, notamment réglosélectlf, des substituants sur le squelette. Les groupements protecteurs incluent sans limitation ceux décrits dans l'ouvrage (Wuts, PGM et al., Greene's Protective Groups in Organic Synthesls 2007).

Le squelette saccharidique peut être obtenu par dégradation d'un polysaccharide de haut poids moléculaire. Les voles de dégradation incluent sans limitation la dégradation chimique et/ou la dégradation enzymatique.

Le squelette saccharidique peut également être obtenu par formation de liaisons glycosidiques entre des molécules monosacchariques ou oligosaccharidiques en utilisant une stratégie de couplage chimique ou enzymatique. Les stratégies de couplage comptent celles décrites dans la publication (Smoot, JT et al., Advances in Carbohydrate Chemistry and Biochemistry 2009, 62, 162-250) et dans l'ouvrage (Lindhorst, TK, Essentials of Carbohydrate Chemistry and Biochemistry 2007, 157-208). Les réactions de couplage peuvent être effectuées en solution ou sur support solide. Les molécules saccharidiques avant couplage peuvent porter des substituants d'intérêt et/ou être fonctionnalisées une fois couplées entre elles de façon statistique ou régiosélective.

Ainsi, à titre d'exemples, les composés selon l'invention peuvent être obtenus selon l'un des procédés suivants :
▪ le greffage statistique des substituants sur un squelette saccharidique
▪ une ou plusieurs étapes de glycosylation entre des molécules mono-saccharidiques ou oligosaccharidiques portant des substituants
▪ une ou plusieurs étapes de glycosylation entre une ou des molécules mono-saccharidiques ou oligosaccharidiques portant des substituants et une ou des molécules mono-saccharidiques ou oligosaccharidiques
▪ une ou plusieurs étapes d'introduction de groupements protecteurs sur des alcools ou acides naturellement portés par le squelette saccharidique suivie d'une ou plusieurs réactions de greffage des substituants et enfin une étape d'élimination des groupements protecteurs
▪ une ou plusieurs étapes de glycosylation entre une ou des molécules mono-saccharidiques ou oligosaccharidiques portant des groupements protecteurs sur des alcools ou acides naturellement portés par le squelette saccharidique, une ou des étapes de greffage de substituants sur le squelette obtenu puis une étape d'élimination des groupements protecteurs
▪ une ou plusieurs étapes de glycosylation entre une ou des molécules mono-saccharidiques ou oligosaccharidiques portant des groupements protecteurs sur des alcools ou acides naturellement portés par le squelette saccharidique, et une ou des molécules mono-saccharidiques ou oligosaccharidiques, une ou des étapes de greffage de substituants puis une étape d'élimination des groupements protecteurs.

Les composés selon l'invention, isolés ou en mélange, peuvent être séparés et/ou purifiés de différentes manières, notamment après leur obtention par les procédés ci-dessus décrits.

On peut en particulier citer les méthodes chromatographiques, notamment celles dites « préparatives » ou « préparatrices » comme :
▪ les chromatographies éclairs ou « flash chromatography », notamment sur silice, et
▪ Les chromatographies du type HPLC (high performance liquid chromatography) (Chromatographie liquide haute performance), en particulier RP-HPLC ou « reverse phase HPLC » (Chromatographie liquide haute performance en phase inverse).

Des méthodes de précipitation sélective peuvent également être utilisées.

L'invention est illustrée par les exemples suivants.

### Exemples

Les structures des composés anioniques substitués selon l'invention sont présentées dans le Tableau 1. Les structures des polysaccharides contre-exemples sont présentées dans le Tableau 2.

### AA Composés anioniques substitués

R = H, R'₁, -[R₁]ₐ-[AA]ₘ,

**Tableau 1**

| Composé | i | j | Enchaînement saccharidique | Substituant -R'₁ | Substituant -[R₁]ₐ-[AA]ₘ |
|---|---|---|---|---|---|
| 1 | 0,65 | 1,0 | 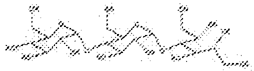 | 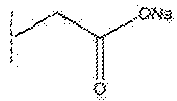 | 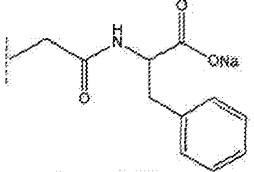 |
| 2 | 1,0 | 0,65 | 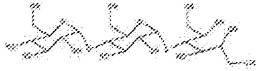 | 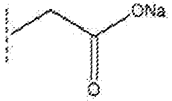 | 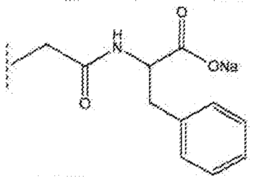 |
| 3 | 0,46 | 1,2 | 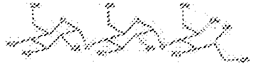 | 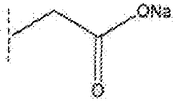 | 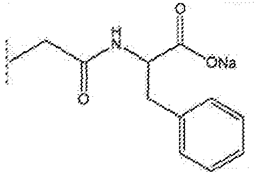 |
| 4 | 0,35 | 0,65 | 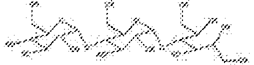 | 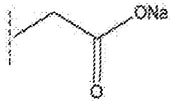 | 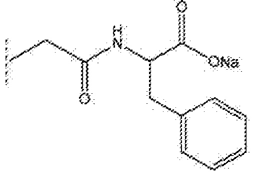 |
| 5 | 1,25 | 0,4 | 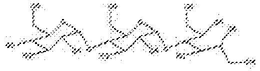 | 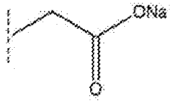 | 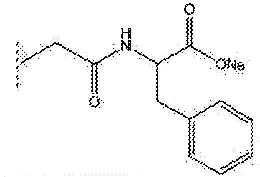 |
| 6 | 0,8 | 0,65 | 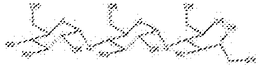 | 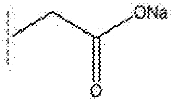 | 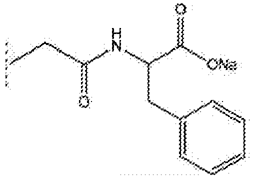 |
| 7 | 2,65 | 0,65 | 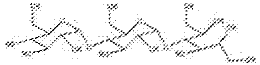 | 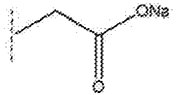 | 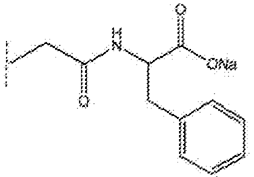 |
| 8 | 1,0 | 0,75 | 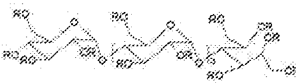 | 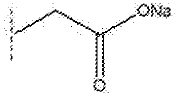 | 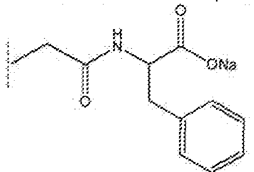 |
| 9 | 1,0 | 0,65 | 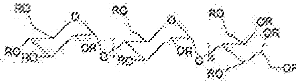 | 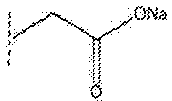 | 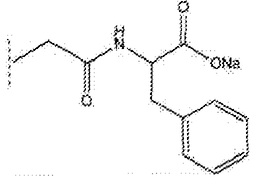 |
| 10 | 0,83 | 0,81 | 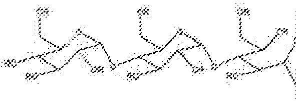 | 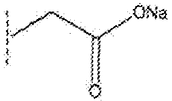 | 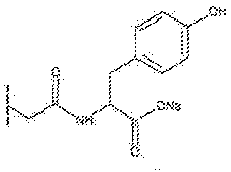 |
| 11 | 1,12 | 0,52 | 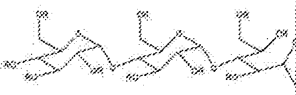 | 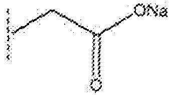 | 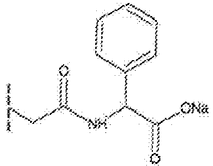 |

### AB Polysaccharides contre-exemples

**Tableau 2**

| Polysaccharides contre-exemples | i | j | Enchaînement saccharidique | Masse molaire moyenne en poids (kg/mol) | Substituant -R'₁ | Substituant - [R₁]ₐ-[AA]ₘ |
|---|---|---|---|---|---|---|
| Polysaccharides contre-exemples AB1, AB2, AB3, AB4 et AB5: R = H, R'₁, -[R₁]a-[AA]ₘ | | | | | | |
| AB1 | 0,6 | 0,46 | 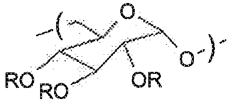 | 10 | 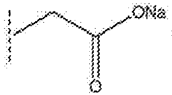 | 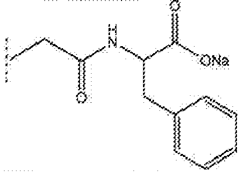 |
| AB2 | 1,01 | 0,64 | 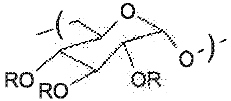 | 5 | 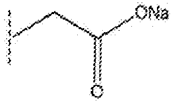 | 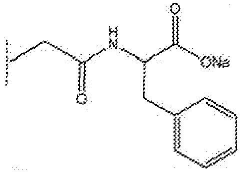 |
| AB3 | 0,65 | 0,45 | 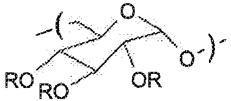 | 5 | 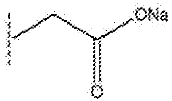 | 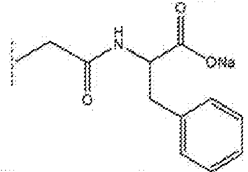 |
| AB4 | 1,01 | 0,64 | 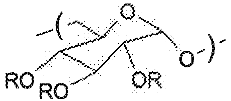 | 10 | 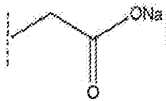 | 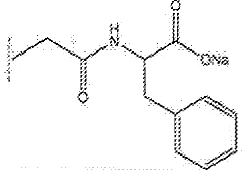 |
| AB5 | 0,45 | 0,65 | 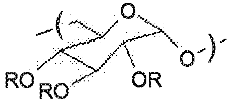 | 5 | 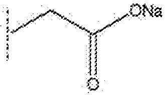 | 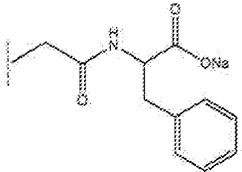 |

### AA1. Composé 1 : Maltotrioseméthylcarboxylate de sodium fonctionnalisé par le L-phénylalaninate de sodium

A 8 g (143 mmol de fonctions hydroxyles) de maltotriose (CarboSynth) dissouts dans de l'eau à 65°C est ajouté 0,6 g (16 mmol) de borohydrure de sodium. Après 30 min d'agitation, 28 g (238 mmol) de chloroacétate de sodium sont ajoutés. A cette solution sont ensuite ajoutés goutte à goutte 24 mL de NaOH 10 N (24 mmol) puis le mélange est chauffé à 65°C pendant 90 minutes. 16,6 g (143 mmol) de chloroacétate de sodium sont ensuite ajoutés au milieu réactionnel ainsi que 14 mL de NaOH 10N (14 mmol) au goutte à goutte. Après 1h de chauffage, le mélange est dilué avec de l'eau, neutralisé avec de l'acide acétique puis purifié par ultrafiltration sur membrane PES de 1 kDa contre de l'eau. La concentration en molécule de la solution finale est déterminée par extrait sec, puis un dosage acide/base dans un mélange eau/acétone 50/50 (V/V) est effectué pour déterminer le degré de substitution en méthylcarboxylate.
D'après l'extrait sec : [composé] = 32,9 mg/g
D'après le dosage acide/base, le degré de substitution en méthylcarboxylate est de 1,65 par unité saccharidique.

La solution de maltotrioseméthylcarboxylate de sodium est acidifiée sur une résine Purolite (anionique) pour obtenir l'acide maltotrioseméthylcarboxylique qui est ensuite lyophilisé pendant 18 heures.

10 g d'acide maltotrioseméthylcarboxylique (63 mmol de fonctions acide méthylcarboxylique) sont solubilisés dans le DMF puis refroidis à 0°C. Un mélange de phénylalaninate d'éthyle, sel d'hydrochlorure (8,7 g, 38 mmol) dans du DMF est préparé. 3,8 g de triéthylamine (38 mmol) sont ajoutés à ce mélange. Une solution de NMM (6,3 g, 63 mmol) et de EtOCOCl (6,8 g, 63 mmol) est ensuite ajoutée au mélange à 0°C. La solution de phénylalaninate d'éthyle est ensuite ajoutée et le mélange agité à 10°C. Une solution aqueuse d'imidazole est ajoutée puis le mélange chauffé à 30°C. Le milieu est dilué avec de l'eau puis la solution obtenue est purifiée par ultrafiltration sur membrane PES de 1 kDa contre NaOH 0,1 N, NaCl 0,9% et de l'eau. La concentration en molécule de la solution finale est déterminée par extrait sec. Un échantillon de solution est lyophilisé et analysé par RMN ¹H dans D₂O pour déterminer le degré de substitution en méthylcarboxylates fonctionnalisés par le L-phénylalaninate de sodium.
D'après l'extrait sec : [composé 1] = 29,4 mg/g
D'après le dosage acide/base, le degré de substitution en méthylcarboxylates de sodium par unité saccharidique est de 0,65.

D'après la RMN ¹H : le degré de substitution en méthylcarboxylates fonctionnalisés par le L-phénylalaninate de sodium par unité saccharidique est de 1,0.

### AA2. Composé 2 : Maltotrioseméthylcarboxylate de sodium fonctionnalisé par le L-phénylalaninate de sodium

Par un procédé similaire à celui utilisé pour la préparation du composé 1, un maltotriosecarboxylate de sodium fonctionnalisé par le L-phénylalaninate de sodium est obtenu. D'après le dosage acide/base, le degré de substitution en méthylcarboxylates de sodium par unité saccharidique est de 1,0.
D'après l'extrait sec : [composé 2] = 20,2 mg/g
D'après la RMN ¹H : le degré de substitution en méthylcarboxylates fonctionnalisés par le L-phénylalaninate de sodium par unité saccharidique est de 0,65.

### AA3. Composé 3 : Maltotrioseméthylcarboxylate de sodium fonctionnalisé par le L-phénylalaninate de sodium

Par un procédé similaire à celui utilisé pour la préparation du composé 1, un maltotriosecarboxylate de sodium fonctionnalisé par le L-phénylalaninate de sodium est obtenu. D'après le dosage acide/base, le degré de substitution en méthylcarboxylates de sodium par unité saccharidique est de 0,46.
D'après l'extrait sec : [composé 3] = 7,2 mg/g
D'après la RMN ¹H : le degré de substitution en méthylcarboxylates fonctionnalisés par le L-phénylalaninate de sodium par unité saccharidique est de 1,2.

### AA4. Composé 4 : Maltotrioseméthylcarboxylate de sodium fonctionnalisé par le L-phénylalaninate de sodium

Par un procédé similaire à celui utilisé pour la préparation du composé 1, un maltotriosecarboxylate de sodium fonctionnalisé par le L-phénylalaninate de sodium est obtenu. D'après le dosage acide/base, le degré de substitution en méthylcarboxylates de sodium par unité saccharidique est de 0,35.
D'après l'extrait sec : [composé 4] = 3,1 mg/g
[000388] D'après la RMN ¹H: le degré de substitution en méthylcarboxylates fonctionnalisés par le L-phénylalaninate de sodium par unité saccharidique est de 0,65.

### AA5. Composé 5 : Maltotrioseméthylcarboxylate de sodium fonctionnalisé par le L-phénylalaninate de sodium

Par un procédé similaire à celui utilisé pour la préparation du composé 1, un maltotrioseméthylcarboxylate de sodium fonctionnalisé par le L-phénylalaninate de sodium est obtenu.
D'après l'extrait sec : [composé 5] = 10,9 mg/g
D'après la RMN ¹H : le degré de substitution en méthylcarboxylates fonctionnalisés par le L-phénylalaninate de sodium par unité saccharidique est de 0,40.

Le degré de substitution en méthylcarboxylates de sodium par unité saccharidique est de 1,25.

### AA6. Composé 6 : Maltotrioseméthylcarboxylate de sodium fonctionnalisé par le L-phénylalaninate de sodium

A 8 g (143 mmol de fonctions hydroxyles) de maltotriose (CarboSynth) dissouts dans de l'eau à 65°C est ajouté 0,6 g (16 mmol) de borohydrure de sodium. Après 30 min d'agitation, 28 g (237 mmol) de chloroacétate de sodium sont ajoutés. A cette solution sont ensuite ajoutés goutte à goutte 24 mL de NaOH 10 N (240 mmol). Après chauffage à 65°C pendant 90 min, le mélange est dilué avec de l'eau, neutralisé par ajout d'acide acétique puis purifié par ultrafiltration sur membrane PES de 1 kDa contre de l'eau. La concentration en composé de la solution finale est déterminée par extrait sec, puis un dosage acide/base dans un mélange eau/acétone 50/50 (V/V) est effectué pour déterminer le degré de substitution en méthylcarboxylate de sodium.
D'après l'extrait sec : [composé] = 14,5 mg/g
D'après le dosage acide/base, le degré de substitution en méthylcarboxylates de sodium par unité saccharidique est de 1,45.

La solution de maltotrioseméthylcarboxylate de sodium est acidifiée sur une résine Purolite (anionique) pour obtenir l'acide maltotrioseméthylcarboxylique qui est ensuite lyophilisé pendant 18 heures.

Par un procédé similaire à celui utilisé pour la préparation du composé 1, un maltotrioseméthylcarboxylate de sodium fonctionnalisé par le L-phénylalaninate de sodium est obtenu.
D'après l'extrait sec : [composé 6] = 10,8 mg/g
D'après la RMN ¹H : le degré de substitution en méthylcarboxylates fonctionnalisés par le L-phénylalaninate de sodium par unité saccharidique est de 0,65.

Le degré de substitution en méthylcarboxylates de sodium par unité saccharidique est de 0,8.

### AA7. Composé 7 : Maltotrioseméthylcarboxylate de sodium fonctionnalisé par le L-phénylalaninate de sodium

Par un procédé similaire à celui décrit dans la préparation du composé 1, 8 g de maltotrioseméthylcarboxylate de sodium caractérisé par un degré de substitution en méthylcarboxylate de sodium de 1,76 sont synthétisés et lyophilisés.

8 g (58 mmol de fonctions hydroxyles) du lyophilisat et 15 g (129 mmol) de chloroacétate de sodium sont dissouts dans l'eau à 65°C. A cette solution sont ajoutés goutte à goutte 13 mL de NaOH 10 N (130 mmol) puis le mélange est chauffé à 65°C pendant 90 minutes. 9 g (78 mmol) de chloroacétate de sodium sont ensuite ajoutés au milieu réactionnel ainsi que 8 mL de NaOH 10N (80 mmol) au goutte à goutte. Après 1h de chauffage, le mélange est dilué avec de l'eau, neutralisé par l'acide acétique puis purifié par ultrafiltration sur membrane PES de 1 kDa contre de l'eau. La concentration en composé de la solution finale est déterminée par extrait sec, puis un dosage acide/base dans un mélange eau/acétone 50 / 50 (V/V) est effectué pour déterminer le degré de substitution en méthylcarboxylates de sodium.
D'après l'extrait sec : [composé] = 11,7 mg/g
D'après le dosage acide/base, le degré de substitution en méthylcarboxylates de sodium par unité saccharidique est de 3,30.

La solution de maltotrioseméthylcarboxylate de sodium est acidifiée sur une résine Purolite (anionique) pour obtenir l'acide maltotrioseméthylcarboxylique qui est ensuite lyophilisé pendant 18 heures.

Par un procédé similaire à celui utilisé pour la préparation du Composé 1, un maltotrioseméthylcarboxylate de sodium fonctionnalisé par le L-phénylalaninate de sodium est obtenu.
D'après l'extrait sec : [composé 7] = 14,9 mg/g
D'après la RMN ¹H : le degré de substitution en méthylcarboxylates fonctionnalisés par le L-phénylalaninate de sodium par unité saccharidique est de 0,65.

Le degré de substitution en méthylcarboxylates de sodium par unité saccharidique est de 2,65.

### AA8. Composé 8 Maltopentaoseméthylcarboxylate de sodium fonctionnalisé par le L-phénylalaninate de sodium

Par un procédé similaire à celui décrit dans la préparation du composé 1 mais conduit avec du maltopentaose (CarboSynth), 10 g d'acide maltopentaoseméthylcarboxylique de degré de substitution en acide méthylcarboxylique de 1,75 par unité saccharidique sont obtenus puis lyophilisés.

Par un procédé similaire à celui utilisé pour la préparation du composé 1, un maltopentaoseméthylcarboxylate de sodium fonctionnalisé par le L-phénylalaninate de sodium est obtenu.
D'après l'extrait sec : [composé 8] = 7,1 mg/g
D'après la RMN ¹H : le degré de substitution en méthylcarboxylates fonctionnalisés par le L-phénylalaninate de sodium par unité saccharidique est de 0,75.

Le degré de substitution en méthylcarboxylates de sodium par unité saccharidique est de 1,0.

### AA9. Composé 9 : Maltooctaoseméthylcarboxylate de sodium fonctionnalisé par le L-phénylalaninate de sodium

Par un procédé similaire à celui décrit dans la préparation du composé 1 mais conduit avec du maltooctaose (CarboSynth), 10 g d'acide maltooctaoseméthylcarboxylique de degré de substitution en acide méthylcarboxylique de 1,65 par unité saccharidique sont obtenus puis lyophilisés.

Par un procédé similaire à celui utilisé pour la préparation du composé 1, un maltooctaoseméthylcarboxylate de sodium fonctionnalisé par le L-phénylalaninate de sodium est obtenu.
D'après l'extrait sec : [composé 9] = 26,3 mg/g
D'après la RMN ¹H: le degré de substitution en méthylcarboxylates fonctionnalisés par le L-phénylalaninate de sodium par unité saccharidique est de 0,65.

Le degré de substitution en méthylcarboxylates de sodium par unité saccharidique est de 1,0.

### AA10. Composé 10: Maltotrioseméthylcarboxylate de sodium fonctionnalisé par le L-tyrosinate de sodium

Par un procédé similaire à celui décrit dans la préparation du composé 1 mais conduit avec le L-tyrosinate de méthyle, sel d'acide chlorhydrique (Bachem), un maltotrioseméthylcarboxylate de sodium, caractérisé par un degré de substitution en méthylcarboxylate de sodium par unité saccharidique de 1,64 est fonctionnalisé par le L-tyrosinate de sodium.
D'après l'extrait sec : [composé 10] = 9,1 mg/g
D'après la RMN ¹H: le degré de substitution en méthylcarboxylates fonctionnalisés par le L-tyrosinate de sodium par unité saccharidique est de 0,81.
Le degré de substitution en méthylcarboxylates de sodium par unité saccharidique est de 0,83.

### AA11. Composé 11: Maltotrioseméthylcarboxylate de sodium fonctionnalisé par l'alpha-phénylglycinate de sodium

Par un procédé similaire à celui décrit dans la préparation du composé 1, 10 g d'acide maltotrioseméthylcarboxylique de degré de substitution en acide méthylcarboxylique par unité saccharidique de 1,64 sont obtenus puis lyophilisés.

8 g d'acide maltotrioseméthylcarboxylique (50 mmol de fonctions acide méthylcarboxylique) sont solubilisés dans le DMF puis refroidis à 0°C. Un mélange d'alpha-phénylglycinate de sodium (Bachem, 5 g ; 33 mmol) et de triéthylamine (33 mmol) est préparé dans de l'eau. Une solution de NMM (4,9 g ; 49 mmol) et de EtOCOCl (5,3 g, 49 mmol) est ensuite ajoutée à la solution d'acide maltotrioseméthylcarboxylique à 0°C. La solution d'alpha-phénylglycinate de sodium et de triéthylamine est ensuite ajoutée et le mélange agité à 30°C. Une solution aqueuse d'imidazole (340 g/L) est ajoutée après 90 minutes. Le milieu est dilué avec de l'eau puis la solution obtenue est purifiée par ultrafiltration sur membrane PES de 1 kDa contre un tampon NaHCO₃/Na₂CO₃ pH 10,4 150 mM, NaCl 0,9% et de l'eau. La concentration en composé de la solution finale est déterminée par extrait sec. Un échantillon de solution est lyophilisé et analysé par RMN ¹H dans D₂O pour déterminer le degré de substitution en méthylcarboxylates fonctionnalisés par l'alpha-phénylglycinate de sodium.
D'après l'extrait sec : [composé 11] = 9,1 mg/g
D'après la RMN ¹H: le degré de substitution en méthylcarboxylates fonctionnalisés par l'alpha-phénylglycinate de sodium par unité saccharidique est de 0,52.

Le degré de substitution en méthylcarboxylates de sodium par unité saccharidique est de 1,12.

### AB Polysaccharides contre-exemples

### AB1. Polysaccharide 1 : Dextraneméthylcarboxylate de sodium fonctionnalisé par le L-phénylalaninate de sodium.

Le polysaccharide 1 est un dextraneméthylcarboxylate de sodium fonctionnalisé par le L-phénylalaninate de sodium obtenu à partir d'un dextrane de masse molaire moyenne en poids 10 kg/mol (DP = 39, Pharmacosmos) selon le procédé décrit dans la demande de brevet FR 07/02316 publiée sous le numéro FR2914305. D'après le dosage acide/base, le degré de substitution en méthylcarboxylates de sodium par unité saccharidique est de 0,6.

D'après la RMN ¹H : le degré de substitution en méthylcarboxylates fonctionnalisés par le L-phénylalaninate de sodium par unité saccharidique est de 0,46.

Ce polysaccharide correspond au polysaccharide 1 de la demande FR0901478.

### AB2. Polysaccharide 2 : Dextraneméthylcarboxylate de sodium fonctionnalisé par le L-phénylalaninate de sodium.

Le polysaccharide 2 est un dextraneméthylcarboxylate de sodium fonctionnalisé par le L-phénylalaninate de sodium obtenu à partir d'un dextrane de masse molaire moyenne en poids 5 kg/mol (DP = 19, Pharmacosmos) selon le procédé décrit dans la demande de brevet FR 07/02316 publiée sous le numéro FR2914305. D'après le dosage acide/base, le degré de substitution en méthylcarboxylates de sodium par unité saccharidique est de 1,01.

D'après la RMN ¹H : le degré de substitution en méthylcarboxylates fonctionnalisés par le L-phénylalaninate de sodium par unité saccharidique est de 0,64.

### AB3. Polysaccharide 3 : Dextraneméthylcarboxylate de sodium fonctionnalisé par le L-phénylalaninate de sodium.

Le polysaccharide 3 est un dextraneméthylcarboxylate de sodium fonctionnalisé par le L-phénylalaninate de sodium obtenu à partir d'un dextrane de masse molaire moyenne en poids 5 kg/mol (DP = 19, Pharmacosmos) selon le procédé décrit dans la demande de brevet FR 07/02316 publiée sous le numéro FR2914305. D'après le dosage acide/base, le degré de substitution en méthylcarboxylates de sodium par unité saccharidique est de 0,65.

D'après la RMN ¹H : le degré de substitution en méthylcarboxylates fonctionnalisés par le L-phénylalaninate de sodium par unité saccharidique est de 0,45.

### AB4. Polysaccharide 4 : Dextraneméthylcarboxylate de sodium fonctionnalisé par le L-phénylalaninate de sodium.

Le polysaccharide 4 est un dextraneméthylcarboxylate de sodium fonctionnalisé par le L-phénylalaninate de sodium obtenu à partir d'un dextrane de masse molaire moyenne en poids 10 kg/mol (DP = 39, Pharmacosmos) selon le procédé décrit dans la demande de brevet FR 07/02316 publiée sous le numéro FR2914305. D'après le dosage acide/base, le degré de substitution en méthylcarboxylates de sodium par unité saccharidique est de 1,01.

D'après la RMN ¹H : le degré de substitution en méthylcarboxylates fonctionnalisés par le L-phénylalaninate de sodium par unité saccharidique est de 0,64.

### AB5. Polysaccharide 5 : Dextraneméthylcarboxylate de sodium fonctionnalisé par le L-phénylalaninate de sodium.

Le polysaccharide 5 est un dextraneméthylcarboxylate de sodium fonctionnalisé par le L-phénylalaninate de sodium obtenu à partir d'un dextrane de masse molaire moyenne en poids 5 kg/mol (DP = 19, Pharmacosmos) selon le procédé décrit dans la demande de brevet FR 07/02316 publiée sous le numéro FR2914305. D'après le dosage acide/base, le degré de substitution en méthylcarboxylates de sodium par unité saccharidique est de 0,45.

D'après la RMN ¹H : le degré de substitution en méthylcarboxylates fonctionnalisés par le L-phénylalaninate de sodium par unité saccharidique est de 0,65.

### AC Composé polyanionique

### Composé polyanionique 1 : Maltotrioseméthylcarboxylate de sodium

A 8 g (143 mmol de fonctions hydroxyles) de maltotriose (CarboSynth) dissouts dans de l'eau à 65°C est ajouté 0,6 g (16 mmol) de borohydrure de sodium. Après 30 min d'agitation, 28 g (238 mmol) de chloroacétate de sodium sont ajoutés. A cette solution sont ensuite ajoutés goutte à goutte 24 mL de NAOH 10 N (240 mmol) puis le mélange est chauffé à 65°C pendant 90 minutes. 16,6 g (143 mmol) de chloroacétate de sodium sont ensuite ajoutés au milieu réactionnel ainsi que 14 mL de NaOH 10 N (140 mmol) au goutte à goutte. Après 1h de chauffage, le mélange est dilué avec de l'eau, neutralisé avec de l'acide acétique puis purifié par ultrafiltration sur membrane PES de 1 kDa contre de l'eau. La concentration en composé de la solution finale est déterminée par extrait sec, puis un dosage acide/base dans un mélange eau/acétone 50/50 (V/V) est effectué pour déterminer le degré de substitution en méthylcarboxylate de sodium.
D'après l'extrait sec : [composé polyanionique 1] = 32,9 mg/g
D'après le dosage acide/base : le degré de substitution en méthylcarboxylates de sodium par unité saccharidique est de 1,65.

### B Préparation des solutions

### B1. Solution d'insuline analogue rapide Novolog® à 100 UI/mL.

Cette solution est une solution commerciale d'insuline aspart de Novo Nordisk vendue sous le nom de Novolog®. Ce produit est une insuline aspart analogue rapide.

### B2. Solution d'insuline analogue rapide Humalog® à 100 UI/mL.

Cette solution est une solution commerciale d'insuline lispro d'Eli Lilly vendue sous le nom de Humalog®. Ce produit est une insuline analogue rapide.

### B3. Solution d'insuline humaine régulière. Actrapid® à 100 UI/mL.

Cette solution est une solution commerciale d'insuline humaine de Novo Nordisk vendue sous le nom d'Actrapid®. Ce produit est une insuline humaine régulière.

### B4. Solution d'insuline humaine régulière Humulin® R à 100 UI/mL.

Cette solution est une solution commerciale d'insuline humaine d'Eli Lilly vendue sous le nom de Humulin® R. Ce produit est une insuline humaine régulière.

### B5. Préparation des solutions d'excipients

Les composés polyanioniques non polymèriques sont sélectionnés par mesure de leur constante de dissociation vis-à-vis des ions calcium et vis-à-vis de leur propriété de ne pas déstabiliser la forme hexamèrique de l'insuline.

S'agissant de la constante de dissociation vis-à-vis des ions calcium, elle est déterminée comme suit.

Des solutions contenant 2,5 mM de CaCl2, 150 mM de NaCl et des concentrations croissantes de composé polyanionique (comprises entre 0 et 20 mM) sont préparées. Le potentiel de toutes ces formulations est mesuré et les concentrations en ions calcium libres dans les formulations déterminées. Après linéarisation par la méthode de Scatchard, les constantes de dissociation sont établies. Ces données permettent de comparer l'affinité des carboxylates et des phosphates des différents composés polyanioniques pour le Ca.

S'agissant de leur propriété de de ne pas déstabiliser la forme hexamèrique de l'insuline cette propriété est mesurée par dichroisme circulaire en comparaison de l'insuline seule (sans composé anionique ni composé polyanionique), voir les protocoles expérimentaux dans la partie expérimentale D.

### Préparation d'une solution de citrate de sodium à 1,188 M.

Une solution de citrate de sodium est obtenue en solubilisant 9,0811 g de citrate de sodium (30,9 mmol) dans 25 mL d'eau dans une fiole jaugée. Le pH est ajusté exactement à 7,4 par ajout de 1 mL d'HCl 1 M. La solution est filtrée sur 0,22 µm.

### Préparation d'une solution de m-crésol 130 mM.

Une solution de *m*-crésol est obtenue en solubilisant 14,114 g de *m*-crésol (130 mmol) dans 986,4 mL d'eau dans une fiole jaugée de 1 L.

### Préparation d'une solution de m-crésol et glycérine (96,6 mM m-crésol et 566 mM glycérine).

73,3 g de la solution de *m*-crésol à 130 mM sont ajoutés à 5,26 g de glycérine et ensuite dilués par ajout de 22,25 g d'eau. La solution obtenue de *m*-crésol et glycérine est homogénéisée pendant 30 minutes puis filtrée sur une membrane 0,22 µm.

### Préparation d'une solution de Tween 20 à 32,7 mM.

Une solution de Tween 20 est obtenue en solubilisant 2,0079 g de Tween 20 (1,636 mmol) dans 50 mL d'eau dans une fiole jaugée. La solution est filtrée sur une membrane 0,22 µm.

### B6. Préparation d'une solution d'insuline humaine à 500 UI/mL.

15 g d'eau sont ajoutés à 563,6 mg d'insuline humaine, puis le pH est abaissé à pH acide par ajout de 5,98 g d'HCl 0,1 N. Après solubilisation complète de l'insuline à pH acide, la solution est neutralisée à pH 7,2 par ajout de 8,3 mL de NaOH 0,1 N. La concentration est ensuite ajustée à 500 UI/mL par ajout de 0,76 g d'eau. La solution est enfin filtrée sur une membrane 0,22 µm.

### B7. Préparation d'une solution d'insuline lispro à 100 UI/mL en présence du composé 1

Pour un volume final de 100 mL de formulation, avec un ratio massique [composé 1]/[insuline lispro] de 2,0, les différents réactifs sont additionnés dans les quantités spécifiées ci-dessous et dans l'ordre suivant :

| | |
|---|---|
| Composé 1 lyophilisé | 730 mg |
| Solution commerciale Humalog® 100 UI/mL | 100 mL |

Le pH final est ajusté à 7,4 ± 0,4.

La solution limpide est filtrée sur une membrane 0,22 µm et stockée à 4°C.

### B8. Préparation d'une solution d'insuline lispro à 100 UI/mL en présence du composé 1 et de citrate

Pour un volume final de 100 mL de formulation, avec un ratio massique [composé 1]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 1 lyophilisé | 730 mg |
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B9. Préparation d'une solution d'insuline lispro à 100 UI/mL en présence du composé 1 et du composé polyanionique 1

Pour un volume final de 100 mL de formulation, avec un ratio massique [composé 1]/[composé polyanionique 1]/[insuline lispro] de 2,0/2,0/1, les différents réactifs sont additionnés dans les quantités spécifiées ci-dessous et dans l'ordre suivant :

| | |
|---|---|
| Composé 1 lyophilisé | 730 mg |
| Composé polyanionique 1 lyophilisé | 730 mg |
| Solution commerciale Humalog®100 UI/mL | 100 mL |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B10. Préparation d'une solution d'insuline lispro à 100 UI/mL en présence du composé 1 et du composé polyanionique 1

Pour un volume final de 100 mL de formulation, avec un ratio massique [composé 1]/[composé polyanionique 1]/[insuline lispro] de 2,0/5,5/1, les différents réactifs sont additionnés dans les quantités spécifiées ci-dessous et dans l'ordre suivant :

| | |
|---|---|
| Composé 1 lyophilisé | 730 mg |
| Composé polyanionique 1 lyophilisé | 2000 mg |
| Solution commerciale Humalog® 100 UI/mL | 100 mL |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B11. Préparation d'une solution d'insuline lispro à 100 UI/mL en présence du composé 2 et de citrate

Pour un volume final de 100 mL de formulation, avec un ratio massique [composé 2]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 2 lyophilisé | 730 mg |
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B12. Préparation d'une solution d'insuline lispro à 100 UI/mL en présence du composé 2 et du composé polyanionique 1

Pour un volume final de 100 mL de formulation, avec un ratio massique [composé 2]/[composé polyanionique 1]/[insuline lispro] de 2,0/2,0/1, les différents réactifs sont additionnés dans les quantités spécifiées ci-dessous et dans l'ordre suivant :

| | |
|---|---|
| Composé 2 lyophilisé | 730 mg |
| Composé polyanionique 1 lyophilisé | 730 mg |
| Solution commerciale Humalog®100 UI/mL | 100 mL |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B13. Préparation d'une solution d'insuline lispro à 100 UI/mL en présence du composé 2 et du composé polyanionique 1

Pour un volume final de 100 mL de formulation, avec un ratio massique [composé 2]/[composé polyanionique 1]/[insuline lispro] de 2,0/5,5/1, les différents réactifs sont additionnés dans les quantités spécifiées ci-dessous et dans l'ordre suivant :

| | |
|---|---|
| Composé 2 lyophilisé | 730 mg |
| Composé polyanionique 1 lyophilisé | 2000 mg |
| Solution commerciale Humalog® 100 UI/mL | 100 mL |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B14. Préparation d'une solution d'insuline lispro à 100 UI/mL en présence du composé 1.

Pour un volume final de 100 mL de formulation, avec un ratio massique [composé 1]/[insuline lispro] de 4, les différents réactifs sont additionnés dans les quantités spécifiées :

| | |
|---|---|
| Composé 1 sous forme lyophilisée | 1460 mg |
| Solution commerciale Humalog® 100 UI/mL | 100 mL |

Le pH final est ajusté à 7,4 ± 0,4. La solution limpide est filtrée sur une membrane 0,22 µm et stockée à 4°C.

### B15. Préparation d'une solution d'insuline lispro à 100 UI/mL en présence du composé 2.

Pour un volume final de 100 mL de formulation, avec un ratio massique [composé 2]/[insuline lispro] de 4, les différents réactifs sont additionnés dans les quantités spécifiées :

| | |
|---|---|
| Composé 2 sous forme lyophilisée | 1460 mg |
| Solution commerciale Humalog® 100 UI/mL | 100 mL |

Le pH final est ajusté à 7,4 ± 0,4. La solution limpide est filtrée sur une membrane 0,22 µm et stockée à 4°C.

### B16. Préparation d'une solution d'insuline analogue lispro à 100 UI/mL en présence du composé 1 et de tartrate de sodium.

Pour un volume final de 100 mL de formulation, avec un ratio massique [composé 1]/[insuline lispro] de 2,0 et une concentration de 80 mM de tartrate de sodium, les différents réactifs sont additionnés dans les quantités spécifiées ci-dessous :

| | |
|---|---|
| Composé 1 sous forme lyophilisée | 730 mg |
| Solution commerciale Humalog® 100 UI/mL | 100 mL |
| Tartrate de sodium | 1,552 g |

Pour le tartrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B17. Préparation d'une solution d'insuline lispro à 100 UI/mL en présence du composé 1 et de composé polyanionique 1.

Pour un volume final de 100 mL de formulation, avec un ratio massique [composé 1]/[composé polyanionique 1]/[insuline lispro] de 2/4/1, les différents réactifs sont additionnés dans les quantités spécifiées ci-dessous :

| | |
|---|---|
| Composé 1 sous forme lyophilisée | 730 mg |
| Composé polyanionique 1 sous forme lyophilisée | 1460 mg |
| Solution commerciale Humalog® 100 UI/mL | 100 mL |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B18. Préparation d'une solution d'insuline lispro à 100 UI/mL en présence du composé 1 et de triphosphate de sodium.

Pour un volume final de 100 mL de formulation les différents réactifs sont additionnés dans les quantités spécifiées ci-dessous :

| | | |
|---|---|---|
| Composé 1 sous forme lyophilisée | | 730 mg |
| Triphosphate de sodium | 184 mg | |
| Solution commerciale Humalog® | 100 UI/mL | 100 mL |

Pour le triphosphate, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B19. Préparation d'une solution d'insuline analogue lispro à 100 UI/mL en présence de composé 2 et de tartrate de sodium.

Pour un volume final de 100 mL de formulation, avec un ratio massique [composé 2]/[insuline lispro] de 2,0 et une concentration de 80 mM de tartrate de sodium, les différents réactifs sont additionnés dans les quantités spécifiées ci-dessous :

| | | |
|---|---|---|
| Composé 2 sous forme lyophilisée | | 730 mg |
| Solution commerciale Humalog® | 100 UI/mL | 100 mL |
| Tartrate de sodium | | 1,552 g |

Pour le tartrate, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B20. Préparation d'une solution d'insuline lispro à 100 UI/mL en présence du composé 2 et de composé polyanionique 1.

Pour un volume final de 100 mL de formulation, avec un ratio massique [composé 2]/[composé polyanionique 1]/[insuline lispro] de 2/4/1, les différents réactifs sont additionnés dans les quantités spécifiées ci-dessous :

| | |
|---|---|
| Composé 2 sous forme lyophilisée | 730 mg |
| Composé polyanionique 1 sous forme lyophilisée | 1460 mg |
| Solution commerciale Humalog® 100 UI/mL | 100 mL |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B21. Préparation d'une solution d'insuline lispro à 100 UI/mL en présence du composé 2 et de triphosphate de sodium.

Pour un volume final de 100 mL de formulation les différents réactifs sont additionnés dans les quantités spécifiées ci-dessous :

| | | |
|---|---|---|
| Composé 2 sous forme lyophilisée | | 730 mg |
| Triphosphate de sodium | 184 mg | |
| Solution commerciale Humalog® | 100 UI/mL | 100 mL |

Pour le triphosphate, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B22. Préparation d'une solution d'insuline analogue (insuline lispro) à 200 UI/mL.

La formulation commerciale d'insuline lispro (Humalog®) a été concentrée en utilisant des tubes de centrifugation AMICON Ultra-15 avec une coupure à 3 kDa. Les tubes amicon ont tout d'abord été rincés avec 12 mL d'eau déionisée. 12 mL de la formulation commerciale ont été centrifugés pendant 35 minutes à 4000 g à 20°C. Le volume du rétentat a été mesuré et la concentration ainsi estimée. Tous les rétentats ont été mis en commun et la concentration globale a été estimée (> 200 UI/mL).

La concentration de cette solution d'insuline lispro concentrée a été ajustée à 200 UI/mL par addition de la formulation commerciale d'insuline lispro (Humalog®). La formulation d'insuline lispro concentrée présente les mêmes concentrations en excipients (*m*-crésol, glycérine, phosphate) que la formulation commerciale à 100 UI/mL.

Le pH final est ajusté à 7,4 ± 0,4. La solution limpide est filtrée sur une membrane 0,22 µm et stockée à 4°C.

### B23. Préparation d'une solution d'insuline lispro à 200 UI/mL en présence du composé 1 et de citrate.

Pour un volume final de 100 mL de formulation avec un ratio massique [composé 1]/[insuline lispro] de 2, les différents réactifs sont mélangés en quantités précisées ci-dessous

| | |
|---|---|
| Insuline lispro à 200 UI/mL | 100 mL |
| Lyophilisat de composé 1 | 1460 mg |
| Solution de citrate de sodium à 1,188 M | 1566 µL |

Le pH final est ajusté à 7,4 ± 0,4.La solution limpide est filtrée sur une membrane 0,22 µm et stockée à 4°C.

### B24. Préparation d'une solution d'insuline lispro à 200 UI/mL en présence du composé 1 et de composé polyanionique 1.

Pour un volume final de 100 mL de formulation avec un ratio massique [composé 1]/[composé polyanionique 1]/[insuline lispro] de 2/2/1, les différents réactifs sont mélangés en quantités précisées ci-dessous.

| | |
|---|---|
| Insuline lispro à 200 UI/mL | 100 mL |
| Lyophilisat de composé 1 | 1460 mg |
| Lyophilisat de composé polyanionique 1 | 1460 mg |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B25. Préparation d'une solution d'insuline lispro à 200 UI/mL en présence du composé 1 et du composé polyanionique 1.

Pour un volume final de 100 mL de formulation avec un ratio massique [composé 1]/[composé polyanionique 1]/[insuline lispro] de 2/4/1, les différents réactifs sont mélangés en quantités précisées ci-dessous.

| | |
|---|---|
| Insuline lispro à 200 UI/mL | 100 mL |
| Lyophilisat de composé 1 | 1460 mg |
| Lyophilisat de composé polyanionique 1 | 2920 mg |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B26. Préparation d'une solution d'insuline lispro à 200 UI/mL en présence du composé 2 et du composé polyanionique 1.

Pour un volume final de 100 mL de formulation avec un ratio massique [composé 2]/[composé polyanionique 1]/[insuline lispro] de 2/4/1, les différents réactifs sont mélangés en quantités précisées ci-dessous.

| | |
|---|---|
| Insuline lispro à 200 UI/mL | 100 mL |
| Lyophilisat de composé 2 | 1460 mg |
| Lyophilisat de composé polyanionique 1 | 2920 mg |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B27. Préparation d'une solution d'insuline humaine à 100 UI/mL en présence du composé 1 et de tartrate.

Pour un volume final de 100 mL de formulation avec un ratio massique [composé 1]/[insuline humaine] de 2 et 80 mM de tartrate, les différents réactifs sont mélangés en quantités précisées ci-dessous :

| | |
|---|---|
| Insuline humaine à 500 UI/mL | 20 mL |
| Solution de composé 1 à 36,01 mg/mL | 20,27 mL |
| Solution 96,6 mM *m*-crésol/566 mM glycérine | 30 mL |
| Eau | 28,95 mL |
| Tartrate de sodium | 1,552 g |

Pour le tartrate, on peut utiliser, la forme acide ou la forme basique sous forme de sel de sodium, de sel de potassium ou d'un autre sel compatible avec une formulation injectable.

Le pH final est de 7,4 ± 0,4. Cette solution limpide est filtrée sur une membrane 0,22 µm puis est placée à +4°C.

### B28. Préparation d'une solution d'insuline humaine à 100 UI/mL en présence du composé 1 et de triphosphate.

Pour un volume final de 100 mL de formulation les différents réactifs sont mélangés en quantités précisées ci-dessous :

| | |
|---|---|
| Insuline humaine à 500 UI/mL | 20 mL |
| Solution de composé 1 à 36,01 mg/mL | 20,27 mL |
| Solution 96,6 mM *m*-crésol/566 mM glycérine | 30 mL |
| Eau | 28,95 mL |
| Triphosphate de sodium | 184 mg |

Pour le triphosphate, on peut utiliser, la forme acide ou la forme basique sous forme de sel de sodium, de sel de potassium ou d'un autre sel compatible avec une formulation injectable.

Le pH final est de 7,4 ± 0,4. Cette solution limpide est filtrée sur une membrane 0,22 µm puis est placée à +4°C.

### B29. Préparation d'une solution d'insuline humaine à 100 UI/mL en présence du composé 2 et de tartrate.

Pour un volume final de 100 mL de formulation avec un ratio massique [composé 2]/[insuline humaine] de 2 et 80 mM de tartrate, les différents réactifs sont mélangés en quantités précisées ci-dessous :

| | |
|---|---|
| Insuline humaine à 500 UI/mL | 20 mL |
| Solution de composé 2 à 36,01 mg/mL | 20,27 mL |
| Solution 96,6 mM *m*-crésol/566 mM glycérine | 30 mL |
| Eau | 28,95 mL |
| Tartrate de sodium | 1,552 g |

Pour le tartrate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B30. Préparation d'une solution d'insuline humaine à 100 UI/mL en présence du composé 2 et de triphosphate.

Pour un volume final de 100 mL de formulation les différents réactifs sont mélangés en quantités précisées ci-dessous :

| | |
|---|---|
| Insuline humaine à 500 UI/mL | 20 mL |
| Solution de composé 2 à 36,01 mg/mL | 20,27 mL |
| Solution 96,6 mM *m*-crésol/566 mM glycérine | 30 mL |
| Eau | 28,95 mL |
| Triphosphate de sodium | 184 mg |

Pour le triphosphate, on peut utiliser, la forme acide ou la forme basique sous forme de sel de sodium, de sel de potassium ou d'un autre sel compatible avec une formulation injectable.

Le pH final est de 7,4 ± 0,4. Cette solution limpide est filtrée sur une membrane 0,22 µm puis est placée à +4°C.

### B31. Préparation d'une solution d'insuline humaine à 200 UI/mL.

La formulation commerciale d'insuline humaine (Humulin® R) a été concentrée en utilisant des tubes de centrifugation AMICON Ultra-15 avec une coupure à 3 kDa. Les tubes amicon ont tout d'abord été rincés avec 12 mL d'eau déionisée. 12 mL de la formulation commerciale ont été centrifugés pendant 35 minutes à 4000 g à 20°C. Le volume du rétentat a été mesuré et la concentration ainsi estimée. Tous les rétentats ont été mis en commun et la concentration globale a été estimée (> 200 UI/mL).

La concentration de cette solution d'insuline humaine concentrée a été ajustée à 200 UI/mL par addition de la formulation commerciale d'insuline humaine (Humulin® R). La formulation d'insuline humaine concentrée présente les mêmes concentrations en excipients (m-crésol, glycérine) que la formulation commerciale à 100 UI/mL.

Le pH final est ajusté à 7,4 ± 0,4. La solution limpide est filtrée sur une membrane 0,22 µm et stockée à 4°C.

### B32. Préparation d'une solution d'insuline humaine à 200 UI/mL en présence du composé 1 et de citrate.

Pour un volume final de 100 mL de formulation avec un ratio massique [composé 1]/[insuline humaine] de 2, les différents réactifs sont mélangés en quantités précisées ci-dessous :

| | |
|---|---|
| Insuline humaine à 200 UI/mL | 100 mL |
| Lyophilisat du composé 1 | 1460 mg |
| Solution de citrate de sodium à 1.188 M | 1566 µL |

Le pH final est ajusté à 7,4 ± 0,4. La solution limpide est filtrée sur une membrane 0,22 µm et stockée à 4°C.

### B33. Préparation d'une solution d'insuline humaine à 200 UI/mL en présence du composé 1 et du composé polyanionique 1.

Pour un volume final de 100 mL de formulation avec un ratio massique [composé 1]/[composé polyanionique 1]/[insuline humaine] de 2/2/1, les différents réactifs sont mélangés en quantités précisées ci-dessous

| | |
|---|---|
| Insuline humaine à 200 UI/mL | 100 mL |
| Lyophilisat du composé 1 | 1460 mg |
| Lyophilisat du composé polyanionique 1 | 1460 mg |

Le pH final est ajusté à 7,4 ± 0,4. La solution limpide est filtrée sur une membrane 0,22 µm et stockée à 4°C.

### B34. Préparation d'une solution d'insuline humaine à 200 UI/mL en présence du composé 1 et du composé polyanionique 1.

Pour un volume final de 100 mL de formulation avec un ratio massique [composé 1]/[composé polyanionique 1]/[insuline humaine] de 2/4/1, les différents réactifs sont mélangés en quantités précisées ci-dessous

| | |
|---|---|
| Insuline humaine à 200 UI/mL | 100 mL |
| Lyophilisat du composé 1 | 1460 mg |
| Lyophilisat du composé polyanionique 1 | 2920 mg |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B35. Préparation d'une solution d'insuline humaine à 200 UI/mL en présence du composé 2 et de citrate.

Pour un volume final de 100 mL de formulation avec un ratio massique [composé 2]/[insuline humaine] de 2, les différents réactifs sont mélangés en quantités précisées ci-dessous

| | |
|---|---|
| Insuline humaine à 200 UI/mL | 100 mL |
| Lyophilisat du composé 2 | 1460 mg |
| Solution de citrate de sodium à 1,188 M | 1566 µL |

Le pH final est ajusté à 7,4 ± 0,4. La solution limpide est filtrée sur une membrane 0,22 µm et stockée à 4°C.

### B36. Préparation d'une solution d'insuline humaine à 200 UI/mL en présence du composé 2 et du composé polyanionique 1.

Pour un volume final de 100 mL de formulation avec un ratio massique [composé 2]/[composé polyanionique 1]/[insuline humaine] de 2/2/1, les différents réactifs sont mélangés en quantités précisées ci-dessous

| | |
|---|---|
| Insuline humaine à 200 UI/mL | 100 mL |
| Lyophilisat du composé 2 | 1460 mg |
| Lyophilisat du composé polyanionique 1 | 1460 mg |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B37. Préparation d'une solution d'insuline humaine à 200 UI/mL en présence du composé 2 et du composé polyanionique 1.

Pour un volume final de 100 mL de formulation avec un ratio massique [composé 2]/[composé polyanionique 1]/[insuline humaine] de 2/4/1, les différents réactifs sont mélangés en quantités précisées ci-dessous

| | |
|---|---|
| Insuline humaine à 200 UI/mL | 100 mL |
| Lyophilisat du composé 2 | 1460 mg |

| | |
|---|---|
| Lyophilisat du composé polyanionique 1 | 2920 mg |

Le composé polyanionique 1 peut être utilisé sous la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B38. Préparation d'une solution d'insuline humaine à 100 UI/mL en présence de composé 2 et de citrate.

Pour un volume final de 100 mL de formulation avec un ratio massique [composé 2]/[insuline humaine] de 2 et 9,3 mM de citrate, les différents réactifs sont mélangés en quantités précisées ci-dessous :

| | |
|---|---|
| Insuline humaine à 500 UI/mL | 20 mL |
| Solution du composé 2 à 36,01 mg/mL | 20,27 mL |
| Solution 96,6 mM *m*-crésol/566 mM glycérine | 30 mL |
| Eau | 28,95 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est de 7,4 ± 0,4. Cette solution limpide est filtrée sur une membrane 0,22 µm puis est placée à +4°C.

### B39. Préparation d'une solution d'insuline humaine à 100 UI/mL en présence du composé 1 et de citrate.

Pour un volume final de 100 mL de formulation avec un ratio massique [composé 1]/[insuline humaine] de 2 et 9,3 mM de citrate, les différents réactifs sont mélangés en quantités précisées ci-dessous :

| | |
|---|---|
| Insuline humaine à 500 UI/mL | 20 mL |
| Solution de composé 1 à 36,01 mg/mL | 27 mL |
| Solution 96,6 mM *m*-crésol/566 mM glycérine | 30 mL |
| Eau | 28,95 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est de 7,4 ± 0,4. Cette solution limpide est filtrée sur une membrane 0,22 µm puis est placée à +4°C.

### B40. Préparation d'une solution d'insuline aspart à 100 UI/mL en présence du composé 1 et de citrate

Pour un volume final de 100 mL de formulation, avec un ratio massique [composé 1]/[insuline aspart] de 2,0 et une concentration de 9,3 mM de citrate, les différents réactifs sont additionnés dans les quantités spécifiées ci-dessous et dans l'ordre suivant :

| | |
|---|---|
| Composé 1 lyophilisé | 730 mg |
| Solution commerciale Novolog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B41. Solution d'insuline analogue rapide Apidra® à 100 UI/mL.

Cette solution est une solution commerciale d'insuline glulisine de Sanofi-Aventis vendue sous le nom d'Apidra®. Ce produit est une insuline analogue rapide.

### B42. Préparation d'une solution d'insuline glulisine à 100 UI/mL en présence du composé 1 et de citrate

Pour un volume final de 100 mL de formulation, avec un ratio massique [composé 1]/[insuline glulisine] de 2,0 et une concentration de 9,3 mM de citrate, les différents réactifs sont additionnés dans les quantités spécifiées ci-dessous et dans l'ordre suivant :

| | |
|---|---|
| Composé 1 lyophilisé | 730 mg |
| Solution commerciale d'Apidra® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B43. Préparation d'une solution d'insuline aspart à 100 UI/mL en présence du composé 2 et de citrate

Pour un volume final de 100 mL de formulation, avec un ratio massique [composé 2]/[insuline aspart] de 2,0 et une concentration de 9,3 mM de citrate, les différents réactifs sont additionnés dans les quantités spécifiées ci-dessous et dans l'ordre suivant :

| | |
|---|---|
| Composé 2 lyophilisé | 730 mg |
| Solution commerciale Novolog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B44. Préparation d'une solution d'insuline glulisine à 100 UI/mL en présence du composé 2 et de citrate

Pour un volume final de 100 mL de formulation, avec un ratio massique [composé 2]/[insuline glulisine] de 2,0 et une concentration de 9,3 mM de citrate, les différents réactifs sont additionnés dans les quantités spécifiées ci-dessous et dans l'ordre suivant :

| | |
|---|---|
| Composé 2 lyophilisé | 730 mg |
| Solution commerciale d'Apidra® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B45. Préparation d'une solution d'insuline lispro à 100 UI/mL en présence du composé 5 et de citrate

Pour un volume final de 100 mL de formulation, avec un ratio massique [composé 5]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 5 lyophilisé | 730 mg |
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B46. Préparation d'une solution d'insuline lispro à 100 UI/mL en présence du composé 6 et de citrate

Pour un volume final de 100 mL de formulation, avec un ratio massique [composé 6]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 6 lyophilisé | 730 mg |
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B47. Préparation d'une solution d'insuline lispro à 100 UI/mL en présence du composé 7 et de citrate

Pour un volume final de 100 mL de formulation, avec un ratio massique [composé 7]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 7 lyophilisé | 730 mg |
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B48. Préparation d'une solution d'insuline lispro à 100 UI/mL en présence du composé 8 et de citrate

Pour un volume final de 100 mL de formulation, avec un ratio massique [composé 8]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 8 lyophilisé | 730 mg |

| | |
|---|---|
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B49. Préparation d'une solution d'insuline lispro à 100 UI/mL en présence du composé 9 et de citrate

Pour un volume final de 100 mL de formulation, avec un ratio massique [composé 9]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 9 lyophilisé | 730 mg |
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

### B50. Préparation d'une solution d'insuline humaine à 100 UI/mL en présence du composé 5 et de citrate

Pour un volume final de 100 mL de formulation, avec un ratio massique [composé 5]/[insuline humaine] de 2,0 et une concentration de 9,3 mM de citrate, les différents réactifs sont additionnés dans les quantités spécifiées ci-dessous et dans l'ordre suivant :

| | |
|---|---|
| Composé 5 lyophilisé | 730 mg |
| Solution commerciale Humulin® R 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B51. Préparation d'une solution d'insuline humaine à 100 UI/mL en présence du composé 6 et de citrate

Pour un volume final de 100 mL de formulation, avec un ratio massique [composé 6]/[insuline humaine] de 2,0 et une concentration de 9,3 mM de citrate, les différents réactifs sont additionnés dans les quantités spécifiées ci-dessous et dans l'ordre suivant :

| | |
|---|---|
| Composé 6 lyophilisé | 730 mg |
| Solution commerciale Humulin® R 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B52. Préparation d'une solution d'insuline humaine à 100 UI/mL en présence du composé 7 et de citrate

Pour un volume final de 100 mL de formulation, avec un ratio massique [composé 7]/[insuline humaine] de 2,0 et une concentration de 9,3 mM de citrate, les différents réactifs sont additionnés dans les quantités spécifiées ci-dessous et dans l'ordre suivant :

| | |
|---|---|
| Composé 7 lyophilisé | 730 mg |
| Solution commerciale Humulin® R 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B53. Préparation d'une solution d'insuline humaine à 100 UI/mL en présence du composé 8 et de citrate

Pour un volume final de 100 mL de formulation, avec un ratio massique [composé 8]/[insuline humaine] de 2,0 et une concentration de 9,3 mM de citrate, les différents réactifs sont additionnés dans les quantités spécifiées ci-dessous et dans l'ordre suivant :

| | |
|---|---|
| Composé 8 lyophilisé | 730 mg |
| Solution commerciale Humulin® R 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B54. Préparation d'une solution d'insuline humaine à 100 UI/mL en présence du composé 9 et de citrate

Pour un volume final de 100 mL de formulation, avec un ratio massique [composé 9]/[insuline humaine] de 2,0 et une concentration de 9,3 mM de citrate, les différents réactifs sont additionnés dans les quantités spécifiées ci-dessous et dans l'ordre suivant :

| | |
|---|---|
| Composé 9 lyophilisé | 730 mg |
| Solution commerciale Humulin® R 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B55. Préparation d'une solution d'insuline humaine à 100 UI/mL en présence du composé 2

Pour un volume final de 100 mL de formulation, avec un ratio massique [composé 2]/[insuline humaine] de 2,0, les différents réactifs sont additionnés dans les quantités spécifiées ci-dessous et dans l'ordre suivant :

| | |
|---|---|
| Composé 2 lyophilisé | 730 mg |
| Solution commerciale Humulin® R 100 UI/mL | 100 mL |

Le pH final est ajusté à 7,4 ± 0,4.

La solution limpide est filtrée sur une membrane 0,22 µm et stockée à 4°C.

### B56. Préparation d'une solution d'insuline humaine à 100 UI/mL en présence du composé 7

Pour un volume final de 100 mL de formulation, avec un ratio massique [composé 7]/[insuline humaine] de 2,0, les différents réactifs sont additionnés dans les quantités spécifiées ci-dessous et dans l'ordre suivant :

| | |
|---|---|
| Composé 7 lyophilisé | 730 mg |
| Solution commerciale Humulin® R 100 UI/mL | 100 mL |

Le pH final est ajusté à 7,4 ± 0,4.

La solution limpide est filtrée sur une membrane 0,22 µm et stockée à 4°C.

### B57. Préparation d'une solution d'insuline lispro à 100 UI/mL en présence du composé 10 et de citrate

Pour un volume final de 100 mL de formulation, avec un ratio massique [composé 10]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 10 lyophilisé | 730 mg |
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B58. Préparation d'une solution d'insuline lispro à 100 UI/mL en présence du composé 11 et de citrate

Pour un volume final de 100 mL de formulation, avec un ratio massique [composé 11]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 11 lyophilisé | 730 mg |
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

### B59. Préparation d'une solution d'insuline humaine à 100 UI/mL en présence du composé 10 et de citrate

Pour un volume final de 100 mL de formulation, avec un ratio massique [composé 10]/[insuline humaine] de 2,0 et une concentration de 9,3 mM de citrate, les différents réactifs sont additionnés dans les quantités spécifiées ci-dessous et dans l'ordre suivant :

| | |
|---|---|
| Composé 10 lyophilisé | 730 mg |
| Solution commerciale Humulin® R 100 UI/mL | 100 mL |

| | |
|---|---|
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B60. Préparation d'une solution d'insuline humaine à 100 UI/mL en présence du composé 11 et de citrate

Pour un volume final de 100 mL de formulation, avec un ratio massique [composé 11]/[insuline humaine] de 2,0 et une concentration de 9,3 mM de citrate, les différents réactifs sont additionnés dans les quantités spécifiées ci-dessous et dans l'ordre suivant :

| | |
|---|---|
| Composé 11 lyophilisé | 730 mg |
| Solution commerciale Humulin® R 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B61. Préparation d'une solution d'insuline aspart à 200 UI/mL.

La formulation commerciale d'insuline aspart (Novolog®) a été concentrée en utilisant des tubes de centrifugation AMICON Ultra-15 avec une coupure à 3 kDa. Les tubes amicon ont tout d'abord été rincés avec 12 mL d'eau déionisée. 12 mL de la formulation commerciale ont été centrifugés pendant 35 minutes à 4000 g à 20°C. Le volume du rétentat a été mesuré et la concentration ainsi estimée. Tous les rétentats ont été mis en commun et la concentration globale a été estimée (> 200 UI/mL).

La concentration de cette solution d'insuline aspart concentrée a été ajustée à 200 UI/mL par addition de la formulation commerciale d'insuline aspart (Novolog®). La formulation concentrée d'insuline aspart concentrée présente les mêmes concentrations en excipients (*m*-crésol, glycérine) que la formulation commerciale à 100 UI/mL.

En jouant sur le temps de centrifugation et sur la dilution finale avec la formulation commerciale, il est possible de préparer de la même manière des formulations d'insuline aspart à 300, 400 ou 500 UI/mL.

Le pH final est ajusté à 7,4 ± 0,4. La solution limpide est filtrée sur une membrane 0,22 µm et stockée à 4°C.

### B62. Préparation d'une solution d'insuline glulisine à 200 UI/mL.

La formulation commerciale d'insuline glulisine (Apidra®) a été concentrée en utilisant des tubes de centrifugation AMICON Ultra-15 avec une coupure à 3 kDa. Les tubes amicon ont tout d'abord été rincés avec 12 mL d'eau déionisée. 12 mL de la formulation commerciale ont été centrifugés pendant 35 minutes à 4000 g à 20°C. Le volume du rétentat a été mesuré et la concentration ainsi estimée. Tous les rétentats ont été mis en commun et la concentration globale a été estimée (> 200 UI/mL).

La concentration de cette solution d'insuline glulisine concentrée a été ajustée à 200 UI/mL par addition de la formulation commerciale d'insuline glulisine (Apidra®). La formulation d'insuline glulisine concentrée présente les mêmes concentrations en excipients (m-crésol, NaCl, TRIS) que la formulation commerciale à 100 UI/mL.

En jouant sur le temps de centrifugation et sur la dilution finale avec la formulation commerciale, il est possible de préparer de la même manière des formulations d'insuline glulisine à 300, 400 ou 500 UI/mL.

Le pH final est ajusté à 7,4 ± 0,4. La solution limpide est filtrée sur une membrane 0,22 µm et stockée à 4°C.

### B63. Préparation d'une solution d'insuline aspart à 200 UI/mL en présence du composé 1 à 14,6 mg/mL et de 18,6 mM de citrate.

Pour un volume final de 100 mL de formulation avec un ratio massique [composé 1]/[insuline aspart] de 2,0 les différents réactifs sont mélangés en quantités précisées ci-dessous et dans l'ordre qui suit:

| | |
|---|---|
| Lyophilisat du composé 1 | 1460 mg |
| Insuline aspart à 200 UI/mL | 100 mL |
| Solution de citrate de sodium à 1.188 M | 1566 µL |

Le pH final est ajusté à 7,4 ± 0,4. La solution limpide est filtrée sur une membrane 0,22 µm et stockée à 4°C.

### B64. Préparation d'une solution d'insuline humaine, d'insuline lispro, d'insuline aspart ou d'insuline glulisine à 300, 400 et 500 UI/mL.

Des formulations concentrées d'insuline humaine, d'insuline lispro, d'insuline aspart ou d'insuline glulisine à 300 UI/mL, 400 UI/mL ou 500 UI/mL (ainsi qu'à toutes concentrations intermédiaires) sont préparées sur la base du protocole de l'exemple B62 relatif à la préparation d'une solution d'insuline glulisine à 200 UI/mL. La formulation commerciale d'insuline est concentrée en utilisant des tubes de centrifugation AMICON Ultra-15 avec une coupure à 3 kDa. Les tubes amicon sont tout d'abord rincés avec 12 mL d'eau déionisée. 12 mL de la formulation commerciale sont centrifugés à 4000g et 20°C. En jouant sur le temps de centrifugation il est possible d'ajuster la concentration finale en insuline dans la formulation. Le volume du rétentat est mesuré et la concentration ainsi estimée. Tous les rétentats sont mis en commun et la concentration globale est estimée (> 300, 400 ou 500 UI/mL).

La concentration de cette solution d'insuline concentrée est ajustée à la concentration désirée (e.g. 300 UI/mL, 400 UI/mL ou 500 UI/mL) par addition de la formulation d'insuline (Humulin® R, Novolog®, Humalog® ou Apidra®). La formulation concentrée d'insuline concentrée présente les mêmes concentrations en excipients que la formulation commerciale à 100 UI/mL.

Le pH final est ajusté à 7,4 ± 0,4. La solution limpide est filtrée sur une membrane 0,22 µm et stockée à 4°C.

### B65. Préparation d'une solution d'insuline glulisine à 200 UI/mL en présence du composé 1 et de citrate.

Pour un volume final de 100 mL de formulation avec un ratio massique [composé 1]/[insuline glulisine] de 2, les différents réactifs sont mélangés en quantités précisées ci-dessous et dans l'ordre qui suit:

| | |
|---|---|
| Lyophilisat du composé 1 | 1460 mg |
| Insuline glulisine à 200 UI/mL | 100 mL |
| Solution de citrate de sodium à 1.188 M | 1566 µL |

Le pH final est ajusté à 7,4 ± 0,4. La solution limpide est filtrée sur une membrane 0,22 µm et stockée à 4°C.

### B66. Préparation d'une solution d'insuline aspart à 300 UI/mL en présence du composé 1 et de citrate.

Pour un volume final de 100 mL de formulation avec un ratio massique [composé 1]/[insuline aspart] de 2,0 les différents réactifs sont mélangés en quantités précisées ci-dessous :

| | |
|---|---|
| Insuline aspart à 300 UI/mL | 100 mL |
| Lyophilisat du composé 1 | 2190 mg |
| Citrate de sodium | 720 mg |

Le pH final est ajusté à 7,4 ± 0,4. La solution limpide est filtrée sur une membrane 0,22 µm et stockée à 4°C.

### B67. Préparation d'une solution d'insuline glulisine à 300 UI/mL en présence du composé 1 et de citrate.

Pour un volume final de 100 mL de formulation avec un ratio massique [composé 1]/[insuline glulisine] de 2,0 les différents réactifs sont mélangés en quantités précisées ci-dessous :

| | |
|---|---|
| Insuline glulisine à 300 UI/mL | 100 mL |
| Lyophilisat du composé 1 | 2190 mg |
| Citrate de sodium | 720 mg |

Le pH final est ajusté à 7,4 ± 0,4. La solution limpide est filtrée sur une membrane 0,22 µm et stockée à 4°C.

### B68. Préparation d'une solution d'insuline aspart à 400 UI/mL en présence du composé 1 et de citrate.

Pour un volume final de 100 mL de formulation avec un ratio massique [composé 1]/[insuline aspart] de 2, les différents réactifs sont mélangés en quantités précisées ci-dessous :

| | |
|---|---|
| Insuline aspart à 400 UI/mL | 100 mL |
| Lyophilisat du composé 1 | 2920 mg |
| Citrate de sodium | 960 mg |

Le pH final est ajusté à 7,4 ± 0,4. La solution limpide est filtrée sur une membrane 0,22 µm et stockée à 4°C.

### B69. Préparation d'une solution d'insuline glulisine à 400 UI/mL en présence du composé 1 et de citrate.

Pour un volume final de 100 mL de formulation avec un ratio massique [composé 1]/[insuline glulisine] de 2,0 les différents réactifs sont mélangés en quantités précisées ci-dessous :

| | |
|---|---|
| Insuline glulisine à 400 UI/mL | 100 mL |
| Lyophilisat du composé 1 | 2920 mg |
| Citrate de sodium | 960 mg |

Le pH final est ajusté à 7,4 ± 0,4. La solution limpide est filtrée sur une membrane 0,22 µm et stockée à 4°C.

### B70. Préparation d'une solution d'insuline aspart à 500 UI/mL en présence du composé 1 et de citrate.

Pour un volume final de 100 mL de formulation avec un ratio massique [composé 1]/[insuline aspart] de 2,0 les différents réactifs sont mélangés en quantités précisées ci-dessous :

| | |
|---|---|
| Insuline aspart à 500 UI/mL | 100 mL |
| Lyophilisat du composé 1 | 3650 mg |
| Citrate de sodium | 1200 mg |

Le pH final est ajusté à 7,4 ± 0,4. La solution limpide est filtrée sur une membrane 0,22 µm et stockée à 4°C.

### B71. Préparation d'une solution d'insuline glulisine à 500 UI/mL en présence du composé 1 et de citrate.

Pour un volume final de 100 mL de formulation avec un ratio massique [composé 1]/[insuline glulisine] de 2,0 les différents réactifs sont mélangés en quantités précisées ci-dessous :

| | |
|---|---|
| Insuline glulisine à 500 UI/mL | 100 mL |
| Lyophilisat du composé 1 | 3650 mg |
| Citrate de sodium | 1200 mg |

Le pH final est ajusté à 7,4 ± 0,4. La solution limpide est filtrée sur une membrane 0,22 µm et stockée à 4°C.

### B72. Préparation d'une solution d'insuline humaine à 300 UI/mL en présence du composé 1 et de citrate.

Pour un volume final de 100 mL de formulation avec un ratio massique [composé 1]/[insuline humaine] de 2,0 les différents réactifs sont mélangés en quantités précisées ci-dessous :

| | |
|---|---|
| Insuline humaine à 300 UI/mL | 100 mL |
| Lyophilisat du composé 1 | 2190 mg |
| Citrate de sodium | 720 mg |

Le pH final est ajusté à 7,4 ± 0,4. La solution limpide est filtrée sur une membrane 0,22 µm et stockée à 4°C.

### B73. Préparation d'une solution d'insuline lispro à 300 UI/mL en présence du composé 1 et de citrate.

Pour un volume final de 100 mL de formulation avec un ratio massique [composé 1]/[insuline lispro] de 2,0 les différents réactifs sont mélangés en quantités précisées ci-dessous :

| | |
|---|---|
| Insuline lispro à 300 UI/mL | 100 mL |
| Lyophilisat du composé 1 | 2190 mg |
| Citrate de sodium | 720 mg |

Le pH final est ajusté à 7,4 ± 0,4. La solution limpide est filtrée sur une membrane 0,22 µm et stockée à 4°C.

### B74. Préparation d'une solution d'insuline humaine à 400 UI/mL en présence du composé 1 et de citrate.

Pour un volume final de 100 mL de formulation avec un ratio massique [composé 1]/[insuline humaine] de 2,0 les différents réactifs sont mélangés en quantités précisées ci-dessous :

| | |
|---|---|
| Insuline humaine à 400 UI/mL | 100 mL |
| Lyophilisat du composé 1 | 2920 mg |
| Citrate de sodium | 960 mg |

Le pH final est ajusté à 7,4 ± 0,4. La solution limpide est filtrée sur une membrane 0,22 µm et stockée à 4°C.

### B75. Préparation d'une solution d'insuline lispro à 400 UI/mL en présence du composé 1 et de citrate.

Pour un volume final de 100 mL de formulation avec un ratio massique [composé 1]/[insuline lispro] de 2,0 les différents réactifs sont mélangés en quantités précisées ci-dessous :

| | |
|---|---|
| Insuline lispro à 400 UI/mL | 100 mL |
| Lyophilisat du composé 1 | 2920 mg |
| Citrate de sodium | 960 mg |

Le pH final est ajusté à 7,4 ± 0,4. La solution limpide est filtrée sur une membrane 0,22 µm et stockée à 4°C.

### B76. Préparation d'une solution d'insuline humaine à 500 UI/mL en présence du composé 1 et de citrate.

Pour un volume final de 100 mL de formulation avec un ratio massique [composé 1]/[insuline humaine] de 2,0 les différents réactifs sont mélangés en quantités précisées ci-dessous :

| | |
|---|---|
| Insuline humaine à 500 UI/mL | 100 mL |
| Lyophilisat du composé 1 | 3650 mg |
| Citrate de sodium | 1200 mg |

Le pH final est ajusté à 7,4 ± 0,4. La solution limpide est filtrée sur une membrane 0,22 µm et stockée à 4°C.

### B77. Préparation d'une solution d'insuline lispro à 500 UI/mL en présence du composé 1 et de citrate.

Pour un volume final de 100 mL de formulation avec un ratio massique [composé 1]/[insuline lispro] de 2,0 les différents réactifs sont mélangés en quantités précisées ci-dessous :

| | |
|---|---|
| Insuline lispro à 500 UI/mL | 100 mL |
| Lyophilisat du composé 1 | 3650 mg |
| Citrate de sodium | 1200 mg |

Le pH final est ajusté à 7,4 ± 0,4. La solution limpide est filtrée sur une membrane 0,22 µm et stockée à 4°C.

### B78. Préparation d'une solution d'insuline lispro à 200 UI/mL en présence du composé 2 et de citrate.

Pour un volume final de 100 mL de formulation avec un ratio massique [composé 2]/[insuline lispro] de 2,0 les différents réactifs sont mélangés en quantités précisées ci-dessous :

| | |
|---|---|
| Insuline lispro à 200 UI/mL | 100 mL |
| Lyophilisat du composé 2 | 1460 mg |
| Solution de citrate de sodium à 1.188 M | 1566 µL |

Le pH final est ajusté à 7,4 ± 0,4. La solution limpide est filtrée sur une membrane 0,22 µm et stockée à 4°C.

### B79. Préparation d'une solution d'insuline aspart à 200 UI/mL en présence du composé 2 et de citrate.

Pour un volume final de 100 mL de formulation avec un ratio massique [composé 2]/[insuline aspart] de 2,0 les différents réactifs sont mélangés en quantités précisées ci-dessous :

| | |
|---|---|
| Insuline aspart à 200 UI/mL | 100 mL |
| Lyophilisat du composé 2 | 1460 mg |
| Solution de citrate de sodium à 1.188 M | 1566 µL |

Le pH final est ajusté à 7,4 ± 0,4. La solution limpide est filtrée sur une membrane 0,22 µm et stockée à 4°C.

### B80. Préparation d'une solution d'insuline glulisine à 200 UI/mL en présence du composé 2 et de citrate.

Pour un volume final de 100 mL de formulation avec un ratio massique [composé 2]/[insuline glulisine] de 2,0 les différents réactifs sont mélangés en quantités précisées ci-dessous :

| | |
|---|---|
| Insuline glulisine à 200 UI/mL | 100 mL |
| Lyophilisat du composé 2 | 1460 mg |
| Solution de citrate de sodium à 1.188 M | 1566 µL |

Le pH final est ajusté à 7,4 ± 0,4. La solution limpide est filtrée sur une membrane 0,22 µm et stockée à 4°C.

### B81. Préparation d'une solution d'insuline aspart à 300 UI/mL en présence du composé 2 et de citrate.

Pour un volume final de 100 mL de formulation avec un ratio massique [composé 2]/[insuline aspart] de 2,0 les différents réactifs sont mélangés en quantités précisées ci-dessous :

| | |
|---|---|
| Insuline aspart à 300 UI/mL | 100 mL |
| Lyophilisat du composé 2 | 2190 mg |
| Citrate de sodium | 720 mg |

Le pH final est ajusté à 7,4 ± 0,4. La solution limpide est filtrée sur une membrane 0,22 µm et stockée à 4°C.

### B82. Préparation d'une solution d'insuline glulisine à 300 UI/mL en présence du composé 2 et de citrate.

Pour un volume final de 100 mL de formulation avec un ratio massique [composé 2]/[insuline glulisine] de 2,0 les différents réactifs sont mélangés en quantités précisées ci-dessous :

| | |
|---|---|
| Insuline glulisine à 300 UI/mL | 100 mL |
| Lyophilisat du composé 2 | 2190 mg |
| Citrate de sodium | 720 mg |

Le pH final est ajusté à 7,4 ± 0,4. La solution limpide est filtrée sur une membrane 0,22 µm et stockée à 4°C.

### B83. Préparation d'une solution d'insuline aspart à 400 UI/mL en présence du composé 2 et de citrate.

Pour un volume final de 100 mL de formulation avec un ratio massique [composé 2]/[insuline aspart] de 2,0 les différents réactifs sont mélangés en quantités précisées ci-dessous :

| | |
|---|---|
| Insuline aspart à 400 UI/mL | 100 mL |
| Lyophilisat du composé 2 | 2920 mg |
| Citrate de sodium | 960 mg |

Le pH final est ajusté à 7,4 ± 0,4. La solution limpide est filtrée sur une membrane 0,22 µm et stockée à 4°C.

### B84. Préparation d'une solution d'insuline glulisine à 400 UI/mL en présence du composé 2 et de citrate.

Pour un volume final de 100 mL de formulation avec un ratio massique [composé 2]/[insuline glulisine] de 2,0 les différents réactifs sont mélangés en quantités précisées ci-dessous :

| | |
|---|---|
| Insuline glulisine à 400 UI/mL | 100 mL |
| Lyophilisat du composé 2 | 2920 mg |
| Citrate de sodium | 960 mg |

Le pH final est ajusté à 7,4 ± 0,4. La solution limpide est filtrée sur une membrane 0,22 µm et stockée à 4°C.

### B85. Préparation d'une solution d'insuline aspart à 500 UI/mL en présence du composé 2 et de citrate.

Pour un volume final de 100 mL de formulation avec un ratio massique [composé 2]/[insuline aspart] de 2,0 les différents réactifs sont mélangés en quantités précisées ci-dessous : ]

| | |
|---|---|
| Insuline aspart à 500 UI/mL | 100 mL |
| Lyophilisat du composé 2 | 3650 mg |
| Citrate de sodium | 1200 mg |

Le pH final est ajusté à 7,4 ± 0,4. La solution limpide est filtrée sur une membrane 0,22 µm et stockée à 4°C.

### B86. Préparation d'une solution d'insuline glulisine à 500 UI/mL en présence du composé 2 et de citrate.

Pour un volume final de 100 mL de formulation avec un ratio massique [composé 2]/[insuline glulisine] de 2,0 les différents réactifs sont mélangés en quantités précisées ci-dessous :

| | |
|---|---|
| Insuline glulisine à 500 UI/mL | 100 mL |
| Lyophilisat du composé 2 | 3650 mg |
| Citrate de sodium | 1200 mg |

Le pH final est ajusté à 7,4 ± 0,4. La solution limpide est filtrée sur une membrane 0,22 µm et stockée à 4°C.

### B87. Préparation d'une solution d'insuline humaine à 300 UI/mL en présence du composé 2 et de citrate.

Pour un volume final de 100 mL de formulation avec un ratio massique [composé 2]/[insuline humaine] de 2, les différents réactifs sont mélangés en quantités précisées ci-dessous :

| | |
|---|---|
| Insuline humaine à 300 UI/mL | 100 mL |
| Lyophilisat du composé 2 | 2190 mg |
| Citrate de sodium | 720 mg |

Le pH final est ajusté à 7,4 ± 0,4. La solution limpide est filtrée sur une membrane 0,22 µm et stockée à 4°C.

### B88. Préparation d'une solution d'insuline lispro à 300 UI/mL en présence du composé 2 et de citrate.

Pour un volume final de 100 mL de formulation avec un ratio massique [composé 2]/[insuline lispro] de 2,0 les différents réactifs sont mélangés en quantités précisées ci-dessous :

| | |
|---|---|
| Insuline lispro à 300 UI/mL | 100 mL |
| Lyophilisat du composé 2 | 2190 mg |
| Citrate de sodium | 720 mg |

Le pH final est ajusté à 7,4 ± 0,4. La solution limpide est filtrée sur une membrane 0,22 µm et stockée à 4°C.

### B89. Préparation d'une solution d'insuline humaine à 400 UI/mL en présence du composé 2 et de citrate.

Pour un volume final de 100 mL de formulation avec un ratio massique [composé 2]/[insuline humaine] de 2,0 les différents réactifs sont mélangés en quantités précisées ci-dessous :

| | |
|---|---|
| Insuline humaine à 400 UI/mL | 100 mL |
| Lyophilisat du composé 2 | 2920 mg |
| Citrate de sodium | 960 mg |

Le pH final est ajusté à 7,4 ± 0,4. La solution limpide est filtrée sur une membrane 0,22 µm et stockée à 4°C.

### B90. Préparation d'une solution d'insuline lispro à 400 UI/mL en présence du composé 2 et de citrate.

Pour un volume final de 100 mL de formulation avec un ratio massique [composé 2]/[insuline lispro] de 2,0 les différents réactifs sont mélangés en quantités précisées ci-dessous :

| | |
|---|---|
| Insuline lispro à 400 UI/mL | 100 mL |
| Lyophilisat du composé 2 | 2920 mg |
| Citrate de sodium | 960 mg |

Le pH final est ajusté à 7,4 ± 0,4. La solution limpide est filtrée sur une membrane 0,22 µm et stockée à 4°C.

### B91. Préparation d'une solution d'insuline humaine à 500 UI/mL en présence du composé 2 et de citrate.

Pour un volume final de 100 mL de formulation avec un ratio massique [composé 2]/[insuline humaine] de 2,0 les différents réactifs sont mélangés en quantités précisées ci-dessous :

| | |
|---|---|
| Insuline humaine à 500 UI/mL | 100 mL |
| Lyophilisat du composé 2 | 3650 mg |
| Citrate de sodium | 1200 mg |

Le pH final est ajusté à 7,4 ± 0,4. La solution limpide est filtrée sur une membrane 0,22 µm et stockée à 4°C.

### B92. Préparation d'une solution d'insuline lispro à 500 UI/mL en présence du composé 2 et de citrate.

Pour un volume final de 100 mL de formulation avec un ratio massique [composé 2]/[insuline lispro] de 2,0 les différents réactifs sont mélangés en quantités précisées ci-dessous :

| | |
|---|---|
| Insuline lispro à 500 UI/mL | 100 mL |
| Lyophilisat du composé 2 | 3650 mg |
| Citrate de sodium | 1200 mg |

Le pH final est ajusté à 7,4 ± 0,4. La solution limpide est filtrée sur une membrane 0,22 µm et stockée à 4°C.

### B93. Préparation d'une solution d'insuline lispro à 100 UI/mL en présence du composé 3 et de citrate

Pour un volume final de 100 mL de formulation, avec un ratio massique [composé 3]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 3 lyophilisé | 730 mg |
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B94. Préparation d'une solution d'insuline lispro à 100 UI/mL en présence du composé 4 et de citrate

Pour un volume final de 100 mL de formulation, avec un ratio massique [composé 4]/[insuline lispro] de 2,0 et une concentration de 9,3 mM de citrate, les différents réactifs sont additionnés dans les quantités spécifiées ci-dessous et dans l'ordre suivant :

| | |
|---|---|
| Composé 4 lyophilisé | 730 mg |
| Solution commerciale Humalog® 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B95. Préparation d'une solution d'insuline humaine à 100 UI/mL en présence du composé 3 et de citrate

Pour un volume final de 100 mL de formulation, avec un ratio massique [composé 3]/[insuline humaine] de 2,0 et une concentration de 9,3 mM de citrate, les différents réactifs sont additionnés dans les quantités spécifiées ci-dessous et dans l'ordre suivant :

| | |
|---|---|
| Composé 3 lyophilisé | 730 mg |
| Solution commerciale Humulin® R 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B96. Préparation d'une solution d'insuline humaine à 100 UI/mL en présence du composé 4 et de citrate

Pour un volume final de 100 mL de formulation, avec un ratio massique [composé 4]/[insuline humaine] de 2,0 et une concentration de 9,3 mM de citrate, les différents réactifs sont additionnés dans les quantités spécifiées ci-dessous et dans l'ordre suivant :

| | |
|---|---|
| Composé 4 lyophilisé | 730 mg |
| Solution commerciale Humulin® R 100 UI/mL | 100 mL |
| Solution de citrate de sodium à 1,188 M | 783 µL |

Pour le citrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### C Pharmacodynamie et pharmacocinétique

### C1 : Protocole de mesure de la pharmacodynamie des solutions d'insuline

12 porcs domestiques d'environ 50 kg, préalablement cathétérisés au niveau de la jugulaire, sont mis à jeun 2,5 heures avant le début de l'expérience. Dans l'heure précédant l'injection d'insuline, 3 prélèvements sanguins sont réalisés afin de déterminer le niveau basal de glucose et d'insuline.

L'injection d'insuline à la dose de 0,09 UI/kg pour l'insuline lispro et à la dose de 0,125 UI/kg pour l'insuline humaine et l'insuline aspart est réalisée en sous-cutané au niveau du cou, sous l'oreille de l'animal à l'aide du stylo à insuline Novopen équipé d'une aiguille 31 G.

Des prélèvements sanguins sont ensuite réalisés toutes les 4 minutes pendant 20 minutes puis toutes les 10 minutes jusqu'à 3 heures. Après chaque prélèvement, le cathéter est rincé avec une solution diluée d'héparine.

Une goutte de sang est prélevée pour déterminer la glycémie au moyen d'un glucomètre.

Les courbes de pharmacodynamie du glucose sont ensuite tracées et le temps nécessaire pour atteindre le taux minimum de glucose dans le sang pour chaque porc est déterminé et reporté comme Tmin glucose. La moyenne des Tmin glucose est ensuite calculée.

Le sang restant est collecté dans un tube sec et est centrifugé pour isoler le sérum. Les taux d'insuline dans les échantillons de sérum sont mesurés par la méthode immuno-enzymatique ELISA en sandwich pour chaque porc.

Les coubes de pharmacocinétique sont ensuite tracées. Le temps nécessaire pour atteindre la concentration maximale d'insuline dans le sérum pour chaque porc est déterminé et reporté comme Tmax insuline. La moyenne des Tmax insuline est ensuite calculée.

### C2 : Résultats de pharmacodynamie et de pharmacocinétique des solutions d'insulines des exemples B2 et B8

| **Exemple** | **Insuline** | **Composé** | **Composé polyanionique** | **Nombre de cochons** |
|---|---|---|---|---|
| B2 | Lispro | - | - | 11 |
| B8 | Lispro | 1 | Citrate 9,3 mM | 10 |

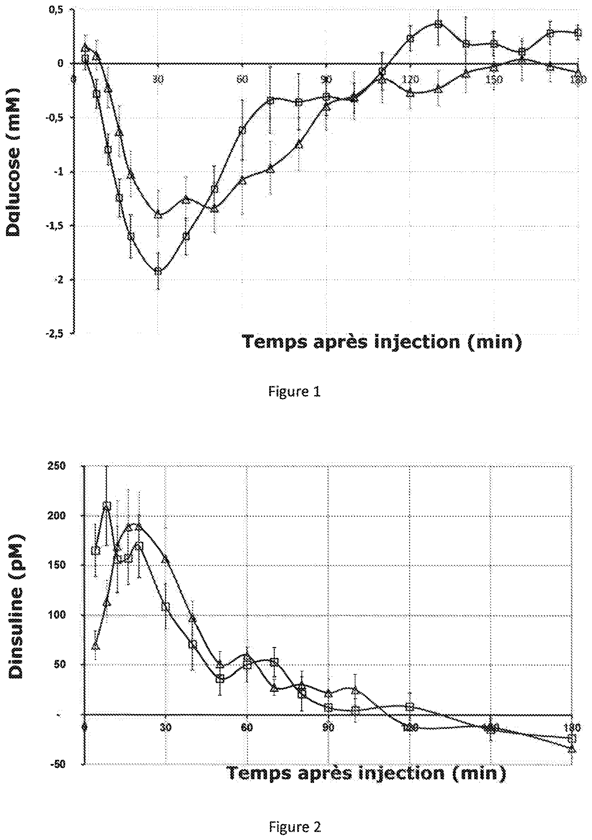

Les résultats de pharmacodynamie obtenus avec les formulations décrites dans les exemples B2 et B8 sont présentés sur la figure 1. Selon l'invention, l'analyse de ces courbes montrent que la formulation de l'exemple B8 comprenant le composé 1 et le citrate à 9,3 mM comme excipient (courbe tracée avec les carrés correspondant à l'exemple B8, Tmin glucose = 30 ± 11 min) permet d'obtenir une action plus rapide que celle de la formulation commerciale Humalog® de l'exemple B2 (courbe tracée avec les triangles correspondant à l'exemple B2, Tmin glucose = 44 ± 14 min).

Les résultats de pharmacocinétique obtenus avec les formulations décrites dans les exemples B2 et B8 sont présentés sur la figure 2. Selon l'invention, l'analyse de ces courbes montre que la formulation de l'exemple B8 comprenant le composé 1 et le citrate à 9,3 mM comme excipients (courbe tracée avec les carrés correspondant à l'exemple B8, Tmax insuline = 11 ± 6 min) induit une absorption plus rapide de l'insuline lispro que la formulation commerciale Humalog® de l'exemple B2 (courbe tracée avec les triangles correspondant à l'exemple B2 , Tmax insuline = 18 ± 8 min).

### C3 : Résultats de pharmacodynamie et de pharmacocinétique des solutions d'insulines des exemples B2 et B10

| **Exemple** | **Insuline** | **Composé** | **Composé polyanionique** | **Nombre de cochons** |
|---|---|---|---|---|
| B2 | Lispro | - | - | 11 |
| B10 | Lispro | 1 | Composé polyanionique 1 | 11 |

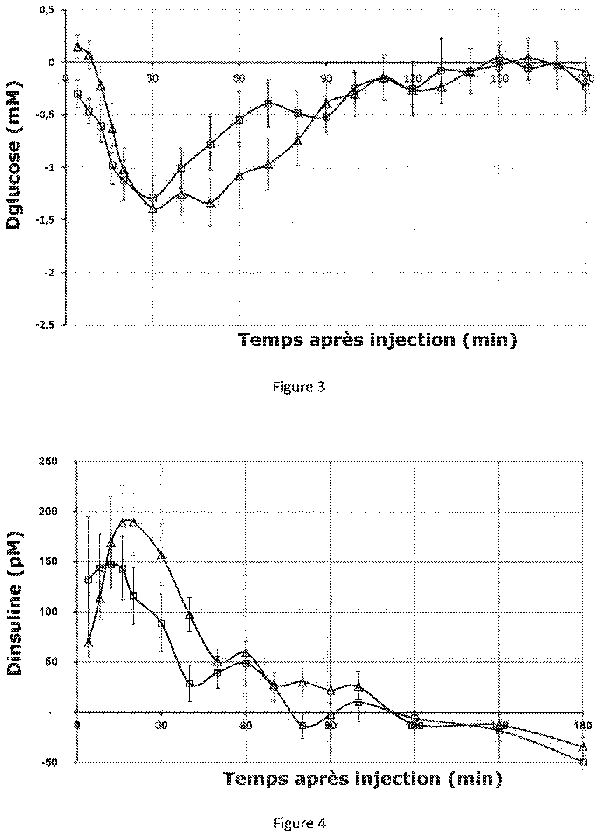

Les résultats de pharmacodynamie obtenus avec les formulations décrites dans les exemples B2 et B10 sont présentés sur la figure 3. Selon l'invention, l'analyse de ces courbes montrent que la formulation de l'exemple B10 comprenant le composé 1 et le composé polyanionique 1 comme excipients à 20 mg/mL (courbe tracée avec les carrés correspondant à l'exemple B10, Tmin glucose = 33 ± 13 min) permet d'obtenir une action plus rapide que celle de la formulation commerciale Humalog® de l'exemple B2 (courbe tracée avec les triangles correspondant à l'exemple B2, Tmin glucose = 44 ± 14 min).

Les résultats de pharmacocinétique obtenus avec les formulations décrites dans les exemples B2 et B10 sont présentés sur la figure 4. Selon l'invention, l'analyse de ces courbes montre que la formulation de l'exemple B10 comprenant le composé 1 et le composé polyanionique 1 comme excipients à 20 mg/mL (courbe tracée avec les carrés correspondant à l'exemple B10, Tmax insuline = 15 ± 9 min) induit une absorption plus rapide de l'insuline lispro que la formulation commerciale Humalog® de l'exemple B2 (courbe tracée avec les triangles correspondant à l'exemple B2, Tmax insuline = 18 ± 8 min).

### C4 : Résultats de pharmacodynamie et de pharmacocinétique des solutions d'insulines des exemples B2 et B7

| **Exemple** | **Insuline** | **Composé** | **Composé polyanionique** | **Nombre de cochons** |
|---|---|---|---|---|
| B2 | Lispro | - | - | 12 |
| B7 | Lispro | 1 | - | 12 |

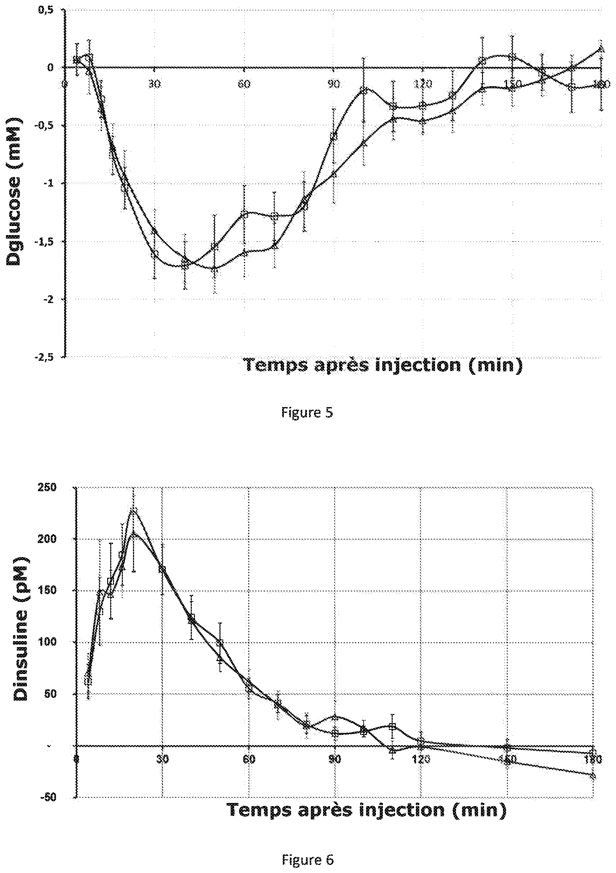

Les résultats de pharmacodynamie obtenus avec les formulations décrites dans les exemples B2 et B7 sont présentés figure 5. Selon l'invention, l'analyse de ces courbes montre que la formulation de l'exemple B7 comprenant le composé 1 comme excipient (courbe tracée avec les carrés correspondant à l'exemple B7, Tmin glucose = 41 ± 16 min) induit un début d'action plus rapide que celle de la formulation commerciale Humalog® de l'exemple B2 (courbe tracée avec les triangles correspondant à l'exemple B2, Tmin glucose = 50 ± 14 min).

Les résultats de pharmacocinétique obtenus avec les formulations décrites dans les exemples B2 et B7 sont présentés figure 6. L'analyse de ces courbes montre que la formulation comprenant le composé 1 comme excipient (courbe tracée avec les carrés correspondant à l'exemple B2, Tmax insuline = 21 ± 10 min) n'induit pas une absorption plus rapide de l'insuline lispro que la formulation commerciale Humalog® de l'exemple B2 (courbe tracée avec les triangles correspondant à l'exemple B2 (Tmax insuline = 20 ± 9 min). Le composé 1 seul n'est donc pas suffisant pour induire une accélération significative de la pharmacocinétique de l'insuline lispro.

### C5 : Résultats de pharmacodynamie et de pharmacocinétique des solutions d'insulines des exemples B1 et B3

| **Exemple** | **Insuline** | **Composé** | **Composé polyanionique** | **Nombre de cochons** |
|---|---|---|---|---|
| B1 | Aspart | - | - | 11 |
| B3 | Humaine | - | - | 11 |

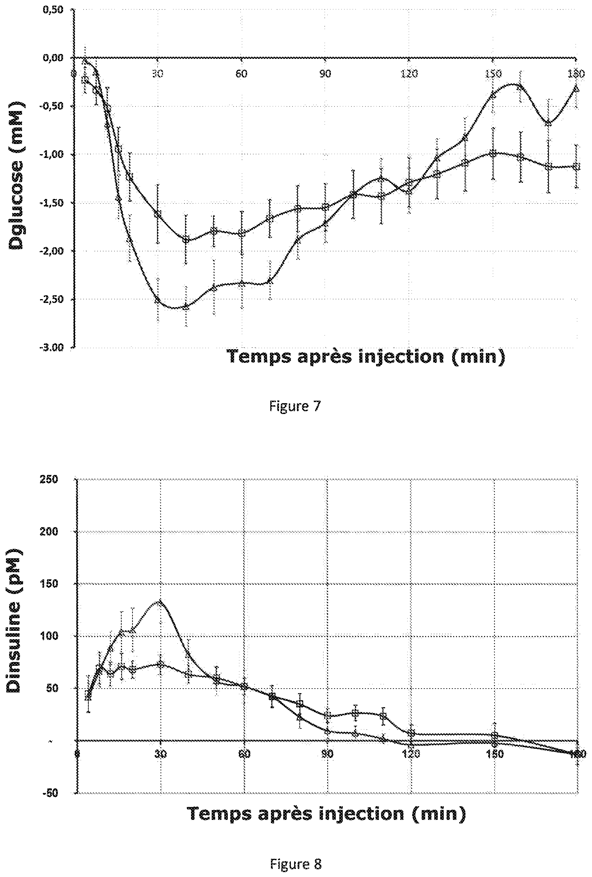

Les résultats de pharmacodynamie obtenus avec les formulations décrites dans les exemples B1 et B3 sont présentés sur la figure 7. L'analyse de ces courbes montre que la formulation d'insuline humaine de l'exemple B3 (courbe tracée avec les carrés correspondant à l'exemple B3, Tmin glucose = 61 ± 31 min) a bien une action plus lente que celle de la formulation commerciale d'insuline aspart de l'exemple B1 (courbe tracée avec les triangles correspondant à l'exemple B1, Tmin glucose = 44 ± 13 min).

Les résultats de pharmacocinétique obtenus avec les formulations décrites dans les exemples B1 et B3 sont présentés sur la figure 8. L'analyse de ces courbes montre que la formulation d'insuline humaine seule de l'exemple B3 (courbe tracée avec les carrés correspondant à l'exemple B3, Tmax insuline = 36 ± 33 min) induit bien une absorption plus lente que la formulation commerciale d'insuline aspart (Novolog®) de l'exemple B1 (courbe tracée avec les triangles correspondant à l'exemple B1, Tmax insuline = 28 ± 13 min).

Ces résultats sont conformes à ceux de la littérature avec une accélération de la baisse de la glycémie et de l'absorption de l'insuline pour un analogue rapide d'insuline par rapport à une insuline humaine.

### C6 : Résultats de pharmacodynamie et de pharmacocinétique des solutions d'insulines des exemples B1 et B39

| **Exemple** | **Insuline** | **Composé** | **Composé polyanionique** | **Nombre de cochons** |
|---|---|---|---|---|
| B1 | Aspart | - | - | 14 |
| B39 | Humaine | 1 | Citrate 9,3 mM | 5 |

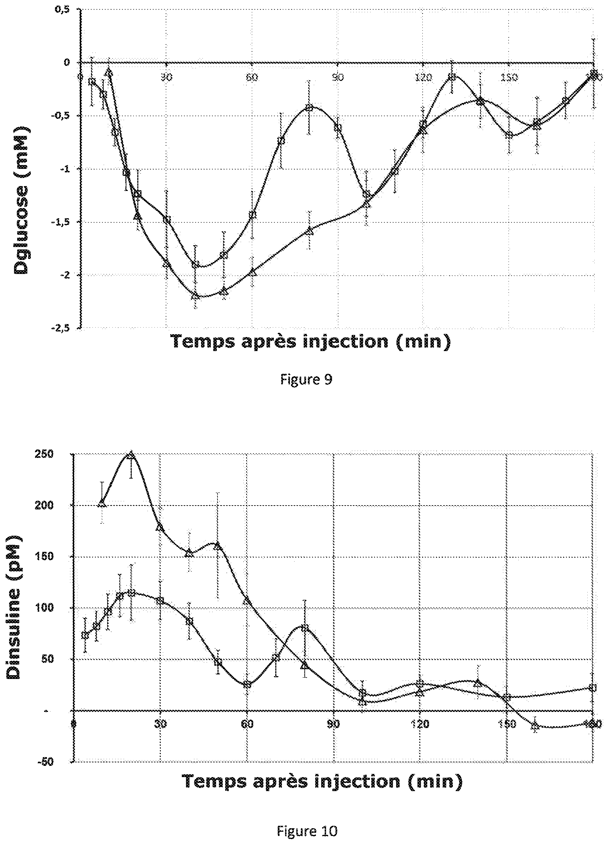

Les résultats de pharmacodynamie obtenus avec les formulations décrites dans les exemples B1 et B39 sont présentés sur la figure 9. L'analyse de ces courbes montre que la formulation à base d'insuline humaine de l'exemple B39 comprenant le composé 1 et le citrate à 9,3 mM comme excipients (courbe tracée avec les carrés correspondant à l'exemple B39, Tmin glucose = 46 ± 9 min) permet d'obtenir une action similaire à celle de la formulation commerciale d'insuline aspart (Novolog®) de l'exemple B1 (courbe tracée avec les triangles correspondant à l'exemple B1, Tmin glucose = 53 ± 24 min).

Les résultats de pharmacocinétique obtenus avec les formulations décrites dans les exemples B1 et B39 sont présentés sur la figure 10. L'analyse de ces courbes montre que la formulation de l'exemple B39 comprenant le composé 1 et le citrate à 9,3 mM comme excipients (courbe tracée avec les carrés correspondant à l'exemple B39, Tmax insuline = 20 ± 7 min) induit une absorption de l'insuline humaine similaire à celle de la formulation commerciale d'insuline aspart (Novolog®) de l'exemple B1 (courbe tracée avec les triangles correspondant à l'exemple B1, Tmax insuline = 22 ± 10 min).

Les paramètres de temps de l'insuline aspart (Novolog®) entre les exemples C5 et C6 étant similaires, on peut en déduire par extrapolation que la formulation de l'exemple B39 induit une accélération de la baisse de la glycémie et de l'absorption de l'insuline humaine par rapport à la formulation commerciale d'insuline humaine (exemple B3).

### C7 : Résultats de pharmacodynamie et de pharmacocinétique des solutions d'insulines des exemples B2 et B11

| **Exemple** | **Insuline** | **Composé** | **Composé polyanionique** | **Nombre de cochons** |
|---|---|---|---|---|
| B2 | Lispro | - | - | 26 |
| B11 | Lispro | 2 | Citrate 9,3 mM | 23 |

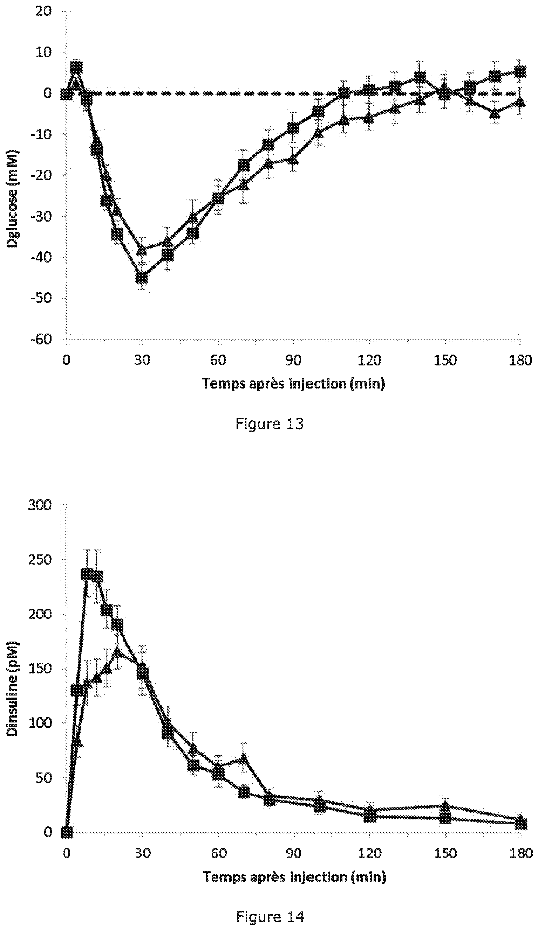

Les résultats de pharmacodynamie obtenus avec les formulations décrites dans les exemples B2 et B11 sont présentés en figure 13. Selon l'invention, l'analyse de ces courbes montre que la formulation de l'exemple B11 comprenant le composé 2 et le citrate à 9,3 mM comme excipients (courbe tracée avec les carrés correspondant à l'exemple B11, Tmin glucose = 32 ± 10 min) permet d'obtenir une action plus rapide que celle de la formulation commerciale Humalog® de l'exemple B2 (courbe tracée avec les triangles correspondant à l'exemple B2, Tmin glucose = 41 ± 21 min).

Les résultats de pharmacocinétique obtenus avec les formulations décrites dans les exemples B2 et B11 sont présentés en figure 14. Selon l'invention, l'analyse de ces courbes montre que la formulation de l'exemple B11 comprenant le composé 2 et le citrate à 9,3 mM comme excipients (courbe tracée avec les carrés correspondant à l'exemple B11, Tmax insuline = 13 ± 5 min) induit une absorption plus rapide de l'insuline lispro que la formulation commerciale Humalog® de l'exemple B2 (courbe tracée avec les triangles correspondant à l'exemple B2 , Tmax insuline = 22 ± 13 min).

### C8 : Résultats de pharmacodynamie et de pharmacocinétique des solutions d'insulines des exemples B1 et B38

| **Exemple** | **Insuline** | **Composé** | **Composé polyanionique** | **Nombre de cochons** |
|---|---|---|---|---|
| B1 | Aspart | - | - | 37 |
| B38 | Humaine | 2 | Citrate 9,3 mM | 31 |

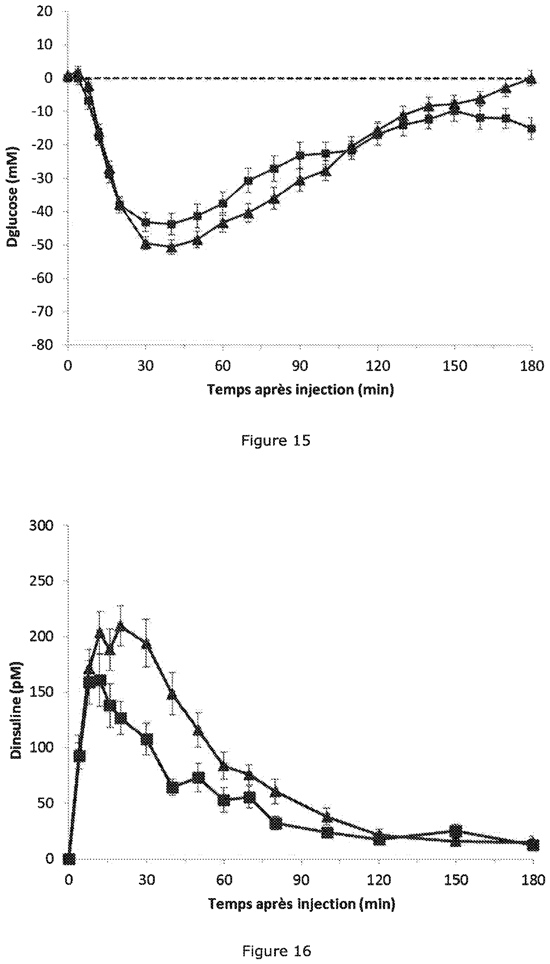

Les résultats de pharmacodynamie obtenus avec les formulations décrites dans les exemples B1 et B38 sont présentés en figure 15. L'analyse de ces courbes montre que la formulation à base d'insuline humaine de l'exemple B38 comprenant le composé 2 et le citrate à 9,3 mM comme excipients (courbe tracée avec les carrés correspondant à l'exemple B102, Tmin glucose = 47 ± 30 min) permet d'obtenir une action similaire à celle de la formulation commerciale d'insuline aspart (Novolog®) de l'exemple B1 (courbe tracée avec les triangles correspondant à l'exemple B1, Tmin glucose = 47 ± 15 min).

Les résultats de pharmacocinétique obtenus avec les formulations décrites dans les exemples B1 et B38 sont présentés en figure 16. L'analyse de ces courbes montre que la formulation de l'exemple B38 comprenant le composé 2 et le citrate à 9,3 mM comme excipients (courbe tracée avec les carrés correspondant à l'exemple B38, Tmax insuline = 22 ± 21 min) induit une absorption de l'insuline humaine similaire à celle de la formulation commerciale d'insuline aspart (Novolog®) de l'exemple B1 (courbe tracée avec les triangles correspondant à l'exemple B1, Tmax insuline = 19 ± 12 min).

Les paramètres de temps de l'insuline aspart (Novolog®) entre les exemples C5 et C8 étant proches, on peut en déduire par extrapolation que la formulation de l'exemple B38 induit une accélération de la baisse de la glycémie et de l'absorption de l'insuline humaine par rapport à la formulation commerciale d'insuline humaine (exemple B3).

### C9: Résultats de pharmacodynamie et de pharmacocinétique des solutions d'insulines des exemples B1 et B53

| **Exemple** | **Insuline** | **Composé** | **Composé polyanionique** | **Nombre de cochons** |
|---|---|---|---|---|
| B1 | Aspart | - | - | 12 |
| B53 | Humaine | Composé 8 | Citrate 9,3 mM | 8 |

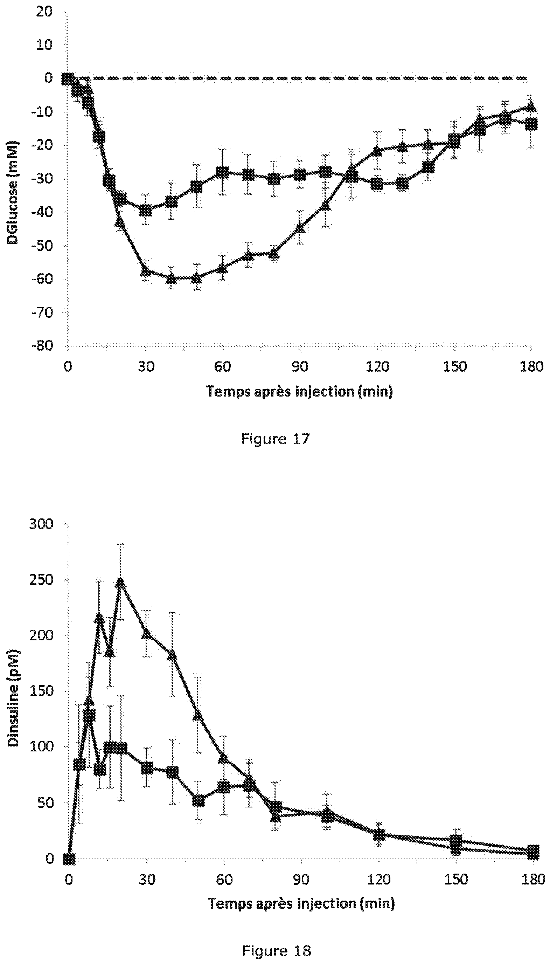

Les résultats de pharmacodynamie obtenus avec les formulations décrites dans les exemples B1 et B53 sont présentés sur la figure 17. L'analyse de ces courbes montre que la formulation à base d'insuline humaine de l'exemple B53 comprenant le composé 8 et le citrate à 9,3 mM comme excipients (courbe tracée avec les carrés correspondant à l'exemple B53, Tmin glucose = 63 ± 36 min) permet d'obtenir une action quasi aussi rapide de celle de la formulation commerciale d'insuline aspart (Novolog®) de l'exemple B1 (courbe tracée avec les triangles correspondant à l'exemple B1, Tmin glucose = 53 ± 19 min).

Les résultats de pharmacocinétique obtenus avec les formulations décrites dans les exemples B1 et B53 sont présentés sur la figure 18. L'analyse de ces courbes montre que la formulation de l'exemple B53 comprenant le composé 8 et le citrate à 9,3 mM comme excipients (courbe tracée avec les carrés correspondant à l'exemple B53, Tmax insuline = 19 ± 12 min) induit une absorption de l'insuline humaine similaire à celle de la formulation commerciale d'insuline aspart (Novolog®) de l'exemple B1 (courbe tracée avec les triangles correspondant à l'exemple B1, Tmax insuline = 19 ± 6 min).

Les paramètres de temps de l'insuline aspart (Novolog®) entre les exemples C5 et C9 étant proches, on peut en déduire par extrapolation que la formulation de l'exemple B53 induit une accélération de la baisse de la glycémie et de l'absorption de l'insuline humaine par rapport à la formulation commerciale d'insuline humaine (exemple B3).

### D Dichroïsme circulaire

### D1 : Etat d'association de l'insuline lispro évalué par dichroïsme circulaire en présence du composé 1

Le dichroïsme circulaire permet d'étudier la structure secondaire et quaternaire de l'insuline. Les monomères d'insuline s'organisent en dimères et en hexamères. L'hexamère est la forme de l'insuline la plus stable physiquement et chimiquement. Il existe deux formes hexamèriques, la forme R6 et la forme T6. L'insuline lispro présente un signal CD fort à 251 nm caractéristique de la forme hexamèrique R6 (forme la plus stable). La perte du signal CD à 251 nm est reliée à une déstabilisation de l'hexamère (et donc le premier signe de transformation de l'hexamère en dimère).

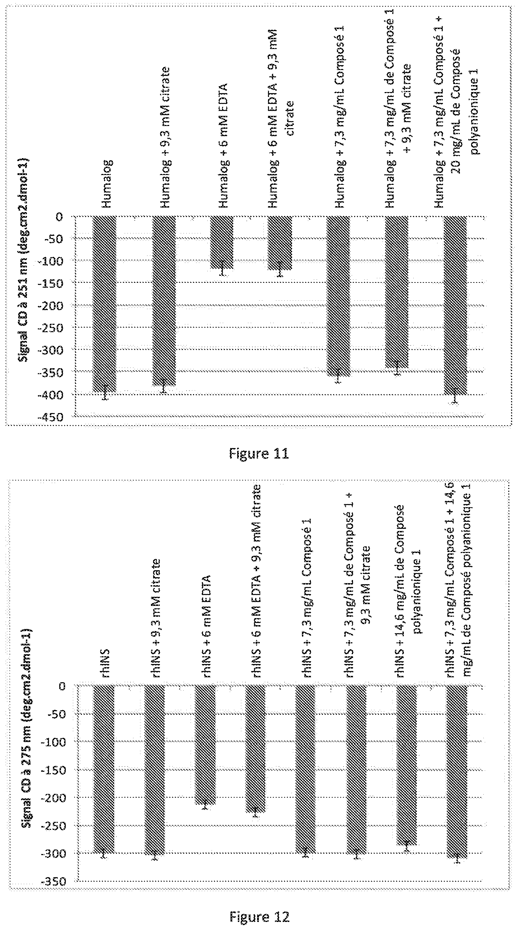

L'EDTA et le mélange EDTA/citrate déstructurent complètement la forme R6 de l'insuline lispro (figure 11). L'EDTA a donc un effet marqué sur l'hexamère.

Au contraire, le citrate seul, le composé 1 seul ainsi que le mélange composé 1/citrate et composé 1/composé polyanionique 1 n'ont quasiment pas d'impact sur le signal CD à 251 nm. Ces composés n'ont donc quasiment aucun impact sur la structure R6 de l'hexamère et a fortiori sur la structure hexamèrique.

### D2 : Etat d'association de l'insuline humaine évalué par dichroïsme circulaire en présence du composé 1

Le dichroïsme circulaire permet d'étudier la structure secondaire et quaternaire de l'insuline. Les monomères d'insuline s'organisent en dimères et en hexamères. L'hexamère est la forme de l'insuline la plus stable physiquement et chimiquement. Le signal CD à 275 nm est caractéristique de la forme hexamèrique de l'insuline (signal hexamère aux alentours de -300°, signal du dimère entre -200° et - 250° et signal du monomère en-dessous de -200°). La perte du signal CD à 275 nm est donc caractéristique d'une déstabilisation de l'hexamère en dimères ou monomères.

L'EDTA et la combinaison EDTA/citrate ont un impact très marqué sur la structure hexamèrique de l'insuline humaine (dissociation complète de l'hexamère en dimères, figure 12). Au contraire, le citrate seul, le composé 1 seul, le composé polyanionique 1 seul ainsi que les combinaisons composé 1/citrate et composé 1/composé polyanionique 1 n'ont pas d'impact sur la structure hexamèrique de l'insuline humaine. Contrairement à l'EDTA, les formulations d'insuline humaine comprenant le composé 1 et le citrate ou le composé polyanionique 1 ne présentent pas de dissociation de l'hexamère de l'insuline humaine.

### D3 : Etat d'association de l'insuline lispro évalué par dichroïsme circulaire en présence des composés 1 à 11

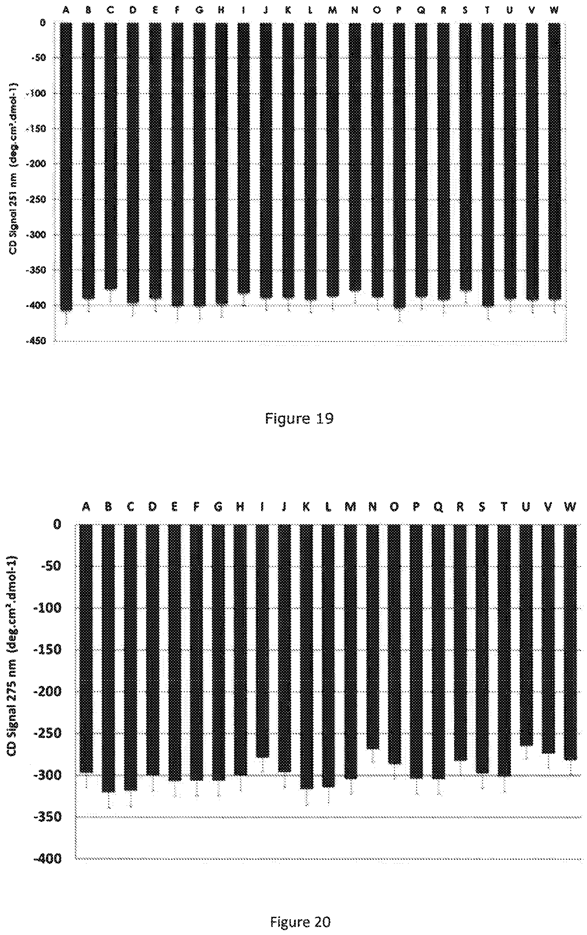

Le dichroïsme circulaire permet d'étudier la structure secondaire et quaternaire de l'insuline. Les monomères d'insuline s'organisent en dimères et en hexamères. L'hexamère est la forme de l'insuline la plus stable physiquement et chimiquement. Il existe deux formes hexamèriques, la forme R6 et la forme T6. L'insuline lispro présente un signal CD fort à 251 nm caractéristique de la forme hexamèrique R6 (forme la plus stable). La perte du signal CD à 251 nm est reliée à une déstabilisation de l'hexamère (et donc le premier signe de transformation de l'hexamère en dimère). Les résultats obtenus sont présentés en figure 19. Cette figure décrit en abscisse :
- A : insuline lispro (100 UI/mL)
- B : insuline lispro + 7,3 mg/mL de composé 2
- C : insuline lispro + 7,3 mg/mL de composé 2 + citrate à 9,3 mM
- D : insuline lispro + 7,3 mg/mL de composé 1
- E : insuline lispro + 7,3 mg/mL de composé 1 + citrate à 9,3 mM
- F : insuline lispro + 7,3 mg/mL de composé 3
- G : insuline lispro + 7,3 mg/mL de composé 3 + citrate à 9,3 mM
- H : insuline lispro + 7,3 mg/mL de composé 4
- I : insuline lispro + 7,3 mg/mL de composé 4 + citrate à 9,3 mM
- J : insuline lispro + 7,3 mg/mL de composé 5
- K : insuline lispro + 7,3 mg/mL de composé 5 + citrate à 9,3 mM
- L : insuline lispro + 7,3 mg/mL de composé 6
- M : insuline lispro + 7,3 mg/mL de composé 6 + citrate à 9,3 mM
- N : insuline lispro + 7,3 mg/mL de composé 7
- O : insuline lispro + 7,3 mg/mL de composé 7 +citrate à 9,3 mM
- P : insuline lispro + 7,3 mg/mL de composé 8
- Q : insuline lispro + 7,3 mg/mL de composé 8 + citrate à 9,3 mM
- R : insuline lispro + 7,3 mg/mL de composé 9
- S : insuline lispro + 7,3 mg/mL de composé 9 + citrate à 9,3 mM
- T : insuline lispro + 7,3 mg/mL de composé 10
- U : insuline lispro + 7,3 mg/mL de composé 10 + citrate à 9,3 mM
- V : insuline lispro + 7,3 mg/mL de composé 11
- W : insuline lispro + 7,3 mg/mL de composé 11 + citrate à 9,3 mM
et en ordonnée le signal dichroisme circulaire à 251 nm (deg.cm².dmol⁻¹).

Les composés 1 à 11 seuls ainsi que les composés 1 à 11 en combinaison avec le citrate n'ont pas d'impact sur le signal CD à 251 nm de l'insuline lispro. Les composés 1 à 11 n'ont donc aucun impact sur la structure R6 de l'hexamère et a fortiori sur la structure hexamèrique de l'insuline lispro.

### D4 : Etat d'association de l'insuline humaine évalué par dichroïsme circulaire en présence des composés 1 à 11

Le dichroïsme circulaire permet d'étudier la structure secondaire et quaternaire de l'insuline. Les monomères d'insuline s'organisent en dimères et en hexamères. L'hexamère est la forme de l'insuline la plus stable physiquement et chimiquement. Le signal CD à 275 nm est caractéristique de la forme hexamèrique de l'insuline (signal hexamère aux alentours de -300°, signal du dimère entre -200° et - 250° et signal du monomère en-dessous de -200°). La perte du signal CD à 275 nm est donc caractéristique d'une déstabilisation de l'hexamère en dimères ou monomères. Les résultats obtenus sont présentés en figure 20. Cette figure décrit en abscisse :
A : insuline humaine (100 UI/mL)
B: insuline humaine + 7,3 mg/mL de composé 2
C : insuline humaine + 7,3 mg/mL de composé 2 + citrate à 9,3 mM
D : insuline humaine + 7,3 mg/mL de composé 1
E : insuline humaine + 7,3 mg/mL de composé 1 + citrate à 9,3 mM
F : insuline humaine + 7,3 mg/mL de composé 3
G : insuline humaine + 7,3 mg/mL de composé 3 + citrate à 9,3 mM
H : insuline humaine + 7,3 mg/mL de composé 4
I : insuline humaine + 7,3 mg/mL de composé 4 + citrate à 9,3 mM
J : insuline humaine + 7,3 mg/mL de composé 5
K : insuline humaine + 7,3 mg/mL de composé 5 + citrate à 9,3 mM
L : insuline humaine + 7,3 mg/mL de composé 6
M : insuline humaine + 7,3 mg/mL de composé 6 + citrate à 9,3 mM
N : insuline humaine + 7,3 mg/mL de composé 7
O : insuline humaine + 7,3 mg/mL de composé 7 + citrate à 9,3 mM
P : insuline humaine + 7,3 mg/mL de composé 8
Q : insuline humaine + 7,3 mg/mL de composé 8 + citrate à 9,3 mM
R : insuline humaine + 7,3 mg/mL de composé 9
S : insuline humaine + 7,3 mg/mL de composé 9 + citrate à 9,3 mM
T : insuline humaine + 7,3 mg/mL de composé 10
U : insuline humaine + 7,3 mg/mL de composé 10 + citrate à 9,3 mM
V : insuline humaine + 7,3 mg/mL de composé 11
W : insuline humaine + 7,3 mg/mL de composé 11 + citrate à 9,3 mM
et en ordonnée le signal dichroisme circulaire à 275 nm (deg.cm².dmol⁻¹).

Les composés 1 à 11 seuls ainsi que les composés 1 à 11 en combinaison avec le citrate n'ont pas d'impact sur le signal CD à 275 nm de l'insuline humaine. Les composés 1 à 11 n'ont donc aucun impact sur la structure hexamèrique de l'insuline humaine.

### E Solubilisation d'insulines humaine et analogue au point isoélectrique

### E1. solubilisation de l'insuline humaine à son point isoélectrique

L'insuline humaine a un point isoélectrique à 5,3. A ce pH de 5,3 l'insuline humaine précipite. Un test démontrant la formation d'un complexe de l'insuline humaine avec les différents composés est exécuté au point isoélectrique. Si une interaction existe, il est possible de solubiliser l'insuline à son point isoélectrique.

Une solution d'insuline humaine à 200 UI/mL est préparée. Des solutions de composés à différentes concentrations (8, 30 ou 100 mg/mL) dans l'eau sont préparées. Un mélange équivolume (50/50) entre la solution d'insuline humaine et la solution de composé est effectué pour mener à une solution contenant 100 UI/mL d'insuline humaine et la concentration désirée en composé (4, 15 ou 50 mg/mL). Le pH des différentes solutions est ajusté à pH 5,3 par ajout d'acide acétique 200 mM.

L'aspect de la solution est documenté. Si la solution est turbide, le composé à la concentration testée ne permet pas la solubilisation de l'insuline humaine. Si la solution est translucide, le composé permet la solubilisation de l'insuline humaine à la concentration testée. De cette façon, la concentration en composé nécessaire pour solubiliser l'insuline humaine à son point isoélectrique peut être déterminée. Plus cette concentration est basse plus l'affinité du composé pour l'insuline humaine est importante.

Les résultats obtenus sont présentés dans le Tableau 3. Les résultats montrent que les composés et les polysaccharides ne présentent pas les mêmes propriétés en termes de solubilisation de l'insuline humaine.

**Tableau 3**

| **Composés (exemples) ou Polysaccharides (contre-exemples)** | **Solubilisation de l'insuline humaine à 100 UI/mL par le composé à 4 mg/mL** | **Solubilisation de l'insuline humaine à 100 UI/mL par le composé à 15 mg/mL** | **Solubilisation de l'insuline humaine à 100 UI/mL par le composé à 50 mg/mL** |
|---|---|---|---|
| **Contre exemples** | | | |
| Polysaccharide 1 | Oui | Oui | Oui |
| Polysaccharide 4 | Oui | Oui | Oui |
| Polysaccharide 3 | Oui | Oui | Oui |
| Polysaccharide 2 | Oui | Oui | Oui |
| Polysaccharide 5 | Oui | Oui | Oui |

| **Exemples** | | | |
|---|---|---|---|
| Composé 1 | Non | Non | Oui |
| Composé 2 | Non | Non | Oui |
| Composé 3 | Non | Non | Oui |
| Composé 4 | Non | Non | Oui |
| Composé 6 | Non | Non | Oui |
| Composé 8 | Non | Non | Oui |
| Composé 9 | Non | Non | Oui |
| Composé 10 | Non | Non | Oui |

### E2.Solubilisation de l'insuline lispro à son point isoélectrique

L'insuline lispro a un point isoélectrique à 5,3. A ce pH l'insuline lispro précipite. Un test démontrant la formation d'un complexe de l'insuline lispro avec les différents composés est exécuté au point isoélectrique. Si une interaction existe, il est possible de solubiliser l'insuline lispro à son point isoélectrique.

La formulation commerciale de l'insuline lispro (Humalog®) est dialysée contre du tampon PO₄ 1 mM (pH 7). Après dialyse, la concentration en insuline lispro est d'environ 90 UI/mL. Le lyophilisat de composé est pesé et solubilisé dans la solution d'insuline lispro pour mener à des formulations contenant l'insuline lispro à 90 UI/mL et le composé aux concentrations désirées (4, 15 ou 50 mg/mL). Le pH des différentes solutions est ajusté à pH 5,3 par ajout d'acide acétique 200 mM.

L'aspect de la solution est documenté. Si la solution est turbide, le composé à la concentration testée ne permet pas la solubilisation de l'insuline lispro. Si la solution est translucide, le composé permet la solubilisation de l'insuline lispro à la concentration testée. De cette façon, la concentration en composé nécessaire pour solubiliser l'insuline lispro à son point isoélectrique peut être déterminée. Plus cette concentration est basse plus l'affinité de composé pour l'insuline lispro est importante.

Les résultats obtenus sont présentés dans le Tableau 4. Les résultats montrent que les composés et les polysaccharides ne présentent pas les mêmes propriétés en termes de solubilisation de l'insuline lispro.

**Tableau 4**

| **Composés (exemples) ou Polysaccharides (contre-exemples)** | **Solubilisation de l'insuline lispro à 90 UI/mL par le composé à 4 mg/mL** | **Solubilisation de l'insuline lispro à 90 UI/mL par le composé à 15 mg/mL** | **Solubilisation de l'insuline lispro à 90 UI/mL par le composé à 50 mg/mL** |
|---|---|---|---|
| **Contre exemples** | | | |
| Polysaccharide 1 | Oui | Oui | Oui |
| Polysaccharide 3 | Oui | Oui | Oui |
| Polysaccharide 2 | Oui | Oui | Oui |

| **Exemples** | | | |
|---|---|---|---|
| Composé 1 | Non | Non | Oui |
| Composé 2 | Non | Non | Oui |
| Composé 3 | Non | Non | Oui |

### F Interaction avec l'albumine

**F1** : Afin de déterminer les interactions entre les différents polysaccharides ou composés et une protéine modèle telle que l'albumine, un essai en Centricon (membrane de CutOff 50 kDa) a été effectué. Une solution de polysaccharide ou de composé à 7,3 mg/mL a été diluée au tiers dans une solution de BSA (albumine du sérum bovin) à 20 mg/mL dans le PBS (concentration dans le mélange : 2,43 mg/mL de polysaccharide ou de composé, 13,3 mg/mL d'albumine et environ 100 mM de sels).

Ce mélange a été centrifugé sur Centricon pour faire passer environ la moitié du volume à travers la membrane. L'albumine est retenue de façon quantitative sur la membrane du Centricon. Les polysaccharides et composés analysés seuls passent en grande partie à travers la membrane (pour les polysaccharides ayant des masses molaires les plus importantes, environ 20% du polysaccharide est retenu).

Après centrifugation le polysaccharide ou composé est dosé par UV dans le filtrat. Le pourcentage de polysaccharide ou composé lié à l'albumine est calculé par l'équation suivante :

(1-[polysaccharide ou composé dans le filtrat en présence d'albumine]/[polysaccharide ou composé dans le filtrat en absence d'albumine])*100

Les résultats obtenus sont présentés dans le Tableau 5. On observe très clairement que les polysaccharides de masse molaire 5-15 kDa sont fortement retenus par l'albumine dans cet essai. Au contraire, les composés de l'invention de plus faible masse molaire sont nettement moins retenus par l'albumine dans cet essai.

**Tableau 5**

| **Polysaccharide ou Composé** | **% Polysaccharide ou % Composé lié à la BSA** |
|---|---|
| **Contre exemples** | |
| Polysaccharide 4 | 97 % |
| Polysaccharide 1 | 95% |
| Polysaccharide 3 | 77% |
| Polysaccharide 5 | 86% |
| Polysaccharide 2 | 82% |

| **Exemples** | |
|---|---|
| Composé 2 | 21 % |
| Composé 1 | 20 % |
| Composé 3 | 27 % |
| composé 4 | 24% |
| Composé 5 | 24% |
| Composé 6 | 26% |
| Composé 7 | 27% |
| Composé 8 | 27% |
| Composé 9 | 43% |
| Composé 11 | 35% |

## Revendications

1. Composition en solution aqueuse, comprenant de l'insuline sous forme hexamèrique, au moins un composé anionique substitué et un composé polyanionique non polymèrique :
- ledit composé anionique substitué étant choisi parmi les composés anioniques substitués, à l'état isolé ou en mélange, constitués d'un squelette formé d'un nombre discret u compris entre 1 et 8 (1 ≤ u ≤ 8) d'unités saccharidiques identiques ou différentes, liées par des liaisons glycosidiques identiques ou différentes, lesdites unités saccharidiques étant choisies dans le groupe constitué par les hexoses, sous forme cyclique ou sous forme réduite ouverte, **caractérisés en ce qu'**ils sont substitués par :
a) au moins un substituant de formule générale I :
-[R₁]ₐ-[AA]ₘ Formule I
• les substituants étant identiques ou différents lorsqu'il y a au moins deux substituants, dans lequel :
• le radical -[AA]- désigne un résidu d'acide aminé,
• le radical -R₁- étant :
- soit une liaison et alors a = 0, et le résidu d'acide aminé -[AA] est directement lié au squelette par une fonction G.
- soit une chaîne carbonée et alors a = 1, en C2 à C15 éventuellement substituée et/ou comportant au moins un hétéroatome choisi parmi O, N et S et au moins une fonction acide avant la réaction avec l'acide aminé, ladite chaîne formant avec le résidu d'acide aminé -[AA] une fonction amide, et est fixée sur le squelette à l'aide d'une fonction F résultant d'une réaction entre une fonction hydroxyle portée par le squelette et une fonction ou un substituant porté par le précurseur du radical -R₁-,
• F est une fonction choisie parmi les fonctions éther, ester ou carbamate,
• G est une fonction carbamate,
• m est égal à 1 ou 2,
• le degré de substitution des unités saccharidiques, j, en -[R₁]ₐ-[AA]ₘ étant strictement supérieur à 0 et inférieur ou égal à 6, 0 < j ≤ 6
b) et, éventuellement, un ou plusieurs substituants -R'₁,
• le substituant -R'₁ étant une chaîne carbonée en C2 à C15, éventuellement substituée et/ou comportant au moins un hétéroatome choisi parmi O, N et S et au moins une fonction acide sous forme de sel de cations alcalins ladite chaine étant liée au squelette par une fonction F' résultant d'une réaction entre une fonction hydroxyle ou une fonction acide carboxylique portée par le squelette et une fonction ou un substituant porté par le précurseur du substituant -R'₁,
• F' est une fonction éther, ester ou carbamate,
• le degré de substitution des unités saccharidiques, i, en -R'₁, étant compris entre 0 et 6-j, 0 ≤ i ≤ 6-j et,
• F et F' sont identiques ou différentes,
• F et G sont identiques ou différentes,
• i+j ≤ 6.
• -R'₁ identique ou différent de -R₁-,
• Les fonctions acides salifiables libres portées par le substituant -R'₁ sont sous forme de sels de cations alcalins,
• lesdites liaisons glycosidiques identiques ou différentes étant choisies dans le groupe constitué par les liaisons glycosidiques de type (1,1), (1,2), (1,3), (1,4) ou (1,6), dans une géométrie alpha ou béta.

2. Composition selon la revendication 1, **caractérisée en ce que** le composé polyanionique est choisi dans le groupe constitué des polyacides carboxyliques ou dans le groupe constitué des polyacides phosphoriques ou dans le groupe des composés constitués d'un squelette saccharidique formé d'un nombre discret d'unités saccharidiques obtenus à partir d'un composé disaccharide choisi dans le groupe constitué par le tréhalose, le maltose, le lactose, le saccharose, le cellobiose, l'isomaltose, le maltitol et l'isomaltitol ou dans le groupe des composés polyanioniques constitués d'un squelette saccharidique formé d'un nombre discret d'unités saccharidiques choisis dans le groupe constitué par le carboxyméthylmaltotriose, le carboxyméthylmaltotétraose, le carboxyméthylmaltopentaose, le carboxyméthylmaltohexaose, le carboxyméthylmaltoheptaose, le carboxyméthylmaltooactose et le carboxyméthylisomaltotriose et leurs sels de Na⁺, K⁺, Ca²⁺ ou Mg²⁺.

3. Composition selon la revendication 2, **caractérisée en ce que**, le polyacide carboxylique est choisi dans le groupe constitué par l'acide citrique, l'acide tartrique, et leurs sels de Na⁺, K⁺, Ca²⁺ ou Mg²⁺.

4. Composition selon la revendication 2, **caractérisée en ce que** le composé polyanionique constitué d'un squelette saccharidique formé d'un nombre discret d'unités saccharidiques est choisi dans le groupe constitué par le carboxyméthylmaltotriose, le carboxyméthylmaltotétraose, le carboxyméthylmaltopentaose, le carboxyméthylmaltohexaose, le carboxyméthylmaltoheptaose, le carboxyméthylmaltooactose et le carboxyméthylisomaltotriose. et leurs sels de Na⁺, K⁺, Ca²⁺ ou Mg²⁺.

5. Composition selon l'une quelconque des revendications précédentes, **caractérisée en ce que** l'insuline est une insuline humaine.

6. Composition selon l'une des revendications 1 à 4, **caractérisée en ce que** l'insuline est une insuline analogue.

7. Composition selon la revendication 6, **caractérisée en ce que** l'insuline analogue est choisie dans le groupe constitué par l'insuline lispro (Humalog®), l'insuline aspart (Novolog®, Novorapid®) et l'insuline glulisine (Apidra®).

8. Composition selon la revendication 7, **caractérisée en ce que** l'insuline analogue est l'insuline lispro (Humalog®).

9. Composition selon l'une quelconque des revendications précédentes, **caractérisée en ce que** le ratio massique composé anionique substitué/insuline est compris entre 0,5 et 10.

10. Composition selon l'une quelconque des revendications précédentes, **caractérisée en ce que** la concentration en composé anionique substitué est comprise entre 1,8 et 36 mg/mL.

11. Formulation pharmaceutique comprenant une composition selon l'une quelconque des revendications précédentes.

12. Formulation pharmaceutique selon la revendication 11, **caractérisée en ce que** la concentration en insuline est comprise entre 240 et 3000 µM (40 à 500 UI/mL).

13. Utilisation d'au moins un composé anionique substitué constitué d'un squelette formé d'un nombre discret u compris entre 1 et 8 ≤ u ≤ 8) d'unités saccharidiques identiques ou différentes, liées par des liaisons glycosidiques identiques ou différentes, lesdites unités saccharidiques étant choisies dans le groupe constitué par, les hexoses, sous forme cyclique ou sous forme réduite ouverte, ledit composé comportant des groupes fonctionnels carboxyles partiellement substitués, les groupes fonctionnels carboxyles non substitués étant salifiables pour préparer une formulation pharmaceutique d'insuline humaine analogue en mélange avec un composé polyanionique permettant, après administration, d'accélérer le passage de l'insuline dans le sang et de réduire plus rapidement la glycémie par rapport à une formulation exempte de composé anionique substitué et éventuellement de composé polyanionique.

14. Utilisation selon la revendication 13, **caractérisée en ce que** le composé anionique substitué, est choisi parmi les composés anioniques substitués constitués d'un squelette saccharidique formé d'un nombre discret u compris entre 1 et 8 (1 ≤ u ≤ 8) d'unités saccharidiques identiques ou différentes, liées par des liaisons glycosidiques identiques ou différentes, lesdites unités saccharidiques étant choisies parmi les hexoses, sous forme cyclique ou sous forme réduite ouverte, **caractérisés** :
a) **en ce qu'**ils sont substitués de façon statistique par :
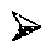 au moins un substituant de formule générale I :
-[R₁]ₐ-[AA]ₘ Formule I
• les substituants étant identiques ou différents lorsqu'il y a au moins deux substituants, dans lequel :
• le radical -[AA]- désigne un résidu d'acide aminé, ledit acide aminé étant choisi dans le groupe constitué de la phénylalanine, l'alpha-méthyl-phénylalanine, la 3,4 dihydroxyphénylalanine la tyrosine, l'alpha-méthyl-tyrosine, la O-méthyl-tyrosine, l'alpha-phénylglycine, la 4-hydroxyphénylglycine, la 3,5-dihydroxyphénylglycine et leurs sels de cations alcalins, lesdits dérivés étant de configuration absolue L ou D, -[AA]- est fixé sur le squelette de la molécule par l'intermédiaire d'un bras de liaison -R₁- ou directement lié au squelette par une fonction G,
• -R₁- étant :
- soit une liaison G, et alors a = 0,
- soit une chaîne carbonée, et alors a = 1, en C1 à C15 éventuellement substituée et/ou comportant au moins un hétéroatome choisi parmi O, N et S et portant au moins une fonction acide avant la réaction avec l'acide aminé, ladite chaîne formant avec le résidu d'acide aminé -[AA]- une liaison amide, et est fixée sur le squelette saccharidique à l'aide d'une fonction F résultant d'une réaction entre une fonction hydroxyle portée par le squelette et une fonction portée par le précurseur de R₁,
• F est une fonction éther, ester ou carbamate,
• G est une fonction ester ou carbamate,
• m est égal à 1 ou 2,
• le degré de substitution, j, en -[R₁]ₐ-[AA]ₘ étant strictement supérieur à 0 et inférieur ou égal à 6, 0 < j ≤ 6,
et, éventuellement,
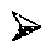 un ou plusieurs substituants -R'₁
• -R'₁ étant une chaîne carbonée en C2 à C15, éventuellement substituée et/ou comportant au moins un hétéroatome (tels que O, N et S) et portantau moins une fonction acide sous forme de sel de cations alcalins ladite chaine étant fixée sur le squelette saccharidique par une fonction F' résultant d'une réaction entre une fonction hydroxyle portée par le squelette et une fonction portée par le précurseur de -R'₁,
• F' est une fonction éther, ester ou carbamate,
• le degré de substitution i, en -R'₁, étant compris entre 0 et 6-j, 0 ≤ i ≤ 6-j, et,
• -R'₁- identique ou différent de -R₁,
• F et F' identiques ou différentes,
• F' et G identiques ou différentes
• Les fonctions acides salifiables libres sont sous forme de sels de cations alcalins,
b) lesdites liaisons glycosidiques identiques ou différentes étant choisies dans le groupe constitué par les liaisons glycosidiques de type (1,1), (1,2), (1,3), (1,4) ou (1,6), dans une géométrie alpha ou béta,
c) i +j ≤ 6

15. Utilisation selon l'une des revendications 13 à 14, **caractérisée en ce que** le composé polyanionique est choisi dans le groupe constitué des polyacides carboxyliques ou dans le groupe constitué des polyacides phosphoriques ou dans le groupe des composés constitués d'un squelette saccharidique formé d'un nombre discret d'unités saccharidiques obtenus à partir d'un composé disaccharide choisi dans le groupe constitué par le tréhalose, le maltose, le lactose, le saccharose, le cellobiose, l'isomaltose, le maltitol et l'isomaltitol ou dans le groupe des composés polyanioniques constitués d'un squelette saccharidique formé d'un nombre discret d'unités saccharidiques choisis dans le groupe constitué par le carboxyméthylmaltotriose, le carboxyméthylmaltotétraose, le carboxyméthylmaltopentaose, le carboxyméthylmaltohexaose, le carboxyméthylmaltoheptaose, le carboxyméthylmaltooactose et le carboxyméthylisomaltotriose et leurs sels de Na⁺, K⁺, Ca²⁺ ou Mg²⁺.

## Patentansprüche

1. Zusammensetzung in wässriger Lösung umfassend Insulin in hexamerer Form, wenigstens eine substituierte anionische Verbindung und eine nicht-polymere polyanionische Verbindung:
- die substituierte anionische Verbindung ist ausgewählt aus substituierten anionischen Verbindungen, isoliert oder als eine Mischung, bestehend aus einem Rückgrat, das gebildet ist aus einer diskreten Anzahl u von zwischen 1 und 8 (1 ≤ u ≤ 8) identischen oder verschiedenen Saccharideinheiten, verknüpft durch identische oder verschiedene glykosidische Bindungen, wobei die Saccharideinheiten ausgewählt sind aus der Gruppe bestehend aus Hexosen, in zyklischer Form oder offener reduzierter Form, **dadurch gekennzeichnet, dass** sie substituiert sind mit
a) wenigstens einem Substituenten mit der allgemeinen Formel I:
- [R₁]ₐ-[AA]ₘ Formel I
• die Substituenten identisch oder verschieden sind, wenn mindestens zwei Substituenten vorhanden sind, wobei
• das Radikal - [AA] - einen Aminosäurerest bezeichnet,
• das Radikal -R₁-
- entweder eine Bindung ist und somit a = 0 ist, und der Aminosäurerest -[AA] direkt mit dem Rückgrat durch eine Funktion G verbunden ist,
- oder eine C2- bis C15-Kohlenstoffkette ist und somit a = 1 ist, die optional substituiert ist und/oder wenigstens ein Heteroatom, das ausgewählt ist aus O, N und S, und wenigstens eine Säurefunktion vor der Reaktion mit der Aminosäure umfasst, wobei die Kette mit dem Aminosäurerest -[AA] eine Amidfunktion ausbildet, und an das Rückgrat gebunden ist mittels einer Funktion F, die ausgebildet ist durch eine Reaktion zwischen einer von dem Rückgrat getragenen Hydroxylfunktion und einer Funktion oder einem Substituenten, die/der von dem Vorläufer des Radikals -R1- getragen ist,
• F eine Funktion ist, die ausgewählt ist aus Ether-, Ester- oder Carbamatfunktionen,
• G eine Carbamatfunktion ist,
• m 1 oder 2 ist,
• der Substitutionsgrad der Saccharideinheiten, j, in - [R₁]ₐ-[AA]ₘ genau größer als 0 und kleiner oder gleich 6 ist, 0 < j ≤ 6 ,
b) und, optional, einem oder mehreren Substituenten -R'₁,
• der Substituent -R'₁ eine C2- bis C15-Kohlenstoffkette ist, die optional substituiert ist und/oder wenigstens ein Heteroatom, das ausgewählt ist aus O, N und S, und wenigstens eine Säurefunktion in der Form eines Alkalimetallkationensalzes umfasst, wobei die Kette mit dem Rückgrat durch eine Funktion F' verknüpft ist, die sich ergibt aus einer Reaktion zwischen einer Hydroxylfunktion oder einer Carbonsäurefunktion, die von dem Rückgrat getragen ist, und einer Funktion oder einem Substituenten, die/der von dem Vorläufer des Substituenten -R'₁ getragen ist,
• F' eine Ether-, Ester oder Carbamatfunktion ist,
• der Substitutionsgrad der Saccharideinheiten, i, in -R'₁, zwischen 0 und 6-j ist, 0 ≤ i ≤ 6-j, und
• F und F' identisch oder verschieden sind,
• F und G identisch oder verschieden sind,
• i + j ≤ 6,
• -R'₁ identisch mit oder verschieden von -R₁- ist,
• die von dem Substituenten -R'₁ getragenen freien salzbildenden Säurefunktionen in der Form von Alkalimetallkationensalzen vorliegen,
• die identischen oder verschiedenen glykosidischen Bindungen ausgewählt sind aus der Gruppe bestehend aus glykosidischen Bindungen vom Typ (1,1), (1,2), (1,3), (1,4) oder (1,6), in einer alpha- oder beta-Konfiguration.

2. Zusammensetzung nach Anspruch 1, **dadurch gekennzeichnet, dass** die polyanionische Verbindung ausgewählt ist aus der Gruppe bestehend aus Polycarbonsäuren oder der Gruppe bestehend aus Polyphosphorsäuren oder der Gruppe bestehend aus Verbindungen bestehend aus einem Saccharidrückgrat, das durch eine diskrete Anzahl von Saccharideinheiten gebildet ist, die erhalten sind von einer Disaccharidverbindung, die ausgewählt ist aus der Gruppe bestehend aus Trehalose, Maltose, Lactose, Saccharose, Zellobiose, Isomaltose, Maltitol, und Isomaltitol, oder der Gruppe von polyanionsichen Verbindungen bestehend aus einem Saccharidrückgrat, das aus einer diskreten Anzahl von Saccharideinheiten gebildet ist, die ausgewählt sind aus der Gruppe bestehend aus Carboxymethylmaltotriose, Carboxymethylmaltotetraose, Carboxymethylmaltopentaose, Carboxymethylmaltohexaose, Carboxymethylmaltoheptaose, Carboxymethylmaltooactose und Carboxymethylisomaltotriose sowie deren Na⁺-, K⁺-, Ca²⁺- oder Mg²⁺-Salzen.

3. Zusammensetzung nach Anspruch 2, **dadurch gekennzeichnet, dass** die Polycarbonsäure ausgewählt ist aus der Gruppe bestehend aus Zitronensäure, Weinsäure und deren Na⁺-, K⁺-, Ca²⁺- oder Mg²⁺-Salzen.

4. Zusammensetzung nach Anspruch 2, **dadurch gekennzeichnet, dass** die polyanionische Verbindung, die aus einem Saccharidrückgrat besteht, das aus einer diskreten Anzahl von Saccharideinheiten gebildet ist, ausgewählt ist aus der Gruppe bestehend aus Carboxymethylmaltotriose, Carboxymethylmaltotetraose, Carboxymethylmaltopentaose, Carboxymethylmaltohexaose, Carboxymethylmaltoheptaose, Carboxymethylmaltooactaose und Carboxymethylisomaltotriose sowie deren Na⁺-, K⁺-, Ca²⁺- oder Mg²⁺-Salzen.

5. Zusammensetzung nach einem der vorangehenden Ansprüche, **dadurch gekennzeichnet, dass** das Insulin menschliches Insulin ist.

6. Zusammensetzung nach einem der Ansprüche 1 bis 4, **dadurch gekennzeichnet, dass** das Insulin ein Insulinanalogon ist.

7. Zusammensetzung nach Anspruch 6, **dadurch gekennzeichnet ist, dass** das Insulinanalogon ausgewählt ist aus der Gruppe bestehen aus Insulin Lispro (Humalog®), Insulin Aspart (Novolog®, Novorapid®) und Insulin Glulisine (Apidra®).

8. Zusammensetzung nach Anspruch 7, **dadurch gekennzeichnet, dass** das Insulinanalogon Insulin Lispro (Humalog®) ist.

9. Zusammensetzung nach einem der vorangehenden Ansprüche, **dadurch gekennzeichnet, dass** das Massenverhältnis substituierte anionische Verbindung/Insulin zwischen 0,5 und 10 beträgt.

10. Zusammensetzung nach einem der vorangehenden Ansprüche, **dadurch gekennzeichnet, dass** die Konzentration von substituierter anionischer Verbindung zwischen 1,8 und 36 mg/ml beträgt.

11. Pharmazeutische Formulierung umfassend eine Zusammensetzung nach einem der vorangehenden Ansprüche.

12. Pharmazeutische Formulierung nach Anspruch 11, **dadurch gekennzeichnet, dass** die Insulinkonzentration zwischen 240 und 3000 µM (40 bis 500 IE/ml) beträgt.

13. Verwendung wenigstens einer substituierten anionischen Verbindung bestehend aus einem Rückgrat, das gebildet ist aus einer diskreten Anzahl u von zwischen 1 und 8 (1 ≤ u ≤ 8) identischen oder verschiedenen Saccharideinheiten, verknüpft durch identische oder verschiedene glykosidische Bindungen, wobei die Saccharideinheiten ausgewählt sind aus der Gruppe bestehend aus Hexosen, in zyklischer Form oder offener reduzierter Form, wobei die Verbindung teilweise substituierte funktionelle Carboxylgruppen trägt, die nichtsubstituierten funktionellen Carboxylgruppen salzbildend sind, um eine pharmazeutische Formulierung von menschlichem Insulinanalogon gemischt mit einer polyanionischen Verbindung herzustellen, die, nach Verabreichung, erlaubt, die Passage des Insulins in das Blut zu beschleunigen und, verglichen mit einer Formulierung ohne substituierter anionischer Verbindung und optional polyanionischer Verbindung, Glykämie schneller zu verringern.

14. Verwendung nach Anspruch 13, **dadurch gekennzeichnet, dass** die substituierte anionischer Verbindung ausgewählt ist aus substituierten anionischen Verbindungen, die aus einem Saccharidskelett bestehen und bestehen aus einer diskreten Anzahl u enthalten zwischen 1 und 8 (1 ≤ u ≤ 8) identischen oder verschiedenen Saccharideinheiten, verknüpft durch identische oder verschiedene Glykosidbindungen, wobei die Saccharideiheiten ausgewählt sind aus Hexosen in zyklischer Form oder offener reduzierter Form, **dadurch gekennzeichnet, dass**
a) sie statistisch substituiert sind durch
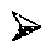 wenigstens einen Substituenten mit der allgemeinen Formel I:
- [R₁]ₐ-[AA]ₘ Formel I
• die Substituenten identisch oder verschieden sind, wenn mindestens zwei Substituenten vorhanden sind, wobei
• das Radikal - [AA] - einen Aminosäurerest bezeichnet, wobei die Aminosäure ausgewählt ist aus der Gruppe bestehend aus Phenylalanin, alpha-Methylphenylalanin, 3,4-Dihydroxyphenylalanin, Tyrosin, alpha-Methyltyrosin, O-Methyltyrosin, alpha-Phenylglycin, 4-Hydroxyphenylglycin, 3,5- Dihydroxyphenylglycin und deren Alkalimetallkationensalze, wobei die Derivate in der absoluten L- oder D-Konfiguration vorliegen, -[AA]- auf dem Rückgrat des Moleküls mittels eines Linkerarms -R1- gebunden ist oder direkt mit dem Rückgrat durch eine Funktion G verknüpft ist,
• -R₁-
- entweder eine Bindung ist und somit a = 0 ist,
- oder eine C1- bis C15-Kohlenstoffkette ist und somit a = 1 ist, die optional substituiert ist und/oder wenigstens ein Heteroatom umfasst, das ausgewählt ist aus O, N und S, und vor der Reaktion mit der Aminosäure wenigstens eine Säurefunktion trägt, wobei die Kette mit dem Aminosäurerest -[AA]- eine Amidfunktion ausbildet, und an das Rückgrat gebunden ist mittels einer Funktion F, die ausgebildet ist durch eine Reaktion zwischen einer von dem Rückgrat getragenen Hydroxylfunktion und einer Funktion, die von dem Vorläufer des Radikals -R1- getragen ist,
• F eine Ether-, Ester- oder Carbamatfunktionen ist,
• G eine Ester- oder Carbamatfunktion ist,
• m 1 oder 2 ist,
• der Substitutionsgrad, j, in - [R₁]ₐ-[AA]ₘ genau größer als 0 und kleiner oder gleich 6 ist, 0 < j ≤ 6 , und, optional, 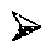
einen oder mehrere Substituenten -R'₁
• -R'₁ eine C2- bis C15-Kohlenstoffkette ist, die optional substituiert ist und/oder wenigstens ein Heteroatom umfasst (wie bspw. O, N und S) und wenigstens eine Säurefunktion in der Form eines Alkalimetallkationensalzes trägt, wobei die Kette an das Saccharidrückgrat durch eine Funktion F' gebunden ist, die sich ergibt aus einer Reaktion zwischen einer Hydroxylfunktion, die von dem Rückgrat getragen ist, und einer Funktion, die von dem Vorläufer des Substituenten -R'₁ getragen ist,
• F' eine Ether-, Ester oder Carbamatfunktion ist,
• der Substitutionsgrad, i, in -R'₁, zwischen 0 und 6-j ist, 0 ≤ i ≤ 6-j, und
• -R'₁- identisch mit oder verschieden von -R₁- ist,
• F und F' identisch oder verschieden sind,
• F' und G identisch oder verschieden sind,
• die freien salzbildenden Säurefunktionen in der Form von Alkalimetallkationensalzen vorliegen,
b) die identischen oder verschiedenen glykosidischen Bindungen ausgewählt sind aus der Gruppe bestehend aus den glykosidischen Bindungen vom Typ (1,1), (1,2), (1,3), (1,4) oder (1,6), in einer alpha- oder beta-Konfiguration.
c) i + j ≤ 6.

15. Verwendung nach einem der Ansprüche 13 bis 14, **dadurch gekennzeichnet, dass** die polyanionische Verbindung ausgewählt ist aus der Gruppe bestehend aus Polycarbonsäuren oder der Gruppe bestehend aus Polyphosphorsäuren oder der Gruppe bestehend aus Verbindungen bestehend aus einem Saccharidrückgrat, das durch eine diskrete Anzahl von Saccharideinheiten gebildet ist, die erhalten sind von einer Disaccharidverbindung, die ausgewählt ist aus der Gruppe umfassend Trehalose, Maltose, Lactose, Saccharose, Zellobiose, Isomaltose, Maltitol, und Isomaltitol, oder der Gruppe von polyanionsichen Verbindungen bestehend aus einem Saccharidrückgrat, das aus einer diskreten Anzahl von Saccharideinheiten gebildet ist, die ausgewählt sind aus der Gruppe bestehend aus Carboxymethylmaltotriose, Carboxymethylmaltotetraose, Carboxymethylmaltopentaose, Carboxymethylmaltohexaose, Carboxymethylmaltoheptaose, Carboxymethylmaltooactose und Carboxymethylisomaltotriose sowie deren Na⁺-, K⁺-, Ca²⁺- oder Mg²⁺-Salzen.

## Claims

1. A composition in aqueous solution, comprising insulin in hexameric form, at least one substituted anionic compound and a nonpolymeric polyanionic compound:
- said substituted anionic compound being chosen from among the substituted anionic compounds in the isolated state or in mixture consisting of a skeleton formed by a discrete number u between l and 8 (l ≤ u ≤ 8) of identical or different saccharide units, linked by identical or different glycoside bonds, said saccharide units being chosen from the group consisting of the hexoses, in ring form or in reduced open form, **characterized in that** they are substituted by:
a) at least one substituent of general formula I:
-[R₁]ₐ-[AA]ₘ Formula I
• the substituents being identical or different when there are at least two substituents, in which:
• the radical -[AA]- denotes an amino acid residue,
• the radical -R₁- being:
either a bond and then a = 0, and the amino acid residue -[AA] is directly connected to the skeleton by a function G.
or a carbon chain and then a = 1, with C2 to C15 possibly substituted and/or comprising at least one heteroatom chosen from among O, N and S and at least one acid function prior to the reaction with the amino acid, said chain forming with the amino acid residue -[AA] an amide function, and is attached to the skeleton with the aid of a function F resulting from a reaction between a hydroxyl function carried by the skeleton and a function or a substituent carried by the precursor of the radical -R₁-,
• F is a function chosen from among the functions ether, ester or carbamate,
• G is a carbamate function,
• m is equal to 1 or 2,
• the degree of substitution of the saccharide units, j, in -[R₁]ₐ-[AA]ₘ being absolutely greater than 0 and less than or equal to 6, 0 < j ≤ 6
b) and, optionally, one or more substituents -R'₁,
• the substituent -R'₁ being a carbon chain with C2 to C15, possibly substituted and/or comprising at least one heteroatom chosen from among O, N and S and at least one acid function in the form of a salt of alkaline cations, said chain being attached to the skeleton by a function F' resulting from a reaction between a hydroxyl function or a carboxylic acid function carried by the skeleton and a function or a substituent carried by the precursor of the substituent -R'₁,
• F' is an ether, ester or carbamate function,
• the degree of substitution of the saccharide units, i, in -R'₁, being between 0 and 6-j, 0 ≤ i ≤ 6-j and,
• F and F' are identical or different,
• F and G are identical or different,
• i+j ≤ 6.
• -R'₁ is identical or different to -R₁-,
• The free salt-forming acid functions carried by the substituent -R'₁ are in the form of salts of alkaline cations,
• said glycosidic bonds, identical or different, being chosen from the group made up of glycosidic bonds of type (1,1), (1,2), (1,3), (1,4) or (1,6), in an alpha or beta geometry.

2. The composition as claimed in claim 1, **characterized in that** the polyanionic compound is chosen from the group made up of carboxylic polyacids or the group made up of phosphoric polyacids or the group made of of compounds comprised of a saccharide skeleton formed by a discrete number of saccharide units obtained from a disaccharide compound chosen from the group made up of trehalose, maltose, lactose, saccharose, cellobiose, isomaltose, maltitol and isomaltitol or from the group of polyanionic compounds consisting of a saccharide skeleton formed by a discrete number of saccharide units chosen from among the group made up of carboxymethylmaltotriose, carboxymethylmaltotetraose, carboxymethylmaltopentaose, carboxymethylmaltohexaose, carboxymethylmaltoheptaose, carboxymethylmaltooactose and carboxymethylisomaltotriose and their salts of Na⁺, K⁺, Ca²⁺ or Mg²⁺.

3. The composition as claimed in claim 2, **characterized in that** the carboxylic polyacid is chosen from the group made up of citric acid, tartaric acid, and their salts of Na⁺, K⁺, Ca²⁺ or Mg²⁺.

4. The composition as claimed in claim 2, **characterized in that** the polyanionic compound consisting of a saccharide skeleton formed by a discrete number of saccharide units is chosen from the group made up of carboxymethylmaltotriose, carboxymethylmaltotetraose, carboxymethylmaltopentaose, carboxymethylmaltohexaose, carboxymethylmaltoheptaose, carboxymethylmaltooactose and carboxymethylisomaltotriose and their salts of Na⁺, K⁺, Ca²⁺ or Mg²⁺.

5. The composition as claimed in any one of the preceding claims, **characterized in that** the insulin is a human insulin.

6. The composition as claimed in any one of claims 1 to 4, **characterized in that** he insulin is an insulin analogue.

7. The composition as claimed in claim 6, **characterized in that** the insulin analogue is chosen from the group comprised of insulin lispro (Humalog®), insulin aspart (Novolog®, Novorapid®) and insulin glulisine (Apidra®).

8. The composition as claimed in claim 7, **characterized in that** the insulin analogue is insulin lispro (Humalog®).

9. The composition as claimed in any one of the preceding claims, **characterized in that** the mass ratio of substituted anionic compound to insulin is between 0.5 and 10.

10. The composition as claimed in any one of the preceding claims, **characterized in that** the concentration of substituted anionic compound is between 1.8 and 36 mg/ml.

11. A pharmaceutical formulation comprising a composition as claimed in any one of the preceding claims.

12. The pharmaceutical formulation as claimed in claim 11, **characterized in that** the concentration of insulin is between 240 and 3000 µM (40 to 500 IU/mL).

13. A use of at least one substituted anionic compound consisting of a skeleton formed by a discrete number u between 1 and 8 (1 ≤ u ≤ 8) of identical or different saccharide units, linked by identical or different glycoside bonds, said saccharide units being chosen from the group consisting of the hexoses, in ring form or in reduced open form, said compound containing partially substituted carboxyl functional groups, the nonsubstituted carboxyl functional groups being salt-forming in order to prepare a pharmaceutical formulation of human insulin analogue in mixture with a polyanionic compound, enabling after administration an accelerated passage of insulin into the blood and more rapid reducing of glycemia as compared to a formulation not having a substituted anionic compound and optionally a polyanionic compound.

14. The use as claimed in claim 13, **characterized in that** the substituted anionic compound is chosen from among the substituted
anionic compounds consisting of a skeleton formed by a discrete number u between l and 8 (l ≤ u ≤ 8) of identical or different saccharide units, linked by identical or different glycoside bonds, said saccharide units being chosen from the group consisting of the hexoses, in ring form or in reduced open form, **characterized**:
a) **in that** they are substituted in statistical manner by:
> at least one substituent of general formula I:
-[R₁]ₐ-[AA]ₘ Formula I
• the substituents being identical or different when there are at least two substituents, in which:
• the radical -[AA]- denotes an amino acid residue, said amino acid being chosen from the group made up of phenylalanine, alpha-meéthyl-pheénylalanine, 3,4 dihydroxypheénylalanine, tyrosine, alpha-methyl-tyrosine, O-mehyl-tyrosine, alpha-phenylglycine, 4-hydroxyphenylglycine, 3,5-dihydroxyphenylglycine and their salts of alkaline cations, said derivates being of absolute L or D configuration, -[AA]- is attached to the skeleton of the molecule by means of a bonding -R₁- or directly connected to the skeleton by a function G,
• -R₁- being:
either a bond G, and then a = 0,
or a carbon chain, and then a = 1, with C1 to C15 possibly substituted and/or comprising at least one heteroatom chosen from among O, N and S and carrying at least one acid function prior to the reaction with the amino acid, said chain forming with the amino acid residue -[AA]- an amide function, and is attached to the saccharide skeleton with the aid of a function F resulting from a reaction between a hydroxyl function carried by the skeleton and a function carried by the precursor of R₁,
• F is an ether, ester or carbamate function,
• G is an ester or carbamate function,
• m is equal to 1 or 2,
• the degree of substitution, j, in -[R₁]ₐ-[AA]ₘ being absolutely greater than 0 and less than or equal to 6, 0 ≤ j ≤ 6,
and optionally
> one or more substituents -R'₁
• -R'₁ being a carbon chain with C2 to C15, possibly substituted and/or comprising at least one heteroatom (such as O, N and S) and carrying at least one acid function in the form of a salt of alkaline cations, said chain being attached to the saccharide skeleton by a function F' resulting from a reaction between a hydroxyl function carried by the skeleton and a function carried by the precursor of -R'₁
• F' is an ether, ester or carbamate function,
• the degree of substitution i, in -R'₁, being between 0 and 6-j, 0 ≤ i ≤ 6-j, and
• -R'₁- is identical or different to -R₁,
• F and F' are identical or different,
• F' and G are identical or different
• The free salt-forming acid functions are in the form of salts of alkaline cations,
b) said glycosidic bonds, identical or different, being chosen from the group made up of glycosidic bonds of type (1,1), (1,2), (1,3), (1,4) or (1,6), in an alpha or beta geometry,
c) i + j ≤ 6

15. The use as claimed in one of claims 13 to 14, **characterized in that** the polyanionic compound is chosen from the group made up of carboxylic polyacids or the group made up of phosphoric polyacids or the group made of of compounds comprised of a saccharide skeleton formed by a discrete number of saccharide units obtained from a disaccharide compound chosen from the group made up of trehalose, maltose, lactose, saccharose, cellobiose, isomaltose, maltitol and isomaltitol or from the group of polyanionic compounds consisting of a saccharide skeleton formed by a discrete number of saccharide units chosen from among the group made up of carboxymethylmaltotriose, carboxymethylmaltotetraose, carboxymethylmaltopentaose, carboxymethylmaltohexaose, carboxymethylmaltoheptaose, carboxymethylmaltooactose and carboxymethylisomaltotriose and their salts of Na⁺, K⁺, Ca²⁺ or Mg²⁺.
